# EUROPÄISCHE PATENTANMELDUNG

(11) **EP 3 644 061 A1**
(43) Veröffentlichungstag der Anmeldung: **29.04.2020**
(21) Anmeldenummer: 19203350.4
(22) Anmeldetag: 15.10.2019
(51) Int. Cl.: G01N 33/564, G01N 33/68, C07K 14/78, C07K 16/18, A61K 38/39, A61K 39/395

(54) **DIAGNOSE BLASENBILDENDER AUTOIMMUNKRANKHEITEN**

(30) Priorität: 22.10.2018 EP 18201752
(71) Anmelder: Euroimmun Medizinische Labordiagnostika AG, 23560 Lübeck (DE); Universität zu Lübeck, 23562 Lübeck (DE)
(72) Erfinder: Probst, Christian, 23909 Ratzeburg (DE); Radzimski, Christiane, 23858 Reinfeld (DE); Komorowski, Lars, 23909 Ratzeburg (DE); Schlumberger, Wolfgang, 23627 Groß Grönau (DE); Stöcker, Winfried, 23627 Groß Grönau (DE); Zillikens, Detlef, 23562 Lübeck (DE); Hammers, Christoph, 23562 Lübeck (DE); Schmidt, Enno, 23562 Lübeck (DE); Goletz, Stephanie, 23562 Lübeck (DE)

(57) **Zusammenfassung**

Die vorliegende Erfindung betrifft ein Polypeptid umfassend Laminin beta-4, ein Träger umfassend das Polypeptid, einen Antikörper, bevorzugt Autoantikörper gegen Laminin beta-4, eine Verwendung des Polypeptides, Trägers oder Autoantikörpers zur Diagnose einer Krankheit sowie ein Verfahren umfassend den Schritt Nachweisen eines Autoantikörpers gegen Laminin beta-4 in einer Probe.

SEQUENCE LISTING

<110>  EUROIMMUN Medizinische Labordiagnostika AG

<120>  Diagnose blasenbildender Autoimmunkrankheiten

<130>  18PP205EP

<160>  31

<170>  PatentIn version 3.5

<210>  1
<211>  45
<212>  DNA
<213>  Artificial Sequence

<220>
<223>  sense LAMB4 Primer

<400>  1
ataggtctca catgcaattt caactgaccc tttttttgca ccttg                    45

<210>  2
<211>  45
<212>  DNA
<213>  Artificial Sequence

<220>
<223>  asense LAMB4 Primer

<400>  2
ataggtctcg tcgaggctat agcacctagc atattttttt tcttg                    45

<210>  3
<211>  48
<212>  DNA
<213>  Artificial Sequence

<220>
<223>  LAMB4-Stop Primer

<400>  3
ataggtctcc tcgagctagc tatagcacct agcatatttt ttttcttg                 48

<210>  4
<211>  5758
<212>  DNA
<213>  Artificial Sequence

<220>
<223>  pTriEx-1

<400>  4
ggggaattgt gagcggataa caattccccg gagttaatcc gggaccttta attcaaccca      60

acacaatata ttatagttaa ataagaatta ttatcaaatc atttgtatat taattaaaat     120

actatactgt aaattacatt ttatttacaa tcaaaggaga tataccatgg cgatatcccg     180

ggagctcgtg gatccgaatt ctcagatctc ggcgcgcctg caggtcgacg gtaccggttc 240

gaagcttgcg gccgcacagc tgtatacacg tgcaagccag ccagaactcg ccccggaaga 300

ccccgaggat ctcgagcacc accatcacca tcaccatcac taagtgatta acctcaggtg 360

caggctgcct atcagaaggt ggtggctggt gtggccaatg ccctggctca caaataccac 420

tgagatcgat ctttttccct ctgccaaaaa ttatggggac atcatgaagc cccttgagca 480

tctgacttct ggctaataaa ggaaatttat tttcattgca atagtgtgtt ggaatttttt 540

gtgtctctca ctcggaagga catatgggag ggcaaatcat ttaaaacatc agaatgagta 600

tttggtttag agtttggcaa catatgccca tatgtaacta gcataacccc ttggggcctc 660

taaacgggtc ttgaggggtt ttttgctgaa agcatgcgga ggaaattctc cttgaagttt 720

ccctggtgtt caaagtaaag gagtttgcac cagacgcacc tctgttcact ggtccggcgt 780

attaaaacac gatacattgt tattagtaca tttattaagc gctagattct gtgcgttgtt 840

gatttacaga caattgttgt acgtatttta ataattcatt aaatttataa tctttagggt 900

ggtatgttag agcgaaaatc aaatgatttt cagcgtcttt atatctgaat ttaaatatta 960

aatcctcaat agatttgtaa aataggtttc gattagtttc aaacaagggt tgtttttccg 1020

aaccgatggc tggactatct aatggatttt cgctcaacgc cacaaaactt gccaaatctt 1080

gtagcagcaa tctagctttg tcgatattcg tttgtgtttt gttttgtaat aaaggttcga 1140

cgtcgttcaa aatattatgc gcttttgtat ttctttcatc actgtcgtta gtgtacaatt 1200

gactcgacgt aaacacgtta aatagagctt ggacatattt aacatcgggc gtgttagctt 1260

tattaggccg attatcgtcg tcgtcccaac cctcgtcgtt agaagttgct tccgaagacg 1320

attttgccat agccacacga cgcctattaa ttgtgtcggc taacacgtcc gcgatcaaat 1380

ttgtagttga gctttttgga attatttctg attgcgggcg tttttgggcg ggtttcaatc 1440

taactgtgcc cgattttaat tcagacaaca cgttagaaag cgatggtgca ggcggtggta 1500

acatttcaga cggcaaatct actaatggcg gcggtggtgg agctgatgat aaatctacca 1560

tcggtggagg cgcaggcggg gctggcggcg gaggcggagg cggaggtggt ggcggtgatg 1620

cagacggcgg tttaggctca aatgtctctt taggcaacac agtcggcacc tcaactattg 1680

tactggtttc gggcgccgtt tttggtttga ccggtctgag acgagtgcga tttttttcgt 1740

ttctaatagc ttccaacaat tgttgtctgt cgtctaaagg tgcagcgggt tgaggttccg 1800

tcggcattgg tggagcgggc ggcaattcag acatcgatgg tggtggtggt ggtggaggcg 1860

ctggaatgtt aggcacggga gaaggtggtg gcggcggtgc cgccggtata atttgttctg 1920

gtttagtttg ttcgcgcacg attgtgggca ccggcgcagg cgccgctggc tgcacaacgg 1980

aaggtcgtct gcttcgaggc agcgcttggg gtggtggcaa ttcaatatta taattggaat 2040

acaaatcgta aaaatctgct ataagcattg taatttcgct atcgtttacc gtgccgatat 2100

ttaacaaccg ctcaatgtaa gcaattgtat tgtaaagaga ttgtctcaag ctcggaacgc 2160

tgcgctcggt cgttcggctg cggcgagcgg tatcagctca ctcaaaggcg gtaatacggt 2220

tatccacaga atcaggggat aacgcaggaa agaacatgtg agcaaaaggc cagcaaaagg 2280

ccaggaaccg taaaaaggcc gcgttgctgg cgtttttcca taggctccgc ccccctgacg 2340

agcatcacaa aaatcgacgc tcaagtcaga ggtggcgaaa cccgacagga ctataaagat 2400

accaggcgtt tccccctgga agctccctcg tgcgctctcc tgttccgacc ctgccgctta 2460

ccggatacct gtccgccttt ctcccttcgg gaagcgtggc gctttctcaa tgctcacgct 2520

gtaggtatct cagttcggtg taggtcgttc gctccaagct gggctgtgtg cacgaacccc 2580

ccgttcagcc cgaccgctgc gccttatccg gtaactatcg tcttgagtcc aacccggtaa 2640

gacacgactt atcgccactg gcagcagcca ctggtaacag gattagcaga gcgaggtatg 2700

taggcggtgc tacagagttc ttgaagtggt ggcctaacta cggctacact agaaggacag 2760

tatttggtat ctgcgctctg ctgaagccag ttaccttcgg aaaaagagtt ggtagctctt 2820

gatccggcaa acaaaccacc gctggtagcg gtggtttttt tgtttgcaag cagcagatta 2880

cgcgcagaaa aaaaggatct caagaagatc ctttgttacc aatgcttaat cagtgaggca 2940

cctatctcag cgatctgtct atttcgttca tccatagttg cctgactccc cgtcgtgtag 3000

ataactacga tacgggaggg cttaccatct ggccccagtg ctgcaatgat accgcgagac 3060

ccacgctcac cggctccaga tttatcagca ataaaccagc cagccggaag ggccgagcgc 3120

agaagtggtc ctgcaacttt atccgcctcc atccagtcta ttaattgttg ccgggaagct 3180

agagtaagta gttcgccagt taatagtttg cgcaacgttg ttgccattgc tacaggcatc 3240

gtggtgtcac gctcgtcgtt tggtatggct tcattcagct ccggttccca acgatcaagg 3300

cgagttacat gatcccccat gttgtgcaaa aaagcggtta gctccttcgg tcctccgatc 3360

gttgtcagaa gtaagttggc cgcagtgtta tcactcatgg ttatggcagc actgcataat 3420

tctcttactg tcatgccatc cgtaagatgc ttttctgtga ctggtgagta ctcaaccaag 3480

tcattctgag aatagtgtat gcggcgaccg agttgctctt gcccggcgtc aatacgggat 3540

aataccgcgc cacatagcag aactttaaaa gtgctcatca ttggaaaacg ttcttcgggg 3600

cgaaaactct caaggatctt accgctgttg agatccagtt cgatgtaacc cactcgtgca 3660

cccaactgat cttcagcatc ttttactttc accagcgttt ctgggtgagc aaaaacagga 3720

aggcaaaatg ccgcaaaaaa gggaataagg gcgacacgga aatgttgaat actcatactc 3780

ttcctttttc aatattattg aagcatttat cagggttatt gtctcatgtc cgcgcgtttc 3840

ctgcatcttt taatcaaatc ccaagatgtg tataaaccac caaactgcca aaaaatgaaa 3900

actgtcgaca agctctgtcc gtttgctggc aactgcaagg gtctcaatcc tatttgtaat 3960

tattgaataa taaaacaatt ataaatgtca aatttgtttt ttattaacga tacaaaccaa 4020

acgcaacaag aacatttgta gtattatcta taattgaaaa cgcgtagtta taatcgctga 4080

ggtaatattt aaaatcattt tcaaatgatt cacagttaat ttgcgacaat ataattttat 4140

tttcacataa actagacgcc ttgtcgtctt cttcttcgta ttccttctct tttcattttt 4200

tctcttcata aaaattaaca tagttattat cgtatccata tatgtatcta tcgtatagag 4260

taaatttttt gttgtcataa atatatatgt cttttttaat ggggtgtata gtaccgctgc 4320

gcatagtttt tctgtaattt acaacagtgc tattttctgg tagttcttcg gagtgtgttg 4380

ctttaattat taaatttata taatcaatga atttgggatc gtcggttttg tacaatatgt 4440

tgccggcata gtacgcagct tcttctagtt caattacacc attttttagc agcaccggat 4500

taacataact ttccaaaatg ttgtacgaac cgttaaacaa aaacagttca cctccctttt 4560

ctatactatt gtctgcgagc agttgtttgt tgttaaaaat aacagccatt gtaatgagac 4620

gcacaaacta atatcacaaa ctggaaatgt ctatcaatat atagttgctc tagttattaa 4680

tagtaatcaa ttacggggtc attagttcat agcccatata tggagttccg cgttacataa 4740

cttacggtaa atggcccgcc tggctgaccg cccaacgacc cccgcccatt gacgtcaata 4800

atgacgtatg ttcccatagt aacgccaata gggactttcc attgacgtca atgggtggac 4860

tatttacggt aaactgccca cttggcagta catcaagtgt atcatatgcc aagtacgccc 4920

cctattgacg tcaatgacgg taaatggccc gcctggcatt atgcccagta catgacctta 4980

tgggactttc ctacttggca gtacatctac gtattagtca tcgctattac catgcatggt 5040

cgaggtgagc cccacgttct gcttcactct ccccatctcc cccccctccc cacccccaat 5100

tttgtattta tttatttttt aattattttg tgcagcgatg ggggcggggg gggggggggg 5160

gcgcgcgcca ggcggggcgg ggcggggcga ggggcggggc ggggcgaggc ggagaggtgc 5220

ggcggcagcc aatcagagcg gcgcgctccg aaagtttcct tttatggcga ggcggcggcg 5280

gcggcggccc tataaaaagc gaagcgcgcg gcgggcggga gtcgctgcga cgctgccttc 5340

gccccgtgcc ccgctccgcc gccgcctcgc gccgcccgcc ccggctctga ctgaccgcgt 5400

tactcccaca ggtgagcggg cgggacggcc cttctccttc gggctgtaat tagcgcttgg 5460

tttaatgacg gcttgtttct tttctgtggc tgcgtgaaag ccttgagggg ctccgggagg 5520

gccctttgtg cggggggagc ggctcggggc tgtccgcggg gggacggctg ccttcggggg 5580

ggacggggca gggcggggtt cggcttctgg cgtgtgaccg gcggctctag agcctctgct 5640

aaccatgttc atgccttctt ctttttccta cagctcctgg gcaacgtgct ggttattgtg 5700

ctgtctcatc attttggcaa agaattggat cggaccgaaa ttaatacgac tcactata 5758

<210> 5
<211> 10897

<212>  DNA
<213>  Artificial Sequence

<220>
<223>  pTriEx-1-LAMB4(IF1)[human]

<400>  5
ggagtcgctg cgacgctgcc ttcgccccgt gccccgctcc gccgccgcct cgcgccgccc      60

gccccggctc tgactgaccg cgttactccc acaggtgagc gggcgggacg gcccttctcc     120

ttcgggctgt aattagcgct tggtttaatg acggcttgtt tcttttctgt ggctgcgtga     180

aagccttgag gggctccggg agggcccttt gtgcgggggg agcggctcgg ggctgtccgc     240

ggggggacgg ctgccttcgg gggggacggg gcagggcggg gttcggcttc tggcgtgtga     300

ccggcggctc tagagcctct gctaaccatg ttcatgcctt cttctttttc ctacagctcc     360

tgggcaacgt gctggttatt gtgctgtctc atcattttgg caaagaattg gatcggaccg     420

aaattaatac gactcactat aggggaattg tgagcggata acaattcccc ggagttaatc     480

cgggaccttt aattcaaccc aacacaatat attatagtta aataagaatt attatcaaat     540

catttgtata ttaattaaaa tactatactg taaattacat tttatttaca atcaaaggag     600

atataccatg caatttcaac tgaccctttt tttgcacctt gggtggctca gttactcaaa     660

agctcaagat gactgcaaca ggggtgcctg tcatcccacc actggtgatc tcctggtggg     720

caggaacacg cagcttatgg cttcttctac ctgtgggctg agcagagccc agaaatactg     780

catcctcagt tacctggagg gggaacaaaa atgcttcatc tgtgactcta gatttccata     840

tgatccgtat gaccaaccca acagccacac cattgagaat gtcattgtaa gttttgaacc     900

agacagagaa aagaaatggt ggcaatctga aaatggtctt gatcatgtca gcatcagact     960

ggacttagag gcattatttc ggttcagcca ccttatcctg acctttaaga cttttcggcc    1020

tgctgcaatg ttagttgaac gttccacaga ctatggacac aactggaaag tgttcaaata    1080

ttttgcaaaa gactgtgcca cttcctttcc taacatcaca tctggccagg cccagggagt    1140

gggagacatt gtttgtgact ccaaatactc ggatattgaa ccctcaacag gtggagaggt    1200

tgttttaaaa gttttggatc ccagttttga aattgaaaac ccttatagcc cctacatcca    1260

agaccttgtg acattgacaa acctgaggat aaactttacc aagctccaca cccttgggga    1320

tgctttgctt ggaaggaggc aaaatgattc ccttgataaa tactactatg ctctgtacga    1380

gatgattgtt cggggaagct gcttttgcaa tggccatgct agcgaatgtc gccctatgca    1440

gaagatgcgg ggagatgttt tcagccctcc tggaatggtt cacggtcagt gtgtgtgtca    1500

gcacaataca gatggtccga actgtgagag atgcaaggac ttcttccagg atgctccttg    1560

gaggccagct gcagacctcc aggacaacgc ttgcagatcg tgcagctgta acagccactc    1620

cagccgctgt cactttgaca tgactacgta cctggcaagc ggtggcctca gcgggggcgt    1680

gtgtgaagac tgccagcaca acactgaggg gcagcactgc gaccgctgca gacccctctt 1740

ctacagggac ccgctcaaga ccatctcaga tccctacgcg tgcattcctt gtgaatgtga 1800

ccccgatggg accatatctg gtggcatttg tgtgagccac tctgatcctg ccttagggtc 1860

tgtggctggc cagtgccttt gtaaagagaa cgtggaagga gccaaatgcg accagtgcaa 1920

acccaaccac tatggactaa gcgccaccga ccccctgggc tgccagccct gcgactgtaa 1980

cccccttggg agtctgccat tcttgacctg tgatgtggat acaggccaat gcttgtgcct 2040

gtcatatgtc accggagcac actgcgaaga atgcactgtt ggatactggg gcctgggaaa 2100

tcatctccat gggtgttctc cctgtgactg tgatattgga ggtgcttatt ctaacgtgtg 2160

ctcacccaag aatgggcagt gtgaatgccg cccacatgtc actggccgta gctgctctga 2220

accagcccct ggctacttct ttgctccttt gaatttctat ctctacgagg cagaggaagc 2280

cacaacactc caaggactgg cgcctttggg ctcggagacg tttggccaga gtcctgctgt 2340

tcacgttgtt ttaggagagc cagttcctgg gaaccctgtt acatggactg gacctggatt 2400

tgccagggtt ctccctgggg ctggcttgag atttgctgtc aacaacattc cctttcctgt 2460

ggacttcacc attgccattc actatgaaac ccagtctgca gctgactgga ctgtccagat 2520

tgtggtgaac ccccctggag ggagtgagca ctgcataccc aagactctac agtcaaagcc 2580

tcagtctttt gccttaccag cggctacgag aatcatgctg cttcccacac ccatctgttt 2640

agaaccagat gtacaatatt ccatagatgt ctatttttct cagcctttgc aaggagagtc 2700

ccacgctcat tcacatgtcc tggtggactc tcttggcctt attccccaaa tcaattcatt 2760

ggagaatttc tgcagcaagc aggacttaga tgagtatcag cttcacaact gtgttgaaat 2820

tgcctcagca atgggacctc aagtgctccc gggtgcctgt gaaaggctga tcatcagcat 2880

gtctgccaag ctgcatgatg gggctgtggc ctgcaagtgt cacccccagg gctcagtcgg 2940

atccagctgc agccgacttg gaggccagtg ccagtgtaaa cctcttgtgg tcgggcgctg 3000

ctgtgacagg tgctcaactg gaagctatga tttggggcat cacggctgtc acccatgtca 3060

ctgccatcct caaggatcaa aggacactgt atgtgaccaa gtaacaggac agtgcccctg 3120

ccatggagag gtgtctggcc gccgctgtga tcgctgcctg gcaggctact ttggatttcc 3180

cagctgccac ccttgccctt gtaataggtt tgctgaactt tgtgatcctg agacagggtc 3240

atgcttcaat tgtggaggct ttacaactgg cagaaactgt gaaaggtgta ttgatggtta 3300

ctatggaaat ccttcttcag gacagccctg tcgtccttgc ctgtgtccag atgatccctc 3360

aagcaatcag tattttgccc attcctgtta tcagaatctg tggagctcag atgtaatctg 3420

caattgtctt caaggttata cgggtactca gtgtggagaa tgctctactg gtttctatgg 3480

aaatccaaga atttcaggag caccttgcca accatgtgcc tgcaacaaca acatagatgt 3540

aaccgatcca gagtcctgca gccgggtaac aggggagtgc cttcgatgtt tgcacaacac 3600

tcagggcgca aactgccagc tctgcaaacc aggtcactat ggatcagccc tcaatcagac 3660

ctgcagaaga tgctcctgcc atgcttccgg cgtgagtccc atggagtgtc cccctggtgg 3720

gggagcttgc ctctgtgacc ctgtcactgg tgcatgtcct tgtctgccga atgtcacagg 3780

cctggcctgt gaccgttgtg ctgatggata ctggaatctg gtccctggca gaggatgtca 3840

gtcatgtgac tgtgacccta ggacctctca aagtagccac tgtgaccagc ttacaggcca 3900

gtgtccgtgt aaattaggtt acggcgggaa acgttgcagt gagtgccagg aaaattatta 3960

tggtgatcca cctgggcgat gcattccatg tgattgtaac agggcaggta cccagaagcc 4020

catctgtgat ccagacacag gcatgtgccg ctgccgggag ggtgtcagcg gccagagatg 4080

tgatcgctgt gcccggggac acagccagga attccctact tgtcttcaat gtcacttgtg 4140

ctttgatcag tgggaccaca ccatttcttc cctctccaaa gcggtgcaag ggttaatgag 4200

actggctgct aacatggaag ataaaagaga gaccctgcct gtctgtgagg cagacttcaa 4260

agacctcaga gggaacgtgt ctgaaataga aaggattttg aaacatcctg ttttcccatc 4320

tgggaaattc ttaaaagtca aggattatca tgactctgtt agaagacaaa tcatgcagct 4380

aaatgaacaa ctgaaagcag tgtatgaatt tcaagatctg aaagatacaa tagaaagagc 4440

aaagaatgaa gcagacctct tacttgaaga ccttcaggaa gaaattgatt tgcaatccag 4500

tgtccttaat gcaagcattg cggactcctc agaaaacatc aagaaatatt atcacatatc 4560

atcatctgct gaaaagaaaa ttaatgaaac tagttccacc attaatacct ctgcaaatac 4620

aaggaatgac ttacttacca tcttagatac actaacctca aaaggaaact tgtcattgga 4680

aagattaaag cagattaaga taccagatat ccaaatattg aatgaaaagg tgtgcggaga 4740

tccaggaaat gtgccatgtg tgcccttgcc ctgtggcggt gctctctgca cgggccggaa 4800

ggggcacagg aagtgtaggg gtcccggctg tcacggctcc ctgaccctct caacgaatgc 4860

cctccaaaaa gcccaggaag caaaatccat tattcgtaat ttggacaaac aggttcgtgg 4920

gttgaaaaat cagatcgaaa gtataagtga acaggcagaa gtctccaaaa acaatgcctt 4980

acagctgagg gaaaaactgg gaaatataag aaaccaaagt gactctgaag aagaaaacat 5040

caatcttttc atcaaaaaag tgaaaaactt tttgttagag gaaaacgtgc ctccagaaga 5100

catcgagaag gttgcgaatg gtgtgcttga cattcaccta ccaattccat cccaaaatct 5160

aaccgatgaa cttgtcaaaa tacagaaaca tatgcaactc tgtgaggatt acaggacaga 5220

tgaaaacagg ttaaatgaag aagcagatgg agcccaaaag cttttggtga aggccaaagc 5280

agctgagaaa gcagcaaata ttctattaaa tcttgacaaa acattgaacc agttacaaca 5340

agctcaaatc actcaaggac gggcaaactc taccattaca cagctgactg ccaatataac 5400

aaaaataaaa aagaatgtgc tgcaggctga aaatcaaacc agggaaatga agagtgagct 5460

ggagttagca aagcagcgat cagggctgga ggatggactt tccctgctgc agaccaagtt 5520

gcaaaggcat caagaccacg ctgtcaatgc gaaagttcag gctgaatctg cccaacacca 5580

ggctgggagt cttgagaagg aatttgttga gctgaaaaaa caatatgcta ttctccaacg 5640

taagacaagc actacaggac taacaaagga gacattagga aaagttaaac agctaaaaga 5700

tgcggcagaa aaattggctg gagatacaga ggccaagata agaagaataa cagatttaga 5760

aaggaaaatc caagatttga atctaagtag acaagcaaaa gctgatcaac tgagaatatt 5820

ggaagatcaa gttgttgcca ttaaaaatga aattgttgaa caagaaaaaa aatatgctag 5880

gtgctatagc ctcgagcacc accatcacca tcaccatcac taagtgatta acctcaggtg 5940

caggctgcct atcagaaggt ggtggctggt gtggccaatg ccctggctca caaataccac 6000

tgagatcgat ctttttccct ctgccaaaaa ttatggggac atcatgaagc cccttgagca 6060

tctgacttct ggctaataaa ggaaatttat tttcattgca atagtgtgtt ggaatttttt 6120

gtgtctctca ctcggaagga catatgggag ggcaaatcat ttaaaacatc agaatgagta 6180

tttggtttag agtttggcaa catatgccca tatgtaacta gcataacccc ttggggcctc 6240

taaacgggtc ttgaggggtt ttttgctgaa agcatgcgga ggaaattctc cttgaagttt 6300

ccctggtgtt caaagtaaag gagtttgcac cagacgcacc tctgttcact ggtccggcgt 6360

attaaaacac gatacattgt tattagtaca tttattaagc gctagattct gtgcgttgtt 6420

gatttacaga caattgttgt acgtatttta ataattcatt aaatttataa tctttagggt 6480

ggtatgttag agcgaaaatc aaatgatttt cagcgtcttt atatctgaat ttaaatatta 6540

aatcctcaat agatttgtaa aataggtttc gattagtttc aaacaagggt tgtttttccg 6600

aaccgatggc tggactatct aatggatttt cgctcaacgc cacaaaactt gccaaatctt 6660

gtagcagcaa tctagctttg tcgatattcg tttgtgtttt gttttgtaat aaaggttcga 6720

cgtcgttcaa aatattatgc gcttttgtat ttctttcatc actgtcgtta gtgtacaatt 6780

gactcgacgt aaacacgtta aatagagctt ggacatattt aacatcgggc gtgttagctt 6840

tattaggccg attatcgtcg tcgtcccaac cctcgtcgtt agaagttgct tccgaagacg 6900

attttgccat agccacacga cgcctattaa ttgtgtcggc taacacgtcc gcgatcaaat 6960

ttgtagttga gctttttgga attatttctg attgcgggcg tttttgggcg ggtttcaatc 7020

taactgtgcc cgattttaat tcagacaaca cgttagaaag cgatggtgca ggcggtggta 7080

acatttcaga cggcaaatct actaatggcg gcggtggtgg agctgatgat aaatctacca 7140

tcggtggagg cgcaggcggg gctggcggcg gaggcggagg cggaggtggt ggcggtgatg 7200

cagacggcgg tttaggctca aatgtctctt taggcaacac agtcggcacc tcaactattg 7260

tactggtttc gggcgccgtt tttggtttga ccggtctgag acgagtgcga tttttttcgt 7320

ttctaatagc ttccaacaat tgttgtctgt cgtctaaagg tgcagcgggt tgaggttccg 7380

tcggcattgg tggagcgggc ggcaattcag acatcgatgg tggtggtggt ggtggaggcg 7440

ctggaatgtt aggcacggga gaaggtggtg gcggcggtgc cgccggtata atttgttctg 7500

gtttagtttg ttcgcgcacg attgtgggca ccggcgcagg cgccgctggc tgcacaacgg 7560

aaggtcgtct gcttcgaggc agcgcttggg gtggtggcaa ttcaatatta taattggaat 7620

acaaatcgta aaaatctgct ataagcattg taatttcgct atcgtttacc gtgccgatat 7680

ttaacaaccg ctcaatgtaa gcaattgtat tgtaaagaga ttgtctcaag ctcggaacgc 7740

tgcgctcggt cgttcggctg cggcgagcgg tatcagctca ctcaaaggcg gtaatacggt 7800

tatccacaga atcaggggat aacgcaggaa agaacatgtg agcaaaaggc cagcaaaagg 7860

ccaggaaccg taaaaaggcc gcgttgctgg cgtttttcca taggctccgc ccccctgacg 7920

agcatcacaa aaatcgacgc tcaagtcaga ggtggcgaaa cccgacagga ctataaagat 7980

accaggcgtt tccccctgga agctccctcg tgcgctctcc tgttccgacc ctgccgctta 8040

ccggatacct gtccgccttt ctcccttcgg gaagcgtggc gctttctcaa tgctcacgct 8100

gtaggtatct cagttcggtg taggtcgttc gctccaagct gggctgtgtg cacgaacccc 8160

ccgttcagcc cgaccgctgc gccttatccg gtaactatcg tcttgagtcc aacccggtaa 8220

gacacgactt atcgccactg gcagcagcca ctggtaacag gattagcaga gcgaggtatg 8280

taggcggtgc tacagagttc ttgaagtggt ggcctaacta cggctacact agaaggacag 8340

tatttggtat ctgcgctctg ctgaagccag ttaccttcgg aaaaagagtt ggtagctctt 8400

gatccggcaa acaaaccacc gctggtagcg gtggtttttt tgtttgcaag cagcagatta 8460

cgcgcagaaa aaaaggatct caagaagatc ctttgttacc aatgcttaat cagtgaggca 8520

cctatctcag cgatctgtct atttcgttca tccatagttg cctgactccc cgtcgtgtag 8580

ataactacga tacgggaggg cttaccatct ggccccagtg ctgcaatgat accgcgagac 8640

ccacgctcac cggctccaga tttatcagca ataaaccagc cagccggaag ggccgagcgc 8700

agaagtggtc ctgcaacttt atccgcctcc atccagtcta ttaattgttg ccgggaagct 8760

agagtaagta gttcgccagt taatagtttg cgcaacgttg ttgccattgc tacaggcatc 8820

gtggtgtcac gctcgtcgtt tggtatggct tcattcagct ccggttccca acgatcaagg 8880

cgagttacat gatcccccat gttgtgcaaa aaagcggtta gctccttcgg tcctccgatc 8940

gttgtcagaa gtaagttggc cgcagtgtta tcactcatgg ttatggcagc actgcataat 9000

tctcttactg tcatgccatc cgtaagatgc ttttctgtga ctggtgagta ctcaaccaag 9060

tcattctgag aatagtgtat gcggcgaccg agttgctctt gcccggcgtc aatacgggat 9120

aataccgcgc cacatagcag aactttaaaa gtgctcatca ttggaaaacg ttcttcgggg 9180

cgaaaactct caaggatctt accgctgttg agatccagtt cgatgtaacc cactcgtgca 9240

cccaactgat cttcagcatc ttttactttc accagcgttt ctgggtgagc aaaaacagga 9300

aggcaaaatg ccgcaaaaaa gggaataagg gcgacacgga aatgttgaat actcatactc 9360

ttcctttttc aatattattg aagcatttat cagggttatt gtctcatgtc cgcgcgtttc 9420

ctgcatcttt taatcaaatc ccaagatgtg tataaaccac caaactgcca aaaaatgaaa 9480

actgtcgaca agctctgtcc gtttgctggc aactgcaagg gtctcaatcc tatttgtaat 9540

tattgaataa taaaacaatt ataaatgtca aatttgtttt ttattaacga tacaaaccaa 9600

acgcaacaag aacatttgta gtattatcta taattgaaaa cgcgtagtta taatcgctga 9660

ggtaatattt aaaatcattt tcaaatgatt cacagttaat ttgcgacaat ataattttat 9720

tttcacataa actagacgcc ttgtcgtctt cttcttcgta ttccttctct tttcattttt 9780

tctcttcata aaaattaaca tagttattat cgtatccata tatgtatcta tcgtatagag 9840

taaatttttt gttgtcataa atatatatgt cttttttaat ggggtgtata gtaccgctgc 9900

gcatagtttt tctgtaattt acaacagtgc tattttctgg tagttcttcg gagtgtgttg 9960

ctttaattat taaatttata taatcaatga atttgggatc gtcggttttg tacaatatgt 10020

tgccggcata gtacgcagct tcttctagtt caattacacc attttttagc agcaccggat 10080

taacataact ttccaaaatg ttgtacgaac cgttaaacaa aaacagttca cctccctttt 10140

ctatactatt gtctgcgagc agttgtttgt tgttaaaaat aacagccatt gtaatgagac 10200

gcacaaacta atatcacaaa ctggaaatgt ctatcaatat atagttgctc tagttattaa 10260

tagtaatcaa ttacggggtc attagttcat agcccatata tggagttccg cgttacataa 10320

cttacggtaa atggcccgcc tggctgaccg cccaacgacc cccgcccatt gacgtcaata 10380

atgacgtatg ttcccatagt aacgccaata gggactttcc attgacgtca atgggtggac 10440

tatttacggt aaactgccca cttggcagta catcaagtgt atcatatgcc aagtacgccc 10500

cctattgacg tcaatgacgg taaatggccc gcctggcatt atgcccagta catgacctta 10560

tgggactttc ctacttggca gtacatctac gtattagtca tcgctattac catgcatggt 10620

cgaggtgagc cccacgttct gcttcactct ccccatctcc cccccctccc cacccccaat 10680

tttgtattta tttatttttt aattattttg tgcagcgatg ggggcggggg gggggggggg 10740

gcgcgcgcca ggcggggcgg ggcggggcga ggggcggggc ggggcgaggc ggagaggtgc 10800

ggcggcagcc aatcagagcg gcgcgctccg aaagtttcct tttatggcga ggcggcggcg 10860

gcggcggccc tataaaaagc gaagcgcgcg gcgggcg 10897

<210> 6
<211> 10900
<212> DNA
<213> Artificial Sequence

<220>

<223>  pTriEx-1-LAMB4(IF1)[human](dHis)

<400>  6
ggagtcgctg cgacgctgcc ttcgccccgt gccccgctcc gccgccgcct cgcgccgccc      60

gccccggctc tgactgaccg cgttactccc acaggtgagc gggcgggacg gcccttctcc     120

ttcgggctgt aattagcgct tggtttaatg acggcttgtt tcttttctgt ggctgcgtga     180

aagccttgag gggctccggg agggcccttt gtgcgggggg agcggctcgg ggctgtccgc     240

ggggggacgg ctgccttcgg gggggacggg gcagggcggg gttcggcttc tggcgtgtga     300

ccggcggctc tagagcctct gctaaccatg ttcatgcctt cttctttttc ctacagctcc     360

tgggcaacgt gctggttatt gtgctgtctc atcattttgg caaagaattg gatcggaccg     420

aaattaatac gactcactat aggggaattg tgagcggata acaattcccc ggagttaatc     480

cgggaccttt aattcaaccc aacacaatat attatagtta aataagaatt attatcaaat     540

catttgtata ttaattaaaa tactatactg taaattacat tttatttaca atcaaaggag     600

atataccatg caatttcaac tgaccctttt tttgcacctt gggtggctca gttactcaaa     660

agctcaagat gactgcaaca ggggtgcctg tcatcccacc actggtgatc tcctggtggg     720

caggaacacg cagcttatgg cttcttctac ctgtgggctg agcagagccc agaaatactg     780

catcctcagt tacctggagg gggaacaaaa atgcttcatc tgtgactcta gatttccata     840

tgatccgtat gaccaaccca acagccacac cattgagaat gtcattgtaa gttttgaacc     900

agacagagaa aagaaatggt ggcaatctga aaatggtctt gatcatgtca gcatcagact     960

ggacttagag gcattatttc ggttcagcca ccttatcctg acctttaaga cttttcggcc    1020

tgctgcaatg ttagttgaac gttccacaga ctatggacac aactggaaag tgttcaaata    1080

ttttgcaaaa gactgtgcca cttcctttcc taacatcaca tctggccagg cccagggagt    1140

gggagacatt gtttgtgact ccaaatactc ggatattgaa ccctcaacag gtggagaggt    1200

tgttttaaaa gttttggatc ccagttttga aattgaaaac ccttatagcc cctacatcca    1260

agaccttgtg acattgacaa acctgaggat aaactttacc aagctccaca cccttgggga    1320

tgctttgctt ggaaggaggc aaaatgattc ccttgataaa tactactatg ctctgtacga    1380

gatgattgtt cggggaagct gcttttgcaa tggccatgct agcgaatgtc gccctatgca    1440

gaagatgcgg ggagatgttt tcagccctcc tggaatggtt cacggtcagt gtgtgtgtca    1500

gcacaataca gatggtccga actgtgagag atgcaaggac ttcttccagg atgctccttg    1560

gaggccagct gcagacctcc aggacaacgc ttgcagatcg tgcagctgta acagccactc    1620

cagccgctgt cactttgaca tgactacgta cctggcaagc ggtggcctca gcgggggcgt    1680

gtgtgaagac tgccagcaca acactgaggg gcagcactgc gaccgctgca gacccctctt    1740

ctacagggac ccgctcaaga ccatctcaga tccctacgcg tgcattcctt gtgaatgtga    1800

ccccgatggg accatatctg gtggcatttg tgtgagccac tctgatcctg ccttagggtc 1860

tgtggctggc cagtgccttt gtaaagagaa cgtggaagga gccaaatgcg accagtgcaa 1920

acccaaccac tatggactaa gcgccaccga ccccctgggc tgccagccct gcgactgtaa 1980

cccccttggg agtctgccat tcttgacctg tgatgtggat acaggccaat gcttgtgcct 2040

gtcatatgtc accggagcac actgcgaaga atgcactgtt ggatactggg gcctgggaaa 2100

tcatctccat gggtgttctc cctgtgactg tgatattgga ggtgcttatt ctaacgtgtg 2160

ctcacccaag aatgggcagt gtgaatgccg cccacatgtc actggccgta gctgctctga 2220

accagcccct ggctacttct ttgctccttt gaatttctat ctctacgagg cagaggaagc 2280

cacaacactc caaggactgg cgcctttggg ctcggagacg tttggccaga gtcctgctgt 2340

tcacgttgtt ttaggagagc cagttcctgg gaaccctgtt acatggactg gacctggatt 2400

tgccagggtt ctccctgggg ctggcttgag atttgctgtc aacaacattc cctttcctgt 2460

ggacttcacc attgccattc actatgaaac ccagtctgca gctgactgga ctgtccagat 2520

tgtggtgaac ccccctggag ggagtgagca ctgcataccc aagactctac agtcaaagcc 2580

tcagtctttt gccttaccag cggctacgag aatcatgctg cttcccacac ccatctgttt 2640

agaaccagat gtacaatatt ccatagatgt ctatttttct cagcctttgc aaggagagtc 2700

ccacgctcat tcacatgtcc tggtggactc tcttggcctt attccccaaa tcaattcatt 2760

ggagaatttc tgcagcaagc aggacttaga tgagtatcag cttcacaact gtgttgaaat 2820

tgcctcagca atgggacctc aagtgctccc gggtgcctgt gaaaggctga tcatcagcat 2880

gtctgccaag ctgcatgatg gggctgtggc ctgcaagtgt cacccccagg gctcagtcgg 2940

atccagctgc agccgacttg gaggccagtg ccagtgtaaa cctcttgtgg tcgggcgctg 3000

ctgtgacagg tgctcaactg gaagctatga tttggggcat cacggctgtc acccatgtca 3060

ctgccatcct caaggatcaa aggacactgt atgtgaccaa gtaacaggac agtgcccctg 3120

ccatggagag gtgtctggcc gccgctgtga tcgctgcctg gcaggctact ttggatttcc 3180

cagctgccac ccttgccctt gtaataggtt tgctgaactt tgtgatcctg agacagggtc 3240

atgcttcaat tgtggaggct ttacaactgg cagaaactgt gaaaggtgta ttgatggtta 3300

ctatggaaat ccttcttcag gacagccctg tcgtccttgc ctgtgtccag atgatccctc 3360

aagcaatcag tattttgccc attcctgtta tcagaatctg tggagctcag atgtaatctg 3420

caattgtctt caaggttata cgggtactca gtgtggagaa tgctctactg gtttctatgg 3480

aaatccaaga atttcaggag caccttgcca accatgtgcc tgcaacaaca acatagatgt 3540

aaccgatcca gagtcctgca gccgggtaac aggggagtgc cttcgatgtt tgcacaacac 3600

tcagggcgca aactgccagc tctgcaaacc aggtcactat ggatcagccc tcaatcagac 3660

ctgcagaaga tgctcctgcc atgcttccgg cgtgagtccc atggagtgtc cccctggtgg 3720

gggagcttgc ctctgtgacc ctgtcactgg tgcatgtcct tgtctgccga atgtcacagg 3780

cctggcctgt gaccgttgtg ctgatggata ctggaatctg gtccctggca gaggatgtca 3840

gtcatgtgac tgtgacccta ggacctctca aagtagccac tgtgaccagc ttacaggcca 3900

gtgtccgtgt aaattaggtt acggcgggaa acgttgcagt gagtgccagg aaaattatta 3960

tggtgatcca cctgggcgat gcattccatg tgattgtaac agggcaggta cccagaagcc 4020

catctgtgat ccagacacag gcatgtgccg ctgccgggag ggtgtcagcg gccagagatg 4080

tgatcgctgt gcccggggac acagccagga attccctact tgtcttcaat gtcacttgtg 4140

ctttgatcag tgggaccaca ccatttcttc cctctccaaa gcggtgcaag ggttaatgag 4200

actggctgct aacatggaag ataaaagaga gaccctgcct gtctgtgagg cagacttcaa 4260

agacctcaga gggaacgtgt ctgaaataga aaggattttg aaacatcctg ttttcccatc 4320

tgggaaattc ttaaaagtca aggattatca tgactctgtt agaagacaaa tcatgcagct 4380

aaatgaacaa ctgaaagcag tgtatgaatt tcaagatctg aaagatacaa tagaaagagc 4440

aaagaatgaa gcagacctct tacttgaaga ccttcaggaa gaaattgatt tgcaatccag 4500

tgtccttaat gcaagcattg cggactcctc agaaaacatc aagaaatatt atcacatatc 4560

atcatctgct gaaaagaaaa ttaatgaaac tagttccacc attaatacct ctgcaaatac 4620

aaggaatgac ttacttacca tcttagatac actaacctca aaaggaaact tgtcattgga 4680

aagattaaag cagattaaga taccagatat ccaaatattg aatgaaaagg tgtgcggaga 4740

tccaggaaat gtgccatgtg tgcccttgcc ctgtggcggt gctctctgca cgggccggaa 4800

ggggcacagg aagtgtaggg gtcccggctg tcacggctcc ctgaccctct caacgaatgc 4860

cctccaaaaa gcccaggaag caaaatccat tattcgtaat ttggacaaac aggttcgtgg 4920

gttgaaaaat cagatcgaaa gtataagtga acaggcagaa gtctccaaaa acaatgcctt 4980

acagctgagg gaaaaactgg gaaatataag aaaccaaagt gactctgaag aagaaaacat 5040

caatcttttc atcaaaaaag tgaaaaactt tttgttagag gaaaacgtgc ctccagaaga 5100

catcgagaag gttgcgaatg gtgtgcttga cattcaccta ccaattccat cccaaaatct 5160

aaccgatgaa cttgtcaaaa tacagaaaca tatgcaactc tgtgaggatt acaggacaga 5220

tgaaaacagg ttaaatgaag aagcagatgg agcccaaaag cttttggtga aggccaaagc 5280

agctgagaaa gcagcaaata ttctattaaa tcttgacaaa acattgaacc agttacaaca 5340

agctcaaatc actcaaggac gggcaaactc taccattaca cagctgactg ccaatataac 5400

aaaaataaaa aagaatgtgc tgcaggctga aaatcaaacc agggaaatga agagtgagct 5460

ggagttagca aagcagcgat cagggctgga ggatggactt tccctgctgc agaccaagtt 5520

gcaaaggcat caagaccacg ctgtcaatgc gaaagttcag gctgaatctg cccaacacca 5580

ggctgggagt cttgagaagg aatttgttga gctgaaaaaa caatatgcta ttctccaacg 5640

taagacaagc actacaggac taacaaagga gacattagga aaagttaaac agctaaaaga 5700

tgcggcagaa aaattggctg gagatacaga ggccaagata agaagaataa cagatttaga 5760

aaggaaaatc caagatttga atctaagtag acaagcaaaa gctgatcaac tgagaatatt 5820

ggaagatcaa gttgttgcca ttaaaaatga aattgttgaa caagaaaaaa aatatgctag 5880

gtgctatagc tagctcgagc accaccatca ccatcaccat cactaagtga ttaacctcag 5940

gtgcaggctg cctatcagaa ggtggtggct ggtgtggcca atgccctggc tcacaaatac 6000

cactgagatc gatctttttc cctctgccaa aaattatggg gacatcatga agccccttga 6060

gcatctgact tctggctaat aaaggaaatt tattttcatt gcaatagtgt gttggaattt 6120

tttgtgtctc tcactcggaa ggacatatgg gagggcaaat catttaaaac atcagaatga 6180

gtatttggtt tagagtttgg caacatatgc ccatatgtaa ctagcataac cccttggggc 6240

ctctaaacgg gtcttgaggg gttttttgct gaaagcatgc ggaggaaatt ctccttgaag 6300

tttccctggt gttcaaagta aaggagtttg caccagacgc acctctgttc actggtccgg 6360

cgtattaaaa cacgatacat tgttattagt acatttatta agcgctagat tctgtgcgtt 6420

gttgatttac agacaattgt tgtacgtatt ttaataattc attaaattta taatctttag 6480

ggtggtatgt tagagcgaaa atcaaatgat tttcagcgtc tttatatctg aatttaaata 6540

ttaaatcctc aatagatttg taaaataggt ttcgattagt ttcaaacaag ggttgttttt 6600

ccgaaccgat ggctggacta tctaatggat tttcgctcaa cgccacaaaa cttgccaaat 6660

cttgtagcag caatctagct ttgtcgatat tcgtttgtgt tttgttttgt aataaaggtt 6720

cgacgtcgtt caaaatatta tgcgcttttg tatttctttc atcactgtcg ttagtgtaca 6780

attgactcga cgtaaacacg ttaaatagag cttggacata tttaacatcg ggcgtgttag 6840

ctttattagg ccgattatcg tcgtcgtccc aaccctcgtc gttagaagtt gcttccgaag 6900

acgattttgc catagccaca cgacgcctat taattgtgtc ggctaacacg tccgcgatca 6960

aatttgtagt tgagcttttt ggaattattt ctgattgcgg gcgtttttgg gcgggtttca 7020

atctaactgt gcccgatttt aattcagaca acacgttaga aagcgatggt gcaggcggtg 7080

gtaacatttc agacggcaaa tctactaatg gcggcggtgg tggagctgat gataaatcta 7140

ccatcggtgg aggcgcaggc ggggctggcg gcggaggcgg aggcggaggt ggtggcggtg 7200

atgcagacgg cggtttaggc tcaaatgtct ctttaggcaa cacagtcggc acctcaacta 7260

ttgtactggt ttcgggcgcc gtttttggtt tgaccggtct gagacgagtg cgattttttt 7320

cgtttctaat agcttccaac aattgttgtc tgtcgtctaa aggtgcagcg ggttgaggtt 7380

ccgtcggcat tggtggagcg ggcggcaatt cagacatcga tggtggtggt ggtggtggag 7440

gcgctggaat gttaggcacg ggagaaggtg gtggcggcgg tgccgccggt ataatttgtt 7500

ctggtttagt ttgttcgcgc acgattgtgg gcaccggcgc aggcgccgct ggctgcacaa 7560

cggaaggtcg tctgcttcga ggcagcgctt ggggtggtgg caattcaata ttataattgg 7620

aatacaaatc gtaaaaatct gctataagca ttgtaatttc gctatcgttt accgtgccga 7680

tatttaacaa ccgctcaatg taagcaattg tattgtaaag agattgtctc aagctcggaa 7740

cgctgcgctc ggtcgttcgg ctgcggcgag cggtatcagc tcactcaaag gcggtaatac 7800

ggttatccac agaatcaggg gataacgcag gaaagaacat gtgagcaaaa ggccagcaaa 7860

aggccaggaa ccgtaaaaag gccgcgttgc tggcgttttt ccataggctc cgcccccctg 7920

acgagcatca caaaaatcga cgctcaagtc agaggtggcg aaacccgaca ggactataaa 7980

gataccaggc gtttccccct ggaagctccc tcgtgcgctc tcctgttccg accctgccgc 8040

ttaccggata cctgtccgcc tttctccctt cgggaagcgt ggcgctttct caatgctcac 8100

gctgtaggta tctcagttcg gtgtaggtcg ttcgctccaa gctgggctgt gtgcacgaac 8160

cccccgttca gcccgaccgc tgcgccttat ccggtaacta tcgtcttgag tccaacccgg 8220

taagacacga cttatcgcca ctggcagcag ccactggtaa caggattagc agagcgaggt 8280

atgtaggcgg tgctacagag ttcttgaagt ggtggcctaa ctacggctac actagaagga 8340

cagtatttgg tatctgcgct ctgctgaagc cagttacctt cggaaaaaga gttggtagct 8400

cttgatccgg caaacaaacc accgctggta gcggtggttt ttttgtttgc aagcagcaga 8460

ttacgcgcag aaaaaaagga tctcaagaag atcctttgtt accaatgctt aatcagtgag 8520

gcacctatct cagcgatctg tctatttcgt tcatccatag ttgcctgact ccccgtcgtg 8580

tagataacta cgatacggga gggcttacca tctggcccca gtgctgcaat gataccgcga 8640

gacccacgct caccggctcc agatttatca gcaataaacc agccagccgg aagggccgag 8700

cgcagaagtg gtcctgcaac tttatccgcc tccatccagt ctattaattg ttgccgggaa 8760

gctagagtaa gtagttcgcc agttaatagt ttgcgcaacg ttgttgccat tgctacaggc 8820

atcgtggtgt cacgctcgtc gtttggtatg gcttcattca gctccggttc ccaacgatca 8880

aggcgagtta catgatcccc catgttgtgc aaaaaagcgg ttagctcctt cggtcctccg 8940

atcgttgtca gaagtaagtt ggccgcagtg ttatcactca tggttatggc agcactgcat 9000

aattctctta ctgtcatgcc atccgtaaga tgcttttctg tgactggtga gtactcaacc 9060

aagtcattct gagaatagtg tatgcggcga ccgagttgct cttgcccggc gtcaatacgg 9120

gataataccg cgccacatag cagaacttta aaagtgctca tcattggaaa acgttcttcg 9180

gggcgaaaac tctcaaggat cttaccgctg ttgagatcca gttcgatgta acccactcgt 9240

gcacccaact gatcttcagc atcttttact ttcaccagcg tttctgggtg agcaaaaaca 9300

ggaaggcaaa atgccgcaaa aaagggaata agggcgacac ggaaatgttg aatactcata 9360

ctcttccttt ttcaatatta ttgaagcatt tatcagggtt attgtctcat gtccgcgcgt 9420

ttcctgcatc ttttaatcaa atcccaagat gtgtataaac caccaaactg ccaaaaaatg 9480

aaaactgtcg acaagctctg tccgtttgct ggcaactgca agggtctcaa tcctatttgt 9540

aattattgaa taataaaaca attataaatg tcaaatttgt tttttattaa cgatacaaac 9600

caaacgcaac aagaacattt gtagtattat ctataattga aaacgcgtag ttataatcgc 9660

tgaggtaata tttaaaatca ttttcaaatg attcacagtt aatttgcgac aatataattt 9720

tattttcaca taaactagac gccttgtcgt cttcttcttc gtattccttc tctttttcat 9780

ttttctcttc ataaaaatta acatagttat tatcgtatcc atatatgtat ctatcgtata 9840

gagtaaattt tttgttgtca taaatatata tgtctttttt aatggggtgt atagtaccgc 9900

tgcgcatagt ttttctgtaa tttacaacag tgctattttc tggtagttct tcggagtgtg 9960

ttgctttaat tattaaattt atataatcaa tgaatttggg atcgtcggtt ttgtacaata 10020

tgttgccggc atagtacgca gcttcttcta gttcaattac accatttttt agcagcaccg 10080

gattaacata actttccaaa atgttgtacg aaccgttaaa caaaaacagt tcacctccct 10140

tttctatact attgtctgcg agcagttgtt tgttgttaaa aataacagcc attgtaatga 10200

gacgcacaaa ctaatatcac aaactggaaa tgtctatcaa tatatagttg ctctagttat 10260

taatagtaat caattacggg gtcattagtt catagcccat atatggagtt ccgcgttaca 10320

taacttacgg taaatggccc gcctggctga ccgcccaacg acccccgccc attgacgtca 10380

ataatgacgt atgttcccat agtaacgcca atagggactt tccattgacg tcaatgggtg 10440

gactatttac ggtaaactgc ccacttggca gtacatcaag tgtatcatat gccaagtacg 10500

ccccctattg acgtcaatga cggtaaatgg cccgcctggc attatgccca gtacatgacc 10560

ttatgggact ttcctacttg gcagtacatc tacgtattag tcatcgctat taccatgcat 10620

ggtcgaggtg agccccacgt tctgcttcac tctccccatc tcccccccct ccccaccccc 10680

aattttgtat ttatttattt tttaattatt ttgtgcagcg atgggggcgg gggggggggg 10740

ggggcgcgcg ccaggcgggg cggggcgggg cgaggggcgg ggcggggcga ggcggagagg 10800

tgcggcggca gccaatcaga gcggcgcgct ccgaaagttt ccttttatgg cgaggcggcg 10860

gcggcggcgg ccctataaaa agcgaagcgc gcggcgggcg 10900

<210> 7
<211> 1771
<212> PRT
<213> Artificial Sequence

<220>
<223> LAMB4-His

<400> 7

Met Gln Phe Gln Leu Thr Leu Phe Leu His Leu Gly Trp Leu Ser Tyr
1               5                   10                  15

Ser Lys Ala Gln Asp Asp Cys Asn Arg Gly Ala Cys His Pro Thr Thr
            20                  25                  30

Gly Asp Leu Leu Val Gly Arg Asn Thr Gln Leu Met Ala Ser Ser Thr
        35                  40                  45

Cys Gly Leu Ser Arg Ala Gln Lys Tyr Cys Ile Leu Ser Tyr Leu Glu
    50                  55                  60

Gly Glu Gln Lys Cys Phe Ile Cys Asp Ser Arg Phe Pro Tyr Asp Pro
65                  70                  75                  80

Tyr Asp Gln Pro Asn Ser His Thr Ile Glu Asn Val Ile Val Ser Phe
                85                  90                  95

Glu Pro Asp Arg Glu Lys Lys Trp Trp Gln Ser Glu Asn Gly Leu Asp
            100                 105                 110

His Val Ser Ile Arg Leu Asp Leu Glu Ala Leu Phe Arg Phe Ser His
        115                 120                 125

Leu Ile Leu Thr Phe Lys Thr Phe Arg Pro Ala Ala Met Leu Val Glu
    130                 135                 140

Arg Ser Thr Asp Tyr Gly His Asn Trp Lys Val Phe Lys Tyr Phe Ala
145                 150                 155                 160

Lys Asp Cys Ala Thr Ser Phe Pro Asn Ile Thr Ser Gly Gln Ala Gln
                165                 170                 175

Gly Val Gly Asp Ile Val Cys Asp Ser Lys Tyr Ser Asp Ile Glu Pro
            180                 185                 190

Ser Thr Gly Gly Glu Val Val Leu Lys Val Leu Asp Pro Ser Phe Glu
        195                 200                 205

Ile Glu Asn Pro Tyr Ser Pro Tyr Ile Gln Asp Leu Val Thr Leu Thr
    210                 215                 220

Asn Leu Arg Ile Asn Phe Thr Lys Leu His Thr Leu Gly Asp Ala Leu
225                 230                 235                 240

Leu Gly Arg Arg Gln Asn Asp Ser Leu Asp Lys Tyr Tyr Tyr Ala Leu
                245                 250                 255

Tyr Glu Met Ile Val Arg Gly Ser Cys Phe Cys Asn Gly His Ala Ser
        260                 265                 270

Glu Cys Arg Pro Met Gln Lys Met Arg Gly Asp Val Phe Ser Pro Pro
    275                 280                 285

Gly Met Val His Gly Gln Cys Val Cys Gln His Asn Thr Asp Gly Pro
290                 295                 300

Asn Cys Glu Arg Cys Lys Asp Phe Phe Gln Asp Ala Pro Trp Arg Pro
305                 310                 315                 320

Ala Ala Asp Leu Gln Asp Asn Ala Cys Arg Ser Cys Ser Cys Asn Ser
                325                 330                 335

His Ser Ser Arg Cys His Phe Asp Met Thr Thr Tyr Leu Ala Ser Gly
            340                 345                 350

Gly Leu Ser Gly Gly Val Cys Glu Asp Cys Gln His Asn Thr Glu Gly
        355                 360                 365

Gln His Cys Asp Arg Cys Arg Pro Leu Phe Tyr Arg Asp Pro Leu Lys
    370                 375                 380

Thr Ile Ser Asp Pro Tyr Ala Cys Ile Pro Cys Glu Cys Asp Pro Asp
385                 390                 395                 400

Gly Thr Ile Ser Gly Gly Ile Cys Val Ser His Ser Asp Pro Ala Leu
                405                 410                 415

Gly Ser Val Ala Gly Gln Cys Leu Cys Lys Glu Asn Val Glu Gly Ala
            420                 425                 430

Lys Cys Asp Gln Cys Lys Pro Asn His Tyr Gly Leu Ser Ala Thr Asp
        435                 440                 445

Pro Leu Gly Cys Gln Pro Cys Asp Cys Asn Pro Leu Gly Ser Leu Pro
    450                 455                 460

Phe Leu Thr Cys Asp Val Asp Thr Gly Gln Cys Leu Cys Leu Ser Tyr
465                 470                 475                 480

Val Thr Gly Ala His Cys Glu Glu Cys Thr Val Gly Tyr Trp Gly Leu
                485                 490                 495

Gly Asn His Leu His Gly Cys Ser Pro Cys Asp Cys Asp Ile Gly Gly
            500                 505                 510

Ala Tyr Ser Asn Val Cys Ser Pro Lys Asn Gly Gln Cys Glu Cys Arg
        515                 520                 525

Pro His Val Thr Gly Arg Ser Cys Ser Glu Pro Ala Pro Gly Tyr Phe
    530                 535                 540

Phe Ala Pro Leu Asn Phe Tyr Leu Tyr Glu Ala Glu Glu Ala Thr Thr
545                 550                 555                 560

Leu Gln Gly Leu Ala Pro Leu Gly Ser Glu Thr Phe Gly Gln Ser Pro
                565                 570                 575

Ala Val His Val Val Leu Gly Glu Pro Val Pro Gly Asn Pro Val Thr
            580                 585                 590

Trp Thr Gly Pro Gly Phe Ala Arg Val Leu Pro Gly Ala Gly Leu Arg
        595                 600                 605

Phe Ala Val Asn Asn Ile Pro Phe Pro Val Asp Phe Thr Ile Ala Ile
    610                 615                 620

His Tyr Glu Thr Gln Ser Ala Ala Asp Trp Thr Val Gln Ile Val Val
625                 630                 635                 640

Asn Pro Pro Gly Gly Ser Glu His Cys Ile Pro Lys Thr Leu Gln Ser
                645                 650                 655

Lys Pro Gln Ser Phe Ala Leu Pro Ala Ala Thr Arg Ile Met Leu Leu
            660                 665                 670

Pro Thr Pro Ile Cys Leu Glu Pro Asp Val Gln Tyr Ser Ile Asp Val
        675                 680                 685

Tyr Phe Ser Gln Pro Leu Gln Gly Glu Ser His Ala His Ser His Val
    690                 695                 700

Leu Val Asp Ser Leu Gly Leu Ile Pro Gln Ile Asn Ser Leu Glu Asn
705                 710                 715                 720

Phe Cys Ser Lys Gln Asp Leu Asp Glu Tyr Gln Leu His Asn Cys Val
                725                 730                 735

Glu Ile Ala Ser Ala Met Gly Pro Gln Val Leu Pro Gly Ala Cys Glu
            740                 745                 750

Arg Leu Ile Ile Ser Met Ser Ala Lys Leu His Asp Gly Ala Val Ala

        755                 760                 765

Cys Lys Cys His Pro Gln Gly Ser Val Gly Ser Ser Cys Ser Arg Leu
    770                 775                 780

Gly Gly Gln Cys Gln Cys Lys Pro Leu Val Val Gly Arg Cys Cys Asp
785                 790                 795                 800

Arg Cys Ser Thr Gly Ser Tyr Asp Leu Gly His His Gly Cys His Pro
                805                 810                 815

Cys His Cys His Pro Gln Gly Ser Lys Asp Thr Val Cys Asp Gln Val
            820                 825                 830

Thr Gly Gln Cys Pro Cys His Gly Glu Val Ser Gly Arg Arg Cys Asp
        835                 840                 845

Arg Cys Leu Ala Gly Tyr Phe Gly Phe Pro Ser Cys His Pro Cys Pro
    850                 855                 860

Cys Asn Arg Phe Ala Glu Leu Cys Asp Pro Glu Thr Gly Ser Cys Phe
865                 870                 875                 880

Asn Cys Gly Gly Phe Thr Thr Gly Arg Asn Cys Glu Arg Cys Ile Asp
                885                 890                 895

Gly Tyr Tyr Gly Asn Pro Ser Ser Gly Gln Pro Cys Arg Pro Cys Leu
            900                 905                 910

Cys Pro Asp Asp Pro Ser Ser Asn Gln Tyr Phe Ala His Ser Cys Tyr
        915                 920                 925

Gln Asn Leu Trp Ser Ser Asp Val Ile Cys Asn Cys Leu Gln Gly Tyr
    930                 935                 940

Thr Gly Thr Gln Cys Gly Glu Cys Ser Thr Gly Phe Tyr Gly Asn Pro
945                 950                 955                 960

Arg Ile Ser Gly Ala Pro Cys Gln Pro Cys Ala Cys Asn Asn Asn Ile
                965                 970                 975

Asp Val Thr Asp Pro Glu Ser Cys Ser Arg Val Thr Gly Glu Cys Leu
            980                 985                 990

Arg Cys Leu His Asn Thr Gln Gly Ala Asn Cys Gln Leu Cys Lys Pro
        995                 1000                1005

Gly His Tyr Gly Ser Ala Leu Asn Gln Thr Cys Arg Arg Cys Ser
    1010                1015                1020

Cys His Ala Ser Gly Val Ser Pro Met Glu Cys Pro Pro Gly Gly
    1025                1030                1035

Gly Ala Cys Leu Cys Asp Pro Val Thr Gly Ala Cys Pro Cys Leu
    1040                1045                1050

Pro Asn Val Thr Gly Leu Ala Cys Asp Arg Cys Ala Asp Gly Tyr
    1055                1060                1065

Trp Asn Leu Val Pro Gly Arg Gly Cys Gln Ser Cys Asp Cys Asp
    1070                1075                1080

Pro Arg Thr Ser Gln Ser Ser His Cys Asp Gln Leu Thr Gly Gln
    1085                1090                1095

Cys Pro Cys Lys Leu Gly Tyr Gly Gly Lys Arg Cys Ser Glu Cys
    1100                1105                1110

Gln Glu Asn Tyr Tyr Gly Asp Pro Pro Gly Arg Cys Ile Pro Cys
    1115                1120                1125

Asp Cys Asn Arg Ala Gly Thr Gln Lys Pro Ile Cys Asp Pro Asp
    1130                1135                1140

Thr Gly Met Cys Arg Cys Arg Glu Gly Val Ser Gly Gln Arg Cys
    1145                1150                1155

Asp Arg Cys Ala Arg Gly His Ser Gln Glu Phe Pro Thr Cys Leu
    1160                1165                1170

Gln Cys His Leu Cys Phe Asp Gln Trp Asp His Thr Ile Ser Ser
    1175                1180                1185

Leu Ser Lys Ala Val Gln Gly Leu Met Arg Leu Ala Ala Asn Met
    1190                1195                1200

Glu Asp Lys Arg Glu Thr Leu Pro Val Cys Glu Ala Asp Phe Lys
    1205                1210                1215

Asp Leu Arg Gly Asn Val Ser Glu Ile Glu Arg Ile Leu Lys His
    1220                1225                1230

Pro Val Phe Pro Ser Gly Lys Phe Leu Lys Val Lys Asp Tyr His
    1235                1240                1245

Asp Ser Val Arg Arg Gln Ile Met Gln Leu Asn Glu Gln Leu Lys
    1250                1255                1260

Ala Val Tyr Glu Phe Gln Asp Leu Lys Asp Thr Ile Glu Arg Ala
    1265                1270                1275

Lys Asn Glu Ala Asp Leu Leu Leu Glu Asp Leu Gln Glu Glu Ile
    1280                1285                1290

Asp Leu Gln Ser Ser Val Leu Asn Ala Ser Ile Ala Asp Ser Ser
    1295                1300                1305

Glu Asn Ile Lys Lys Tyr Tyr His Ile Ser Ser Ser Ala Glu Lys
    1310                1315                1320

Lys Ile Asn Glu Thr Ser Ser Thr Ile Asn Thr Ser Ala Asn Thr
    1325                1330                1335

Arg Asn Asp Leu Leu Thr Ile Leu Asp Thr Leu Thr Ser Lys Gly
    1340                1345                1350

Asn Leu Ser Leu Glu Arg Leu Lys Gln Ile Lys Ile Pro Asp Ile
    1355                1360                1365

Gln Ile Leu Asn Glu Lys Val Cys Gly Asp Pro Gly Asn Val Pro
    1370                1375                1380

Cys Val Pro Leu Pro Cys Gly Gly Ala Leu Cys Thr Gly Arg Lys
    1385                1390                1395

Gly His Arg Lys Cys Arg Gly Pro Gly Cys His Gly Ser Leu Thr
    1400                1405                1410

Leu Ser Thr Asn Ala Leu Gln Lys Ala Gln Glu Ala Lys Ser Ile
    1415                1420                1425

Ile Arg Asn Leu Asp Lys Gln Val Arg Gly Leu Lys Asn Gln Ile
    1430                1435                1440

Glu Ser Ile Ser Glu Gln Ala Glu Val Ser Lys Asn Asn Ala Leu
    1445                1450                1455

Gln Leu Arg Glu Lys Leu Gly Asn Ile Arg Asn Gln Ser Asp Ser
    1460                1465                1470

Glu Glu Glu Asn Ile Asn Leu Phe Ile Lys Lys Val Lys Asn Phe
    1475                1480                1485

Leu Leu Glu Glu Asn Val Pro Pro Glu Asp Ile Glu Lys Val Ala
    1490                1495                1500

Asn Gly Val Leu Asp Ile His Leu Pro Ile Pro Ser Gln Asn Leu
    1505                1510                1515

Thr Asp Glu Leu Val Lys Ile Gln Lys His Met Gln Leu Cys Glu
    1520                1525                1530

Asp Tyr Arg Thr Asp Glu Asn Arg Leu Asn Glu Glu Ala Asp Gly
    1535                1540                1545

Ala Gln Lys Leu Leu Val Lys Ala Lys Ala Ala Glu Lys Ala Ala
    1550                1555                1560

Asn Ile Leu Leu Asn Leu Asp Lys Thr Leu Asn Gln Leu Gln Gln
    1565                1570                1575

Ala Gln Ile Thr Gln Gly Arg Ala Asn Ser Thr Ile Thr Gln Leu
    1580                1585                1590

Thr Ala Asn Ile Thr Lys Ile Lys Lys Asn Val Leu Gln Ala Glu
    1595                1600                1605

Asn Gln Thr Arg Glu Met Lys Ser Glu Leu Glu Leu Ala Lys Gln
    1610                1615                1620

Arg Ser Gly Leu Glu Asp Gly Leu Ser Leu Leu Gln Thr Lys Leu
    1625                1630                1635

Gln Arg His Gln Asp His Ala Val Asn Ala Lys Val Gln Ala Glu
    1640                1645                1650

Ser Ala Gln His Gln Ala Gly Ser Leu Glu Lys Glu Phe Val Glu
    1655                1660                1665

Leu Lys Lys Gln Tyr Ala Ile Leu Gln Arg Lys Thr Ser Thr Thr
    1670                1675                1680

Gly Leu Thr Lys Glu Thr Leu Gly Lys Val Lys Gln Leu Lys Asp
    1685                1690                1695

Ala Ala Glu Lys Leu Ala Gly Asp Thr Glu Ala Lys Ile Arg Arg
    1700                1705                1710

Ile Thr Asp Leu Glu Arg Lys Ile Gln Asp Leu Asn Leu Ser Arg

1715                    1720                    1725

Gln Ala  Lys Ala Asp Gln Leu  Arg Ile Leu Glu Asp  Gln Val Val
    1730                    1735                    1740

Ala Ile  Lys Asn Glu Ile Val  Glu Gln Glu Lys Lys  Tyr Ala Arg
    1745                    1750                    1755

Cys Tyr  Ser Leu Glu His His  His His His His His  His
    1760                    1765                    1770

<210>  8
<211>  5
<212>  PRT
<213>  Artificial Sequence

<220>
<223>  Autoantikörper Epitop 1

<400>  8

Glu Phe Gln Asp Leu
1               5

<210>  9
<211>  10
<212>  PRT
<213>  Artificial Sequence

<220>
<223>  Autoantikörper-Epitop 2

<400>  9

Ala Asp Leu Leu Leu Glu Asp Leu Gln Glu
1               5                   10

<210>  10
<211>  16
<212>  PRT
<213>  Artificial Sequence

<220>
<223>  Autoantikörper-Epitop 3

<400>  10

Ala Asp Leu Leu Leu Glu Asp Leu Gln Glu Glu Ile Asp Leu Gln Ser
1               5                   10                  15

<210>  11
<211>  12
<212>  PRT
<213>  Artificial Sequence

<220>

<223>  Autoantikörper-Epitop 4

<400>  11

Asp Leu Leu Thr Ile Leu Asp Thr Leu Thr Ser Lys
1               5                   10


<210>  12
<211>  13
<212>  PRT
<213>  Artificial Sequence

<220>
<223>  Autoantikörper-Epitop 5

<400>  12

Gln Ile Lys Ile Pro Asp Ile Gln Ile Leu Asn Glu Lys
1               5                   10


<210>  13
<211>  13
<212>  PRT
<213>  Artificial Sequence

<220>
<223>  Autoantikörper-Epitop 6

<400>  13

Val Arg Gly Leu Lys Asn Gln Ile Glu Ser Ile Ser Glu
1               5                   10


<210>  14
<211>  11
<212>  PRT
<213>  Artificial Sequence

<220>
<223>  Autoantikörper-Epitop 7

<400>  14

Leu Leu Glu Glu Asn Val Pro Pro Glu Asp Ile
1               5                   10


<210>  15
<211>  300
<212>  PRT
<213>  Artificial Sequence

<220>
<223>  Teilfragment 1

<400>  15

Met Gln Asp Asp Cys Asn Arg Gly Ala Cys His Pro Thr Thr Gly Asp
1               5                   10                  15

Leu Leu Val Gly Arg Asn Thr Gln Leu Met Ala Ser Ser Thr Cys Gly
            20                  25                  30

Leu Ser Arg Ala Gln Lys Tyr Cys Ile Leu Ser Tyr Leu Glu Gly Glu
        35                  40                  45

Gln Lys Cys Phe Ile Cys Asp Ser Arg Phe Pro Tyr Asp Pro Tyr Asp
    50                  55                  60

Gln Pro Asn Ser His Thr Ile Glu Asn Val Ile Val Ser Phe Glu Pro
65                  70                  75                  80

Asp Arg Glu Lys Lys Trp Trp Gln Ser Glu Asn Gly Leu Asp His Val
                85                  90                  95

Ser Ile Arg Leu Asp Leu Glu Ala Leu Phe Arg Phe Ser His Leu Ile
            100                 105                 110

Leu Thr Phe Lys Thr Phe Arg Pro Ala Ala Met Leu Val Glu Arg Ser
        115                 120                 125

Thr Asp Tyr Gly His Asn Trp Lys Val Phe Lys Tyr Phe Ala Lys Asp
    130                 135                 140

Cys Ala Thr Ser Phe Pro Asn Ile Thr Ser Gly Gln Ala Gln Gly Val
145                 150                 155                 160

Gly Asp Ile Val Cys Asp Ser Lys Tyr Ser Asp Ile Glu Pro Ser Thr
                165                 170                 175

Gly Gly Glu Val Val Leu Lys Val Leu Asp Pro Ser Phe Glu Ile Glu
            180                 185                 190

Asn Pro Tyr Ser Pro Tyr Ile Gln Asp Leu Val Thr Leu Thr Asn Leu
        195                 200                 205

Arg Ile Asn Phe Thr Lys Leu His Thr Leu Gly Asp Ala Leu Leu Gly
    210                 215                 220

Arg Arg Gln Asn Asp Ser Leu Asp Lys Tyr Tyr Tyr Ala Leu Tyr Glu
225                 230                 235                 240

Met Ile Val Arg Gly Ser Cys Phe Cys Asn Gly His Ala Ser Glu Cys
                245                 250                 255

Arg Pro Met Gln Lys Met Arg Gly Asp Val Phe Ser Pro Pro Gly Met
            260                 265                 270

Val His Gly Gln Cys Val Cys Gln His Asn Thr Asp Gly Pro Asn Cys
        275                 280                 285

Glu Arg Cys Lys Asp Phe Phe Gln Asp Ala Pro Trp
    290                 295                 300

<210>  16
<211>  301
<212>  PRT
<213>  Artificial Sequence

<220>
<223>  Teilfragment 2

<400>  16

Met Pro Asn Cys Glu Arg Cys Lys Asp Phe Phe Gln Asp Ala Pro Trp
1               5                   10                  15

Arg Pro Ala Ala Asp Leu Gln Asp Asn Ala Cys Arg Ser Cys Ser Cys
            20                  25                  30

Asn Ser His Ser Ser Arg Cys His Phe Asp Met Thr Thr Tyr Leu Ala
        35                  40                  45

Ser Gly Gly Leu Ser Gly Gly Val Cys Glu Asp Cys Gln His Asn Thr
    50                  55                  60

Glu Gly Gln His Cys Asp Arg Cys Arg Pro Leu Phe Tyr Arg Asp Pro
65                  70                  75                  80

Leu Lys Thr Ile Ser Asp Pro Tyr Ala Cys Ile Pro Cys Glu Cys Asp
                85                  90                  95

Pro Asp Gly Thr Ile Ser Gly Gly Ile Cys Val Ser His Ser Asp Pro
            100                 105                 110

Ala Leu Gly Ser Val Ala Gly Gln Cys Leu Cys Lys Glu Asn Val Glu
        115                 120                 125

Gly Ala Lys Cys Asp Gln Cys Lys Pro Asn His Tyr Gly Leu Ser Ala
    130                 135                 140

Thr Asp Pro Leu Gly Cys Gln Pro Cys Asp Cys Asn Pro Leu Gly Ser
145                 150                 155                 160

Leu Pro Phe Leu Thr Cys Asp Val Asp Thr Gly Gln Cys Leu Cys Leu
                165                 170                 175

Ser Tyr Val Thr Gly Ala His Cys Glu Glu Cys Thr Val Gly Tyr Trp
            180                 185                 190

Gly Leu Gly Asn His Leu His Gly Cys Ser Pro Cys Asp Cys Asp Ile
        195                 200                 205

Gly Gly Ala Tyr Ser Asn Val Cys Ser Pro Lys Asn Gly Gln Cys Glu
    210                 215                 220

Cys Arg Pro His Val Thr Gly Arg Ser Cys Ser Glu Pro Ala Pro Gly
225                 230                 235                 240

Tyr Phe Phe Ala Pro Leu Asn Phe Tyr Leu Tyr Glu Ala Glu Glu Ala
                245                 250                 255

Thr Thr Leu Gln Gly Leu Ala Pro Leu Gly Ser Glu Thr Phe Gly Gln
            260                 265                 270

Ser Pro Ala Val His Val Val Leu Gly Glu Pro Val Pro Gly Asn Pro
        275                 280                 285

Val Thr Trp Thr Gly Pro Gly Phe Ala Arg Val Leu Trp
    290                 295                 300

<210> 17
<211> 318
<212> PRT
<213> Artificial Sequence

<220>
<223> Teilfragment 3

<400> 17

Met Gly Asn Pro Val Thr Trp Thr Gly Pro Gly Phe Ala Arg Val Leu
1               5                   10                  15

Pro Gly Ala Gly Leu Arg Phe Ala Val Asn Asn Ile Pro Phe Pro Val
            20                  25                  30

Asp Phe Thr Ile Ala Ile His Tyr Glu Thr Gln Ser Ala Ala Asp Trp
        35                  40                  45

Thr Val Gln Ile Val Val Asn Pro Pro Gly Gly Ser Glu His Cys Ile
    50                  55                  60

Pro Lys Thr Leu Gln Ser Lys Pro Gln Ser Phe Ala Leu Pro Ala Ala
65                  70                  75                  80

Thr Arg Ile Met Leu Leu Pro Thr Pro Ile Cys Leu Glu Pro Asp Val
                85                  90                  95

Gln Tyr Ser Ile Asp Val Tyr Phe Ser Gln Pro Leu Gln Gly Glu Ser
            100                 105                 110

His Ala His Ser His Val Leu Val Asp Ser Leu Gly Leu Ile Pro Gln
        115                 120                 125

Ile Asn Ser Leu Glu Asn Phe Cys Ser Lys Gln Asp Leu Asp Glu Tyr
    130                 135                 140

Gln Leu His Asn Cys Val Glu Ile Ala Ser Ala Met Gly Pro Gln Val
145                 150                 155                 160

Leu Pro Gly Ala Cys Glu Arg Leu Ile Ile Ser Met Ser Ala Lys Leu
                165                 170                 175

His Asp Gly Ala Val Ala Cys Lys Cys His Pro Gln Gly Ser Val Gly
            180                 185                 190

Ser Ser Cys Ser Arg Leu Gly Gly Gln Cys Gln Cys Lys Pro Leu Val
        195                 200                 205

Val Gly Arg Cys Cys Asp Arg Cys Ser Thr Gly Ser Tyr Asp Leu Gly
    210                 215                 220

His His Gly Cys His Pro Cys His Cys His Pro Gln Gly Ser Lys Asp
225                 230                 235                 240

Thr Val Cys Asp Gln Val Thr Gly Gln Cys Pro Cys His Gly Glu Val
                245                 250                 255

Ser Gly Arg Arg Cys Asp Arg Cys Leu Ala Gly Tyr Phe Gly Phe Pro
            260                 265                 270

Ser Cys His Pro Cys Pro Cys Asn Arg Phe Ala Glu Leu Cys Asp Pro
        275                 280                 285

Glu Thr Gly Ser Cys Phe Asn Cys Gly Gly Phe Thr Thr Gly Arg Asn
    290                 295                 300

Cys Glu Arg Cys Ile Asp Gly Tyr Tyr Gly Asn Trp Gly Trp
305                 310                 315

<210>  18
<211>  286
<212>  PRT

<213>  Artificial Sequence

<220>
<223>  Teilfragment 4

<400>  18

Met Thr Gly Arg Asn Cys Glu Arg Cys Ile Asp Gly Tyr Tyr Gly Asn
1               5                   10                  15

Pro Ser Ser Gly Gln Pro Cys Arg Pro Cys Leu Cys Pro Asp Asp Pro
            20                  25                  30

Ser Ser Asn Gln Tyr Phe Ala His Ser Cys Tyr Gln Asn Leu Trp Ser
        35                  40                  45

Ser Asp Val Ile Cys Asn Cys Leu Gln Gly Tyr Thr Gly Thr Gln Cys
    50                  55                  60

Gly Glu Cys Ser Thr Gly Phe Tyr Gly Asn Pro Arg Ile Ser Gly Ala
65                  70                  75                  80

Pro Cys Gln Pro Cys Ala Cys Asn Asn Asn Ile Asp Val Thr Asp Pro
                85                  90                  95

Glu Ser Cys Ser Arg Val Thr Gly Glu Cys Leu Arg Cys Leu His Asn
            100                 105                 110

Thr Gln Gly Ala Asn Cys Gln Leu Cys Lys Pro Gly His Tyr Gly Ser
        115                 120                 125

Ala Leu Asn Gln Thr Cys Arg Arg Cys Ser Cys His Ala Ser Gly Val
    130                 135                 140

Ser Pro Met Glu Cys Pro Pro Gly Gly Gly Ala Cys Leu Cys Asp Pro
145                 150                 155                 160

Val Thr Gly Ala Cys Pro Cys Leu Pro Asn Val Thr Gly Leu Ala Cys
                165                 170                 175

Asp Arg Cys Ala Asp Gly Tyr Trp Asn Leu Val Pro Gly Arg Gly Cys
            180                 185                 190

Gln Ser Cys Asp Cys Asp Pro Arg Thr Ser Gln Ser Ser His Cys Asp
        195                 200                 205

Gln Leu Thr Gly Gln Cys Pro Cys Lys Leu Gly Tyr Gly Gly Lys Arg
    210                 215                 220

Cys Ser Glu Cys Gln Glu Asn Tyr Tyr Gly Asp Pro Pro Gly Arg Cys
225                 230                 235                 240

Ile Pro Cys Asp Cys Asn Arg Ala Gly Thr Gln Lys Pro Ile Cys Asp
                245                 250                 255

Pro Asp Thr Gly Met Cys Arg Cys Arg Glu Gly Val Ser Gly Gln Arg
            260                 265                 270

Cys Asp Arg Cys Ala Arg Gly His Ser Gln Glu Trp Gly Trp
        275                 280                 285

<210>  19
<211>  320
<212>  PRT
<213>  Artificial Sequence

<220>
<223>  Teilfragment 5

<400>  19

Met Ser Gly Gln Arg Cys Asp Arg Cys Ala Arg Gly His Ser Gln Glu
1               5                   10                  15

Phe Pro Thr Cys Leu Gln Cys His Leu Cys Phe Asp Gln Trp Asp His
            20                  25                  30

Thr Ile Ser Ser Leu Ser Lys Ala Val Gln Gly Leu Met Arg Leu Ala
        35                  40                  45

Ala Asn Met Glu Asp Lys Arg Glu Thr Leu Pro Val Cys Glu Ala Asp
    50                  55                  60

Phe Lys Asp Leu Arg Gly Asn Val Ser Glu Ile Glu Arg Ile Leu Lys
65                  70                  75                  80

His Pro Val Phe Pro Ser Gly Lys Phe Leu Lys Val Lys Asp Tyr His
                85                  90                  95

Asp Ser Val Arg Arg Gln Ile Met Gln Leu Asn Glu Gln Leu Lys Ala
            100                 105                 110

Val Tyr Glu Phe Gln Asp Leu Lys Asp Thr Ile Glu Arg Ala Lys Asn
        115                 120                 125

Glu Ala Asp Leu Leu Leu Glu Asp Leu Gln Glu Glu Ile Asp Leu Gln
    130                 135                 140

Ser Ser Val Leu Asn Ala Ser Ile Ala Asp Ser Ser Glu Asn Ile Lys

145                 150                 155                 160

Lys Tyr Tyr His Ile Ser Ser Ser Ala Glu Lys Lys Ile Asn Glu Thr
                165                 170                 175

Ser Ser Thr Ile Asn Thr Ser Ala Asn Thr Arg Asn Asp Leu Leu Thr
            180                 185                 190

Ile Leu Asp Thr Leu Thr Ser Lys Gly Asn Leu Ser Leu Glu Arg Leu
        195                 200                 205

Lys Gln Ile Lys Ile Pro Asp Ile Gln Ile Leu Asn Glu Lys Val Cys
    210                 215                 220

Gly Asp Pro Gly Asn Val Pro Cys Val Pro Leu Pro Cys Gly Gly Ala
225                 230                 235                 240

Leu Cys Thr Gly Arg Lys Gly His Arg Lys Cys Arg Gly Pro Gly Cys
                245                 250                 255

His Gly Ser Leu Thr Leu Ser Thr Asn Ala Leu Gln Lys Ala Gln Glu
            260                 265                 270

Ala Lys Ser Ile Ile Arg Asn Leu Asp Lys Gln Val Arg Gly Leu Lys
        275                 280                 285

Asn Gln Ile Glu Ser Ile Ser Glu Gln Ala Glu Val Ser Lys Asn Asn
    290                 295                 300

Ala Leu Gln Leu Arg Glu Lys Leu Gly Asn Ile Trp Gly Trp Gly Trp
305                 310                 315                 320

<210>  20
<211>  317
<212>  PRT
<213>  Artificial Sequence

<220>
<223>  Teilfragment 6

<400>  20

Met Ser Lys Asn Asn Ala Leu Gln Leu Arg Glu Lys Leu Gly Asn Ile
1               5                   10                  15

Arg Asn Gln Ser Asp Ser Glu Glu Glu Asn Ile Asn Leu Phe Ile Lys
            20                  25                  30

Lys Val Lys Asn Phe Leu Leu Glu Glu Asn Val Pro Pro Glu Asp Ile
        35                  40                  45

Glu Lys Val Ala Asn Gly Val Leu Asp Ile His Leu Pro Ile Pro Ser
    50                  55                  60

Gln Asn Leu Thr Asp Glu Leu Val Lys Ile Gln Lys His Met Gln Leu
65                  70                  75                  80

Cys Glu Asp Tyr Arg Thr Asp Glu Asn Arg Leu Asn Glu Glu Ala Asp
                85                  90                  95

Gly Ala Gln Lys Leu Leu Val Lys Ala Lys Ala Ala Glu Lys Ala Ala
            100                 105                 110

Asn Ile Leu Leu Asn Leu Asp Lys Thr Leu Asn Gln Leu Gln Gln Ala
        115                 120                 125

Gln Ile Thr Gln Gly Arg Ala Asn Ser Thr Ile Thr Gln Leu Thr Ala
    130                 135                 140

Asn Ile Thr Lys Ile Lys Lys Asn Val Leu Gln Ala Glu Asn Gln Thr
145                 150                 155                 160

Arg Glu Met Lys Ser Glu Leu Glu Leu Ala Lys Gln Arg Ser Gly Leu
                165                 170                 175

Glu Asp Gly Leu Ser Leu Leu Gln Thr Lys Leu Gln Arg His Gln Asp
            180                 185                 190

His Ala Val Asn Ala Lys Val Gln Ala Glu Ser Ala Gln His Gln Ala
        195                 200                 205

Gly Ser Leu Glu Lys Glu Phe Val Glu Leu Lys Lys Gln Tyr Ala Ile
    210                 215                 220

Leu Gln Arg Lys Thr Ser Thr Thr Gly Leu Thr Lys Glu Thr Leu Gly
225                 230                 235                 240

Lys Val Lys Gln Leu Lys Asp Ala Ala Glu Lys Leu Ala Gly Asp Thr
                245                 250                 255

Glu Ala Lys Ile Arg Arg Ile Thr Asp Leu Glu Arg Lys Ile Gln Asp
            260                 265                 270

Leu Asn Leu Ser Arg Gln Ala Lys Ala Asp Gln Leu Arg Ile Leu Glu
        275                 280                 285

Asp Gln Val Val Ala Ile Lys Asn Glu Ile Val Glu Gln Glu Lys Lys

290                 295                 300

Tyr Ala Arg Cys Tyr Ser Trp Gly Trp Gly Trp Gly Trp
305                 310                 315

<210>  21
<211>  308
<212>  PRT
<213>  Artificial Sequence

<220>
<223>  Teilfragment 1, mit vier Tryptophanen und C-terminalem His-Tag
       (AMB4-TF1-4W-His)

<400>  21

Met Gln Asp Asp Cys Asn Arg Gly Ala Cys His Pro Thr Thr Gly Asp
1               5                   10                  15

Leu Leu Val Gly Arg Asn Thr Gln Leu Met Ala Ser Ser Thr Cys Gly
            20                  25                  30

Leu Ser Arg Ala Gln Lys Tyr Cys Ile Leu Ser Tyr Leu Glu Gly Glu
        35                  40                  45

Gln Lys Cys Phe Ile Cys Asp Ser Arg Phe Pro Tyr Asp Pro Tyr Asp
    50                  55                  60

Gln Pro Asn Ser His Thr Ile Glu Asn Val Ile Val Ser Phe Glu Pro
65                  70                  75                  80

Asp Arg Glu Lys Lys Trp Trp Gln Ser Glu Asn Gly Leu Asp His Val
                85                  90                  95

Ser Ile Arg Leu Asp Leu Glu Ala Leu Phe Arg Phe Ser His Leu Ile
            100                 105                 110

Leu Thr Phe Lys Thr Phe Arg Pro Ala Ala Met Leu Val Glu Arg Ser
        115                 120                 125

Thr Asp Tyr Gly His Asn Trp Lys Val Phe Lys Tyr Phe Ala Lys Asp
    130                 135                 140

Cys Ala Thr Ser Phe Pro Asn Ile Thr Ser Gly Gln Ala Gln Gly Val
145                 150                 155                 160

Gly Asp Ile Val Cys Asp Ser Lys Tyr Ser Asp Ile Glu Pro Ser Thr
                165                 170                 175

Gly Gly Glu Val Val Leu Lys Val Leu Asp Pro Ser Phe Glu Ile Glu

180                 185                 190

Asn Pro Tyr Ser Pro Tyr Ile Gln Asp Leu Val Thr Leu Thr Asn Leu
        195                 200                 205

Arg Ile Asn Phe Thr Lys Leu His Thr Leu Gly Asp Ala Leu Leu Gly
    210                 215                 220

Arg Arg Gln Asn Asp Ser Leu Asp Lys Tyr Tyr Tyr Ala Leu Tyr Glu
225                 230                 235                 240

Met Ile Val Arg Gly Ser Cys Phe Cys Asn Gly His Ala Ser Glu Cys
                245                 250                 255

Arg Pro Met Gln Lys Met Arg Gly Asp Val Phe Ser Pro Pro Gly Met
            260                 265                 270

Val His Gly Gln Cys Val Cys Gln His Asn Thr Asp Gly Pro Asn Cys
        275                 280                 285

Glu Arg Cys Lys Asp Phe Phe Gln Asp Ala Pro Trp Leu Glu His His
    290                 295                 300

His His His His
305

<210>  22
<211>  309
<212>  PRT
<213>  Artificial Sequence

<220>
<223>  Teilfragment 2, mit vier Tryptophanen und C-terminalem His-Tag
       (LAMB4-TF2-4W-His)

<400>  22

Met Pro Asn Cys Glu Arg Cys Lys Asp Phe Phe Gln Asp Ala Pro Trp
1               5                   10                  15

Arg Pro Ala Ala Asp Leu Gln Asp Asn Ala Cys Arg Ser Cys Ser Cys
            20                  25                  30

Asn Ser His Ser Ser Arg Cys His Phe Asp Met Thr Thr Tyr Leu Ala
        35                  40                  45

Ser Gly Gly Leu Ser Gly Gly Val Cys Glu Asp Cys Gln His Asn Thr
    50                  55                  60

Glu Gly Gln His Cys Asp Arg Cys Arg Pro Leu Phe Tyr Arg Asp Pro

65                  70                  75                  80

Leu Lys Thr Ile Ser Asp Pro Tyr Ala Cys Ile Pro Cys Glu Cys Asp
                85                  90                  95

Pro Asp Gly Thr Ile Ser Gly Gly Ile Cys Val Ser His Ser Asp Pro
            100                 105                 110

Ala Leu Gly Ser Val Ala Gly Gln Cys Leu Cys Lys Glu Asn Val Glu
        115                 120                 125

Gly Ala Lys Cys Asp Gln Cys Lys Pro Asn His Tyr Gly Leu Ser Ala
    130                 135                 140

Thr Asp Pro Leu Gly Cys Gln Pro Cys Asp Cys Asn Pro Leu Gly Ser
145                 150                 155                 160

Leu Pro Phe Leu Thr Cys Asp Val Asp Thr Gly Gln Cys Leu Cys Leu
                165                 170                 175

Ser Tyr Val Thr Gly Ala His Cys Glu Glu Cys Thr Val Gly Tyr Trp
            180                 185                 190

Gly Leu Gly Asn His Leu His Gly Cys Ser Pro Cys Asp Cys Asp Ile
        195                 200                 205

Gly Gly Ala Tyr Ser Asn Val Cys Ser Pro Lys Asn Gly Gln Cys Glu
    210                 215                 220

Cys Arg Pro His Val Thr Gly Arg Ser Cys Ser Glu Pro Ala Pro Gly
225                 230                 235                 240

Tyr Phe Phe Ala Pro Leu Asn Phe Tyr Leu Tyr Glu Ala Glu Glu Ala
                245                 250                 255

Thr Thr Leu Gln Gly Leu Ala Pro Leu Gly Ser Glu Thr Phe Gly Gln
            260                 265                 270

Ser Pro Ala Val His Val Val Leu Gly Glu Pro Val Pro Gly Asn Pro
        275                 280                 285

Val Thr Trp Thr Gly Pro Gly Phe Ala Arg Val Leu Trp Leu Glu His
    290                 295                 300

His His His His His
305

<210>  23
<211>  326
<212>  PRT
<213>  Artificial Sequence

<220>
<223>  Teilfragment 3, mit vier Tryptophanen und C-terminalem His-Tag
       (LAMB4-TF3-4W-His)

<400>  23

Met Gly Asn Pro Val Thr Trp Thr Gly Pro Gly Phe Ala Arg Val Leu
1               5                   10                  15

Pro Gly Ala Gly Leu Arg Phe Ala Val Asn Asn Ile Pro Phe Pro Val
            20                  25                  30

Asp Phe Thr Ile Ala Ile His Tyr Glu Thr Gln Ser Ala Ala Asp Trp
        35                  40                  45

Thr Val Gln Ile Val Val Asn Pro Pro Gly Gly Ser Glu His Cys Ile
    50                  55                  60

Pro Lys Thr Leu Gln Ser Lys Pro Gln Ser Phe Ala Leu Pro Ala Ala
65                  70                  75                  80

Thr Arg Ile Met Leu Leu Pro Thr Pro Ile Cys Leu Glu Pro Asp Val
                85                  90                  95

Gln Tyr Ser Ile Asp Val Tyr Phe Ser Gln Pro Leu Gln Gly Glu Ser
            100                 105                 110

His Ala His Ser His Val Leu Val Asp Ser Leu Gly Leu Ile Pro Gln
        115                 120                 125

Ile Asn Ser Leu Glu Asn Phe Cys Ser Lys Gln Asp Leu Asp Glu Tyr
    130                 135                 140

Gln Leu His Asn Cys Val Glu Ile Ala Ser Ala Met Gly Pro Gln Val
145                 150                 155                 160

Leu Pro Gly Ala Cys Glu Arg Leu Ile Ile Ser Met Ser Ala Lys Leu
                165                 170                 175

His Asp Gly Ala Val Ala Cys Lys Cys His Pro Gln Gly Ser Val Gly
            180                 185                 190

Ser Ser Cys Ser Arg Leu Gly Gly Gln Cys Gln Cys Lys Pro Leu Val
        195                 200                 205

Val Gly Arg Cys Cys Asp Arg Cys Ser Thr Gly Ser Tyr Asp Leu Gly
    210                 215                 220

His His Gly Cys His Pro Cys His Cys His Pro Gln Gly Ser Lys Asp
225                 230                 235                 240

Thr Val Cys Asp Gln Val Thr Gly Gln Cys Pro Cys His Gly Glu Val
                245                 250                 255

Ser Gly Arg Arg Cys Asp Arg Cys Leu Ala Gly Tyr Phe Gly Phe Pro
            260                 265                 270

Ser Cys His Pro Cys Pro Cys Asn Arg Phe Ala Glu Leu Cys Asp Pro
        275                 280                 285

Glu Thr Gly Ser Cys Phe Asn Cys Gly Gly Phe Thr Thr Gly Arg Asn
    290                 295                 300

Cys Glu Arg Cys Ile Asp Gly Tyr Tyr Gly Asn Trp Gly Trp Leu Glu
305                 310                 315                 320

His His His His His His
                325

<210>  24
<211>  294
<212>  PRT
<213>  Artificial Sequence

<220>
<223>  Teilfragment 4, mit vier Tryptophanen und C-terminalem His-Tag
       (LAMB4-TF4-4W-His)

<400>  24

Met Thr Gly Arg Asn Cys Glu Arg Cys Ile Asp Gly Tyr Tyr Gly Asn
1               5                   10                  15

Pro Ser Ser Gly Gln Pro Cys Arg Pro Cys Leu Cys Pro Asp Asp Pro
            20                  25                  30

Ser Ser Asn Gln Tyr Phe Ala His Ser Cys Tyr Gln Asn Leu Trp Ser
        35                  40                  45

Ser Asp Val Ile Cys Asn Cys Leu Gln Gly Tyr Thr Gly Thr Gln Cys
    50                  55                  60

Gly Glu Cys Ser Thr Gly Phe Tyr Gly Asn Pro Arg Ile Ser Gly Ala
65                  70                  75                  80

Pro Cys Gln Pro Cys Ala Cys Asn Asn Asn Ile Asp Val Thr Asp Pro
                85                  90                  95

Glu Ser Cys Ser Arg Val Thr Gly Glu Cys Leu Arg Cys Leu His Asn
            100                 105                 110

Thr Gln Gly Ala Asn Cys Gln Leu Cys Lys Pro Gly His Tyr Gly Ser
        115                 120                 125

Ala Leu Asn Gln Thr Cys Arg Arg Cys Ser Cys His Ala Ser Gly Val
    130                 135                 140

Ser Pro Met Glu Cys Pro Pro Gly Gly Gly Ala Cys Leu Cys Asp Pro
145                 150                 155                 160

Val Thr Gly Ala Cys Pro Cys Leu Pro Asn Val Thr Gly Leu Ala Cys
                165                 170                 175

Asp Arg Cys Ala Asp Gly Tyr Trp Asn Leu Val Pro Gly Arg Gly Cys
            180                 185                 190

Gln Ser Cys Asp Cys Asp Pro Arg Thr Ser Gln Ser Ser His Cys Asp
        195                 200                 205

Gln Leu Thr Gly Gln Cys Pro Cys Lys Leu Gly Tyr Gly Gly Lys Arg
    210                 215                 220

Cys Ser Glu Cys Gln Glu Asn Tyr Tyr Gly Asp Pro Pro Gly Arg Cys
225                 230                 235                 240

Ile Pro Cys Asp Cys Asn Arg Ala Gly Thr Gln Lys Pro Ile Cys Asp
                245                 250                 255

Pro Asp Thr Gly Met Cys Arg Cys Arg Glu Gly Val Ser Gly Gln Arg
            260                 265                 270

Cys Asp Arg Cys Ala Arg Gly His Ser Gln Glu Trp Gly Trp Leu Glu
        275                 280                 285

His His His His His His
    290

<210>  25
<211>  328
<212>  PRT
<213>  Artificial Sequence

<220>
<223>  Teilfragment 5, mit vier Tryptophanen und C-terminalem His-Tag

(LAMB4-TF5-4W-His)

<400>  25

Met Ser Gly Gln Arg Cys Asp Arg Cys Ala Arg Gly His Ser Gln Glu
1               5                   10                  15

Phe Pro Thr Cys Leu Gln Cys His Leu Cys Phe Asp Gln Trp Asp His
            20                  25                  30

Thr Ile Ser Ser Leu Ser Lys Ala Val Gln Gly Leu Met Arg Leu Ala
        35                  40                  45

Ala Asn Met Glu Asp Lys Arg Glu Thr Leu Pro Val Cys Glu Ala Asp
    50                  55                  60

Phe Lys Asp Leu Arg Gly Asn Val Ser Glu Ile Glu Arg Ile Leu Lys
65                  70                  75                  80

His Pro Val Phe Pro Ser Gly Lys Phe Leu Lys Val Lys Asp Tyr His
                85                  90                  95

Asp Ser Val Arg Arg Gln Ile Met Gln Leu Asn Glu Gln Leu Lys Ala
            100                 105                 110

Val Tyr Glu Phe Gln Asp Leu Lys Asp Thr Ile Glu Arg Ala Lys Asn
        115                 120                 125

Glu Ala Asp Leu Leu Leu Glu Asp Leu Gln Glu Glu Ile Asp Leu Gln
    130                 135                 140

Ser Ser Val Leu Asn Ala Ser Ile Ala Asp Ser Ser Glu Asn Ile Lys
145                 150                 155                 160

Lys Tyr Tyr His Ile Ser Ser Ser Ala Glu Lys Lys Ile Asn Glu Thr
                165                 170                 175

Ser Ser Thr Ile Asn Thr Ser Ala Asn Thr Arg Asn Asp Leu Leu Thr
            180                 185                 190

Ile Leu Asp Thr Leu Thr Ser Lys Gly Asn Leu Ser Leu Glu Arg Leu
        195                 200                 205

Lys Gln Ile Lys Ile Pro Asp Ile Gln Ile Leu Asn Glu Lys Val Cys
    210                 215                 220

Gly Asp Pro Gly Asn Val Pro Cys Val Pro Leu Pro Cys Gly Gly Ala
225                 230                 235                 240

Leu Cys Thr Gly Arg Lys Gly His Arg Lys Cys Arg Gly Pro Gly Cys
                245                 250                 255

His Gly Ser Leu Thr Leu Ser Thr Asn Ala Leu Gln Lys Ala Gln Glu
            260                 265                 270

Ala Lys Ser Ile Ile Arg Asn Leu Asp Lys Gln Val Arg Gly Leu Lys
        275                 280                 285

Asn Gln Ile Glu Ser Ile Ser Glu Gln Ala Glu Val Ser Lys Asn Asn
    290                 295                 300

Ala Leu Gln Leu Arg Glu Lys Leu Gly Asn Ile Trp Gly Trp Gly Trp
305                 310                 315                 320

Leu Glu His His His His His His
                325

<210>  26
<211>  325
<212>  PRT
<213>  Artificial Sequence

<220>
<223>  Teilfragment 6, mit vier Tryptophanen und C-terminalem His-Tag
       (LAMB4-TF6-4W-His)

<400>  26

Met Ser Lys Asn Asn Ala Leu Gln Leu Arg Glu Lys Leu Gly Asn Ile
1               5                   10                  15

Arg Asn Gln Ser Asp Ser Glu Glu Glu Asn Ile Asn Leu Phe Ile Lys
            20                  25                  30

Lys Val Lys Asn Phe Leu Leu Glu Glu Asn Val Pro Pro Glu Asp Ile
        35                  40                  45

Glu Lys Val Ala Asn Gly Val Leu Asp Ile His Leu Pro Ile Pro Ser
    50                  55                  60

Gln Asn Leu Thr Asp Glu Leu Val Lys Ile Gln Lys His Met Gln Leu
65                  70                  75                  80

Cys Glu Asp Tyr Arg Thr Asp Glu Asn Arg Leu Asn Glu Glu Ala Asp
                85                  90                  95

Gly Ala Gln Lys Leu Leu Val Lys Ala Lys Ala Ala Glu Lys Ala Ala
            100                 105                 110

Asn Ile Leu Leu Asn Leu Asp Lys Thr Leu Asn Gln Leu Gln Gln Ala
        115                 120                 125

Gln Ile Thr Gln Gly Arg Ala Asn Ser Thr Ile Thr Gln Leu Thr Ala
    130                 135                 140

Asn Ile Thr Lys Ile Lys Lys Asn Val Leu Gln Ala Glu Asn Gln Thr
145                 150                 155                 160

Arg Glu Met Lys Ser Glu Leu Glu Leu Ala Lys Gln Arg Ser Gly Leu
                165                 170                 175

Glu Asp Gly Leu Ser Leu Leu Gln Thr Lys Leu Gln Arg His Gln Asp
            180                 185                 190

His Ala Val Asn Ala Lys Val Gln Ala Glu Ser Ala Gln His Gln Ala
        195                 200                 205

Gly Ser Leu Glu Lys Glu Phe Val Glu Leu Lys Lys Gln Tyr Ala Ile
    210                 215                 220

Leu Gln Arg Lys Thr Ser Thr Thr Gly Leu Thr Lys Glu Thr Leu Gly
225                 230                 235                 240

Lys Val Lys Gln Leu Lys Asp Ala Ala Glu Lys Leu Ala Gly Asp Thr
                245                 250                 255

Glu Ala Lys Ile Arg Arg Ile Thr Asp Leu Glu Arg Lys Ile Gln Asp
            260                 265                 270

Leu Asn Leu Ser Arg Gln Ala Lys Ala Asp Gln Leu Arg Ile Leu Glu
        275                 280                 285

Asp Gln Val Val Ala Ile Lys Asn Glu Ile Val Glu Gln Glu Lys Lys
    290                 295                 300

Tyr Ala Arg Cys Tyr Ser Trp Gly Trp Gly Trp Gly Trp Leu Glu His
305                 310                 315                 320

His His His His His
                325

<210>  27
<211>  1761
<212>  PRT
<213>  Homo sapiens

<400>  27

Met Gln Phe Gln Leu Thr Leu Phe Leu His Leu Gly Trp Leu Ser Tyr
1               5                   10                  15

Ser Lys Ala Gln Asp Asp Cys Asn Arg Gly Ala Cys His Pro Thr Thr
            20                  25                  30

Gly Asp Leu Leu Val Gly Arg Asn Thr Gln Leu Met Ala Ser Ser Thr
        35                  40                  45

Cys Gly Leu Ser Arg Ala Gln Lys Tyr Cys Ile Leu Ser Tyr Leu Glu
    50                  55                  60

Gly Glu Gln Lys Cys Phe Ile Cys Asp Ser Arg Phe Pro Tyr Asp Pro
65                  70                  75                  80

Tyr Asp Gln Pro Asn Ser His Thr Ile Glu Asn Val Ile Val Ser Phe
                85                  90                  95

Glu Pro Asp Arg Glu Lys Lys Trp Trp Gln Ser Glu Asn Gly Leu Asp
            100                 105                 110

His Val Ser Ile Arg Leu Asp Leu Glu Ala Leu Phe Arg Phe Ser His
        115                 120                 125

Leu Ile Leu Thr Phe Lys Thr Phe Arg Pro Ala Ala Met Leu Val Glu
    130                 135                 140

Arg Ser Thr Asp Tyr Gly His Asn Trp Lys Val Phe Lys Tyr Phe Ala
145                 150                 155                 160

Lys Asp Cys Ala Thr Ser Phe Pro Asn Ile Thr Ser Gly Gln Ala Gln
                165                 170                 175

Gly Val Gly Asp Ile Val Cys Asp Ser Lys Tyr Ser Asp Ile Glu Pro
            180                 185                 190

Ser Thr Gly Gly Glu Val Val Leu Lys Val Leu Asp Pro Ser Phe Glu
        195                 200                 205

Ile Glu Asn Pro Tyr Ser Pro Tyr Ile Gln Asp Leu Val Thr Leu Thr
    210                 215                 220

Asn Leu Arg Ile Asn Phe Thr Lys Leu His Thr Leu Gly Asp Ala Leu
225                 230                 235                 240

Leu Gly Arg Arg Gln Asn Asp Ser Leu Asp Lys Tyr Tyr Tyr Ala Leu
                245                 250                 255

Tyr Glu Met Ile Val Arg Gly Ser Cys Phe Cys Asn Gly His Ala Ser
        260                 265                 270

Glu Cys Arg Pro Met Gln Lys Met Arg Gly Asp Val Phe Ser Pro Pro
    275                 280                 285

Gly Met Val His Gly Gln Cys Val Cys Gln His Asn Thr Asp Gly Pro
    290                 295                 300

Asn Cys Glu Arg Cys Lys Asp Phe Phe Gln Asp Ala Pro Trp Arg Pro
305                 310                 315                 320

Ala Ala Asp Leu Gln Asp Asn Ala Cys Arg Ser Cys Ser Cys Asn Ser
                325                 330                 335

His Ser Ser Arg Cys His Phe Asp Met Thr Thr Tyr Leu Ala Ser Gly
        340                 345                 350

Gly Leu Ser Gly Gly Val Cys Glu Asp Cys Gln His Asn Thr Glu Gly
    355                 360                 365

Gln His Cys Asp Arg Cys Arg Pro Leu Phe Tyr Arg Asp Pro Leu Lys
    370                 375                 380

Thr Ile Ser Asp Pro Tyr Ala Cys Ile Pro Cys Glu Cys Asp Pro Asp
385                 390                 395                 400

Gly Thr Ile Ser Gly Gly Ile Cys Val Ser His Ser Asp Pro Ala Leu
                405                 410                 415

Gly Ser Val Ala Gly Gln Cys Leu Cys Lys Glu Asn Val Glu Gly Ala
        420                 425                 430

Lys Cys Asp Gln Cys Lys Pro Asn His Tyr Gly Leu Ser Ala Thr Asp
    435                 440                 445

Pro Leu Gly Cys Gln Pro Cys Asp Cys Asn Pro Leu Gly Ser Leu Pro
    450                 455                 460

Phe Leu Thr Cys Asp Val Asp Thr Gly Gln Cys Leu Cys Leu Ser Tyr
465                 470                 475                 480

Val Thr Gly Ala His Cys Glu Glu Cys Thr Val Gly Tyr Trp Gly Leu
                485                 490                 495

Gly Asn His Leu His Gly Cys Ser Pro Cys Asp Cys Asp Ile Gly Gly

500                 505                 510

Ala Tyr Ser Asn Val Cys Ser Pro Lys Asn Gly Gln Cys Glu Cys Arg
        515                 520                 525

Pro His Val Thr Gly Arg Ser Cys Ser Glu Pro Ala Pro Gly Tyr Phe
    530                 535                 540

Phe Ala Pro Leu Asn Phe Tyr Leu Tyr Glu Ala Glu Glu Ala Thr Thr
545                 550                 555                 560

Leu Gln Gly Leu Ala Pro Leu Gly Ser Glu Thr Phe Gly Gln Ser Pro
                565                 570                 575

Ala Val His Val Val Leu Gly Glu Pro Val Pro Gly Asn Pro Val Thr
            580                 585                 590

Trp Thr Gly Pro Gly Phe Ala Arg Val Leu Pro Gly Ala Gly Leu Arg
        595                 600                 605

Phe Ala Val Asn Asn Ile Pro Phe Pro Val Asp Phe Thr Ile Ala Ile
    610                 615                 620

His Tyr Glu Thr Gln Ser Ala Ala Asp Trp Thr Val Gln Ile Val Val
625                 630                 635                 640

Asn Pro Pro Gly Gly Ser Glu His Cys Ile Pro Lys Thr Leu Gln Ser
                645                 650                 655

Lys Pro Gln Ser Phe Ala Leu Pro Ala Ala Thr Arg Ile Met Leu Leu
            660                 665                 670

Pro Thr Pro Ile Cys Leu Glu Pro Asp Val Gln Tyr Ser Ile Asp Val
        675                 680                 685

Tyr Phe Ser Gln Pro Leu Gln Gly Glu Ser His Ala His Ser His Val
    690                 695                 700

Leu Val Asp Ser Leu Gly Leu Ile Pro Gln Ile Asn Ser Leu Glu Asn
705                 710                 715                 720

Phe Cys Ser Lys Gln Asp Leu Asp Glu Tyr Gln Leu His Asn Cys Val
                725                 730                 735

Glu Ile Ala Ser Ala Met Gly Pro Gln Val Leu Pro Gly Ala Cys Glu
            740                 745                 750

Arg Leu Ile Ile Ser Met Ser Ala Lys Leu His Asp Gly Ala Val Ala
        755                 760                 765

Cys Lys Cys His Pro Gln Gly Ser Val Gly Ser Ser Cys Ser Arg Leu
    770                 775                 780

Gly Gly Gln Cys Gln Cys Lys Pro Leu Val Val Gly Arg Cys Cys Asp
785                 790                 795                 800

Arg Cys Ser Thr Gly Ser Tyr Asp Leu Gly His His Gly Cys His Pro
                805                 810                 815

Cys His Cys His Pro Gln Gly Ser Lys Asp Thr Val Cys Asp Gln Val
            820                 825                 830

Thr Gly Gln Cys Pro Cys His Gly Glu Val Ser Gly Arg Arg Cys Asp
        835                 840                 845

Arg Cys Leu Ala Gly Tyr Phe Gly Phe Pro Ser Cys His Pro Cys Pro
    850                 855                 860

Cys Asn Arg Phe Ala Glu Leu Cys Asp Pro Glu Thr Gly Ser Cys Phe
865                 870                 875                 880

Asn Cys Gly Gly Phe Thr Thr Gly Arg Asn Cys Glu Arg Cys Ile Asp
                885                 890                 895

Gly Tyr Tyr Gly Asn Pro Ser Ser Gly Gln Pro Cys Arg Pro Cys Leu
            900                 905                 910

Cys Pro Asp Asp Pro Ser Ser Asn Gln Tyr Phe Ala His Ser Cys Tyr
        915                 920                 925

Gln Asn Leu Trp Ser Ser Asp Val Ile Cys Asn Cys Leu Gln Gly Tyr
    930                 935                 940

Thr Gly Thr Gln Cys Gly Glu Cys Ser Thr Gly Phe Tyr Gly Asn Pro
945                 950                 955                 960

Arg Ile Ser Gly Ala Pro Cys Gln Pro Cys Ala Cys Asn Asn Asn Ile
                965                 970                 975

Asp Val Thr Asp Pro Glu Ser Cys Ser Arg Val Thr Gly Glu Cys Leu
            980                 985                 990

Arg Cys Leu His Asn Thr Gln Gly  Ala Asn Cys Gln Leu  Cys Lys Pro
        995                 1000                1005

Gly His Tyr Gly Ser Ala Leu Asn Gln Thr Cys Arg Arg Cys Ser
    1010                1015                1020

Cys His Ala Ser Gly Val Ser Pro Met Glu Cys Pro Pro Gly Gly
    1025                1030                1035

Gly Ala Cys Leu Cys Asp Pro Val Thr Gly Ala Cys Pro Cys Leu
    1040                1045                1050

Pro Asn Val Thr Gly Leu Ala Cys Asp Arg Cys Ala Asp Gly Tyr
    1055                1060                1065

Trp Asn Leu Val Pro Gly Arg Gly Cys Gln Ser Cys Asp Cys Asp
    1070                1075                1080

Pro Arg Thr Ser Gln Ser Ser His Cys Asp Gln Leu Thr Gly Gln
    1085                1090                1095

Cys Pro Cys Lys Leu Gly Tyr Gly Gly Lys Arg Cys Ser Glu Cys
    1100                1105                1110

Gln Glu Asn Tyr Tyr Gly Asp Pro Pro Gly Arg Cys Ile Pro Cys
    1115                1120                1125

Asp Cys Asn Arg Ala Gly Thr Gln Lys Pro Ile Cys Asp Pro Asp
    1130                1135                1140

Thr Gly Met Cys Arg Cys Arg Glu Gly Val Ser Gly Gln Arg Cys
    1145                1150                1155

Asp Arg Cys Ala Arg Gly His Ser Gln Glu Phe Pro Thr Cys Leu
    1160                1165                1170

Gln Cys His Leu Cys Phe Asp Gln Trp Asp His Thr Ile Ser Ser
    1175                1180                1185

Leu Ser Lys Ala Val Gln Gly Leu Met Arg Leu Ala Ala Asn Met
    1190                1195                1200

Glu Asp Lys Arg Glu Thr Leu Pro Val Cys Glu Ala Asp Phe Lys
    1205                1210                1215

Asp Leu Arg Gly Asn Val Ser Glu Ile Glu Arg Ile Leu Lys His
    1220                1225                1230

Pro Val Phe Pro Ser Gly Lys Phe Leu Lys Val Lys Asp Tyr His
    1235                1240                1245

Asp Ser Val Arg Arg Gln Ile Met Gln Leu Asn Glu Gln Leu Lys
    1250                1255                1260

Ala Val Tyr Glu Phe Gln Asp Leu Lys Asp Thr Ile Glu Arg Ala
    1265                1270                1275

Lys Asn Glu Ala Asp Leu Leu Leu Glu Asp Leu Gln Glu Glu Ile
    1280                1285                1290

Asp Leu Gln Ser Ser Val Leu Asn Ala Ser Ile Ala Asp Ser Ser
    1295                1300                1305

Glu Asn Ile Lys Lys Tyr Tyr His Ile Ser Ser Ser Ala Glu Lys
    1310                1315                1320

Lys Ile Asn Glu Thr Ser Ser Thr Ile Asn Thr Ser Ala Asn Thr
    1325                1330                1335

Arg Asn Asp Leu Leu Thr Ile Leu Asp Thr Leu Thr Ser Lys Gly
    1340                1345                1350

Asn Leu Ser Leu Glu Arg Leu Lys Gln Ile Lys Ile Pro Asp Ile
    1355                1360                1365

Gln Ile Leu Asn Glu Lys Val Cys Gly Asp Pro Gly Asn Val Pro
    1370                1375                1380

Cys Val Pro Leu Pro Cys Gly Gly Ala Leu Cys Thr Gly Arg Lys
    1385                1390                1395

Gly His Arg Lys Cys Arg Gly Pro Gly Cys His Gly Ser Leu Thr
    1400                1405                1410

Leu Ser Thr Asn Ala Leu Gln Lys Ala Gln Glu Ala Lys Ser Ile
    1415                1420                1425

Ile Arg Asn Leu Asp Lys Gln Val Arg Gly Leu Lys Asn Gln Ile
    1430                1435                1440

Glu Ser Ile Ser Glu Gln Ala Glu Val Ser Lys Asn Asn Ala Leu
    1445                1450                1455

Gln Leu Arg Glu Lys Leu Gly Asn Ile Arg Asn Gln Ser Asp Ser
    1460                1465                1470

Glu Glu Glu Asn Ile Asn Leu Phe Ile Lys Lys Val Lys Asn Phe

1475                1480                1485

Leu Leu Glu Glu Asn Val Pro Pro Glu Asp Ile Glu Lys Val Ala
    1490                1495                1500

Asn Gly Val Leu Asp Ile His Leu Pro Ile Pro Ser Gln Asn Leu
    1505                1510                1515

Thr Asp Glu Leu Val Lys Ile Gln Lys His Met Gln Leu Cys Glu
    1520                1525                1530

Asp Tyr Arg Thr Asp Glu Asn Arg Leu Asn Glu Glu Ala Asp Gly
    1535                1540                1545

Ala Gln Lys Leu Leu Val Lys Ala Lys Ala Ala Glu Lys Ala Ala
    1550                1555                1560

Asn Ile Leu Leu Asn Leu Asp Lys Thr Leu Asn Gln Leu Gln Gln
    1565                1570                1575

Ala Gln Ile Thr Gln Gly Arg Ala Asn Ser Thr Ile Thr Gln Leu
    1580                1585                1590

Thr Ala Asn Ile Thr Lys Ile Lys Lys Asn Val Leu Gln Ala Glu
    1595                1600                1605

Asn Gln Thr Arg Glu Met Lys Ser Glu Leu Glu Leu Ala Lys Gln
    1610                1615                1620

Arg Ser Gly Leu Glu Asp Gly Leu Ser Leu Leu Gln Thr Lys Leu
    1625                1630                1635

Gln Arg His Gln Asp His Ala Val Asn Ala Lys Val Gln Ala Glu
    1640                1645                1650

Ser Ala Gln His Gln Ala Gly Ser Leu Glu Lys Glu Phe Val Glu
    1655                1660                1665

Leu Lys Lys Gln Tyr Ala Ile Leu Gln Arg Lys Thr Ser Thr Thr
    1670                1675                1680

Gly Leu Thr Lys Glu Thr Leu Gly Lys Val Lys Gln Leu Lys Asp
    1685                1690                1695

Ala Ala Glu Lys Leu Ala Gly Asp Thr Glu Ala Lys Ile Arg Arg
    1700                1705                1710

Ile Thr Asp Leu Glu Arg Lys Ile Gln Asp Leu Asn Leu Ser Arg
    1715                    1720                    1725

Gln Ala Lys Ala Asp Gln Leu Arg Ile Leu Glu Asp Gln Val Val
    1730                    1735                    1740

Ala Ile Lys Asn Glu Ile Val Glu Gln Glu Lys Lys Tyr Ala Arg
    1745                    1750                    1755

Cys Tyr Ser
    1760

<210>  28
<211>  779
<212>  PRT
<213>  Artificial Sequence

<220>
<223>  SEQ ID NO 8 aus EP3260864

<400>  28

Met Asp Cys Thr Phe Lys Pro Asp Phe Glu Met Thr Val Lys Glu Cys
1               5                   10                  15

Gln His Ser Gly Glu Leu Ser Ser Arg Asn Thr Gly His Leu His Pro
            20                  25                  30

Thr Pro Arg Ser Pro Leu Leu Arg Trp Thr Gln Glu Pro Gln Pro Leu
        35                  40                  45

Glu Glu Lys Trp Gln His Arg Val Val Glu Gln Ile Pro Lys Glu Val
    50                  55                  60

Gln Phe Gln Pro Pro Gly Ala Pro Leu Glu Lys Glu Lys Ser Gln Gln
65                  70                  75                  80

Cys Tyr Ser Glu Tyr Phe Ser Gln Thr Ser Thr Glu Leu Gln Ile Thr
                85                  90                  95

Phe Asp Glu Thr Asn Pro Ile Thr Arg Leu Ser Glu Ile Glu Lys Ile
            100                 105                 110

Arg Asp Gln Ala Leu Asn Asn Ser Arg Pro Pro Val Arg Tyr Gln Asp
        115                 120                 125

Asn Ala Cys Glu Met Glu Leu Val Lys Val Leu Thr Pro Leu Glu Ile
    130                 135                 140

Ala Lys Asn Lys Gln Tyr Asp Met His Thr Glu Val Thr Thr Leu Lys

145                 150                 155                 160

Gln Glu Lys Asn Pro Val Pro Ser Ala Glu Glu Trp Met Leu Glu Gly
                165                 170                 175

Cys Arg Ala Ser Gly Gly Leu Lys Lys Gly Asp Phe Leu Lys Lys Gly
            180                 185                 190

Leu Glu Pro Glu Thr Phe Gln Asn Phe Asp Gly Asp His Ala Cys Ser
        195                 200                 205

Val Arg Asp Asp Glu Phe Lys Phe Gln Gly Leu Arg His Thr Val Thr
    210                 215                 220

Ala Arg Gln Leu Val Glu Ala Lys Leu Leu Asp Met Arg Thr Ile Glu
225                 230                 235                 240

Gln Leu Arg Leu Gly Leu Lys Thr Val Glu Glu Val Gln Lys Thr Leu
                245                 250                 255

Asn Lys Phe Leu Thr Lys Ala Thr Ser Ile Ala Gly Leu Tyr Leu Glu
            260                 265                 270

Ser Thr Lys Glu Lys Ile Ser Phe Ala Ser Ala Ala Glu Arg Ile Ile
        275                 280                 285

Ile Asp Lys Met Val Ala Leu Ala Phe Leu Glu Ala Gln Ala Ala Thr
    290                 295                 300

Gly Phe Ile Ile Asp Pro Ile Ser Gly Gln Thr Tyr Ser Val Glu Asp
305                 310                 315                 320

Ala Val Leu Lys Gly Val Val Asp Pro Glu Phe Arg Ile Arg Leu Leu
                325                 330                 335

Glu Ala Glu Lys Ala Ala Val Gly Tyr Ser Tyr Ser Ser Lys Thr Leu
            340                 345                 350

Ser Val Phe Gln Ala Met Glu Asn Arg Met Leu Asp Arg Gln Lys Gly
        355                 360                 365

Lys His Ile Leu Glu Ala Gln Ile Ala Ser Gly Gly Val Ile Asp Pro
    370                 375                 380

Val Arg Gly Ile Arg Val Pro Pro Glu Ile Ala Leu Gln Gln Gly Leu
385                 390                 395                 400

Leu Asn Asn Ala Ile Leu Gln Phe Leu His Glu Pro Ser Ser Asn Thr
                405                 410                 415

Arg Val Phe Pro Asn Pro Asn Asn Lys Gln Ala Leu Tyr Tyr Ser Glu
            420                 425                 430

Leu Leu Arg Met Cys Val Phe Asp Val Glu Ser Gln Cys Phe Leu Phe
        435                 440                 445

Pro Phe Gly Glu Arg Asn Ile Ser Asn Leu Asn Val Lys Lys Thr His
    450                 455                 460

Arg Ile Ser Val Val Asp Thr Lys Thr Gly Ser Glu Leu Thr Val Tyr
465                 470                 475                 480

Glu Ala Phe Gln Arg Asn Leu Ile Glu Lys Ser Ile Tyr Leu Glu Leu
                485                 490                 495

Ser Gly Gln Gln Tyr Gln Trp Lys Glu Ala Met Phe Phe Glu Ser Tyr
            500                 505                 510

Gly His Ser Ser His Met Leu Thr Asp Thr Lys Thr Gly Leu His Phe
        515                 520                 525

Asn Ile Asn Glu Ala Ile Glu Gln Gly Thr Ile Asp Lys Ala Leu Val
    530                 535                 540

Lys Lys Tyr Gln Glu Gly Leu Ile Thr Leu Thr Glu Leu Ala Asp Ser
545                 550                 555                 560

Leu Leu Ser Arg Leu Val Pro Lys Lys Asp Leu His Ser Pro Val Ala
                565                 570                 575

Gly Tyr Trp Leu Thr Ala Ser Gly Glu Arg Ile Ser Val Leu Lys Ala
            580                 585                 590

Ser Arg Arg Asn Leu Val Asp Arg Ile Thr Ala Leu Arg Cys Leu Glu
        595                 600                 605

Ala Gln Val Ser Thr Gly Gly Ile Ile Asp Pro Leu Thr Gly Lys Lys
    610                 615                 620

Tyr Arg Val Ala Glu Ala Leu His Arg Gly Leu Val Asp Glu Gly Phe
625                 630                 635                 640

Ala Gln Gln Leu Arg Gln Cys Glu Leu Val Ile Thr Gly Ile Gly His
                645                 650                 655

Pro Ile Thr Asn Lys Met Met Ser Val Val Glu Ala Val Asn Ala Asn
            660                 665                 670

Ile Ile Asn Lys Glu Met Gly Ile Arg Cys Leu Glu Phe Gln Tyr Leu
        675                 680                 685

Thr Gly Gly Leu Ile Glu Pro Gln Val His Ser Arg Leu Ser Ile Glu
    690                 695                 700

Glu Ala Leu Gln Val Gly Ile Ile Asp Val Leu Ile Ala Thr Lys Leu
705                 710                 715                 720

Lys Asp Gln Lys Ser Tyr Val Arg Asn Ile Ile Cys Pro Gln Thr Lys
                725                 730                 735

Arg Lys Leu Thr Tyr Lys Glu Ala Leu Glu Lys Ala Asp Phe Asp Phe
            740                 745                 750

His Thr Gly Leu Lys Leu Leu Glu Val Ser Glu Pro Leu Met Thr Gly
        755                 760                 765

Ile Ser Ser Leu Tyr Tyr Ser Ser Leu Leu Glu
    770                 775

<210>  29
<211>  25
<212>  PRT
<213>  Artificial Sequence

<220>
<223>  Gliadin Peptid

<400>  29

Leu Gly Gln Gln Gln Pro Phe Pro Pro Gln Gln Pro Tyr Pro Gln Pro
1               5                   10                  15

Gln Pro Phe Pro Ser Gln Gln Pro Tyr
            20                  25

<210>  30
<211>  17
<212>  PRT
<213>  Artificial Sequence

<220>
<223>  Gliadin Peptid

<400>  30

Gln Leu Gln Pro Phe Pro Gln Pro Glu Leu Pro Tyr Pro Gln Pro Gln
1               5                   10                  15

Ser

<210>  31
<211>  20
<212>  PRT
<213>  Artificial Sequence

<220>
<223>  Gliadin Peptid

<400>  31

Gln Gln Leu Pro Gln Pro Glu Gln Pro Gln Gln Ser Phe Pro Glu Gln
1               5                   10                  15

Glu Arg Pro Phe
            20

## Beschreibung

Die vorliegende Erfindung betrifft ein Polypeptid umfassend Laminin beta-4, einen Träger umfassend das Polypeptid, einen Antikörper, bevorzugt Autoantikörper gegen Laminin beta-4, eine Verwendung des Polypeptides, Trägers oder Autoantikörpers zur Diagnose einer Krankheit sowie ein Verfahren umfassend den Schritt Nachweisen eines Autoantikörpers gegen Laminin beta-4 in einer Probe.

Unter Autoimmunkrankheiten versteht man Krankheiten, bei denen sich das Immunsystem gegen intakte Strukturen des eigenen Körpers richtet. Bei vielen Autoimmunkrankheiten produziert der Körper nachweisbare Autoantikörper, die gegen Proteine des eigenen Körpers binden und damit Störungen verursachen kann. In anderen Fällen scheint die Anwesenheit von Autoantikörpern nicht kausal für den Ausbruch der Krankheit verantwortlich zu sein.

Blasenbildende Autoimmunkrankheiten zeichnen sich dadurch aus, dass Autoantikörper Zielstrukturen in der Haut angreifen. Immunmechanismen bewirken, dass es zur Ablösung der oberen Hautbestandteile kommt, was zur Bildung von Blasen führt.

Anhand der Zielantigene und der Lokalisation der Spaltbildung unterscheidet man 4 Hauptgruppen: Die Pemphigus- und Pemphigoid-Erkrankungen, die Epidermolysis bullosa acquisita (EBA) und die Dermatitis herpetiformis Duhring. Bei den Pemphiguserkrankungen liegt die Blasenbildung intra-, bei den übrigen bullösen Autoimmundermatosen subepithelial.

Pemphigus vulgaris (PV) ist die häufigste Pemphiguserkrankung, gefolgt vom Pemphigus foliaceus, während andere Formen von Pemphiguserkrankungen wie der paraneoplastische Pemphigus sehr selten auftreten. Der Pemphigus vulgaris (PV) zeichnet sich durch relativ schnell platzende Blasen aus, die Rötungen, Erosionen und Krusten auf der Haut hinterlassen, bedingt durch eine Auflösung des Zellverbandes in der Epidermis. An den betroffenen Stellen kommt es häufig zu Infektionen. Von den Bläschen sind häufig und zu Beginn der Erkrankung die Mundschleimhäute oder die Schleimhäute im Genitalbereich mit starker Schmerzhaftigkeit betroffen. Wird die Erkrankung nicht rechtzeitig erkannt und therapiert, so sind schwere, in manchen Fällen tödliche Verläufe zu erwarten. Der PV tritt mit etwa 1 bis 2 Neuerkrankungen pro einer Million Menschen selten auf.

Bei den subepidermal Blasen bildenden Autoimmunerkrankungen sind die Autoantikörper gegen Strukturproteine im Bereich der dermo-epidermalen Junktionszone (DEJ) gerichtet. Diese Proteine sind von großer Bedeutung für die Adhäsion der basalen Keratinozyten auf der darunter liegenden Dermis. Bei dieser Erkrankungsgruppe unterscheidet man bullöses Pemphigoid, Anti-p200-Pemphigoid, Pemphigoid gestationis, Schleimhautpemphigoid, lineare IgA-Dermatose, Lichen ruber pemphigoides, Epidermolysis bullosa acquisita (EBA) und Dermatits Duhring.

Das bullöse Pemphigoid (BP) ist die häufigste subepidermal blasenbildende Autoimmunkrankheit. Charakteristisch sind aufgrund einer Ablösung der Oberhaut gebildete, prall gefüllte Blasen auf gesunder oder geröteter Haut, die mit starkem Juckreiz als Vorboten einhergehen können. Die Blasen treten häufig am Körperstamm sowie den Oberschenkeln und Armen auf. Bei 20 Prozent der Patienten kommt es auch zu einer Mitbeteiligung der Schleimhäute. Platzen die Blasen, so heilen die Wunden meist ohne Narbenbildung ab. Das BP ist eine relativ häufige Autoimmunerkrankung, mit etwa 15 bis 20 Neuerkrankungen pro einer Million Menschen.

Nach dem bullösen Pemphigoid ist das Schleimhaut-Pemphigoid (SHP, eine Variante davon ist das Anti-Laminin 332 SHP) die zweit-häufigste bullöse Autoimmundermatose. Das SHP stellt klinisch ein heterogenes Krankheitsbild dar, bei dem definitionsgemäß überwiegend die Schleimhäute betroffen sind. Am häufigsten sind die Schleimhäute von Mund und Augen betroffen. Blasenbildende Läsionen heilen manchmal mit Narbenbildung ab. Sind die Konjunktiven betroffen, kann dieses zur Erblindung führen.

Das klinische Bild des Anti-p200-Pemphigoids ist variabel, wobei der BP-ähnliche Typ am häufigsten auftritt. Diese Erkrankung ist seltener als das BP, was auch daran liegen kann, dass viele dieser Patienten aufgrund ihres ähnlichen Immunfluoreszenzmusters als EBA oder Anti-Laminin 332 Schleimhautpemphigoid fehldiagnostiziert werden

Die Epidermolysis bullosa acquisita (EBA) ist eine sehr seltene subepidermale bullöse Autoimmunkrankheit, die durch Autoantikörper gegen Typ VII Kollagen charakterisiert ist. Klinisch können die mechano-bullöse Form und entzündliche Varianten (ähnlich dem BP oder dem Schleimhautpemphigoid) unterschieden werden.

Die klinische oder histologische Unterscheidung der Krankheiten ist schwierig. Im Stand der Technik sind jedoch eine Reihe von serologischen Tests beschrieben, die in vielen Fällen eine Diagnose gestatten. Bei PV treten Autoantikörper gegen die Proteine Desmoglein 1 und/oder 3 auf. Bei BP lassen sich in fast allen Fällen Autoantikörper gegen die Proteine BP180 und/oder BP230 nachweisen, in manchen Fällen auch gegen Laminin 332.

Das Anti-p200-Pemphigoid ist durch Autoantikörper gegen ein Protein der dermalenepidermalen Junktikonszone mit einer apparenten Molmasse von etwa 200kDa charakterisiert. Dieses Protein wurde 2009 (Dainichi, T, Kurono, S., Ohyama, B., Ishii, N., Sanzen, N., Hayashi, M., Shimono, C., Taniguchi, Y., Koga, H., Karashima, T., Yasumoto, S., Zillikens, D., Sekiguchi, K., Hashimoto, T. (2009). Anti-laminin gamma-1 pemphigoid. 106(8):2800-5;) als Laminin gamma-1 beschrieben. Zusätzlich können im Rahmen eines Epitopspreadings auch Antikörper gegen Typ VII Kollagen, BP180 oder auch gegen Laminin 332 gefunden werden.

Zur Anwendung kommen dabei insbesondere ELISA und indirekte Immunfluoreszenz, prinzipiell sind aber auch andere immunbiochemischen Methoden einsetzbar. Geeignet ist beispielsweise das Dermatologie-Profil der EUROIMMUN Medizinische Labordiagnostika AG (EA 1490-1208-1 G), das BP180, NC16A-4X, BP230-CF, Desmoglein 1, Desmoglein 3, Envoplakin und Kollegen Typ VII umfasst.

Der Stand der Technik offenbart diagnostische Verfahrensweisen, insbesondere die indirekte Immunfluoreszenz mit Ösophagusgewebeschnitten von Primaten oder ELISA mit den rekombinanten Autoantigenen als Substrat (vergleiche van Beek, N., Zilikens, D., und Schmidt, E. (2018): Diagnosis of autoimmune bullous diseases, J. Dtsch. Dermatol. Ges. 16 (9), S. 1077-1091). Ein diagnostisch besonders zuverlässiger Test zum Nachweis vom Anti-Laminin 332 Schleimhautpemphigoid kann mittels Immunfluoreszenz und rekombinantem Laminin 332 durchgeführt werden (Goletz, S., Probst, C., Komorowski, L., Schlumberger, W., Fechner, K., van Beek, N., Holtsche, M. M., Recke, A., Yancey, K. B., Hashimoto, T., Antonicelli, F., Di Zenzo, G., Zillikens, D., Stöcker W., und Schmidt, E. (2018): Sensitive and specific assay for the serological diagnosis of anti-laminin 332 mucous membrane pemphigoid, Br. J. Dermatol., DOI 10-1111/bjd.17202). Der Stand der Technik beschreibt keine Autoantikörper gegen Laminin beta-4.

Es zeigt sich jedoch, dass der Nachweis über Laminin gamma-1 nicht bei allen Patienten positiv ist. Im Westernblot mit rekombinanten Formen von Laminin gamma-1 konnten 90% und im ELISA mit der C-terminalen Domäne von Laminin gamma-1 69% der getesteten Anti-p200-Pemphigoid Patienten detektiert werden (Dainichi et al., 2009; Groth, S., Recke, A., Vafia, K., Ludwig, R.J., Hashimoto, T., Zillikens, D., Schmidt, E. (2011). Development of a simple enzyme-linked immunosorbent assay for the detection of autoantibodies in anti-p200 pemphigoid, Br J Dermatol. 164(1):76-82).

Der klinische Verlauf des Anti-p200-Pemphigoid ist variabel. Im Gegensatz zur Epidermolysis bullosa acquisita und dem bullösen Pemphigoid handelt es sich hier um eine relativ benigne Erkrankung mit gutem therapeutischen Ansprechen. Deshalb ist die Differenzierung dieser Erkrankungen von großer klinischer Bedeutung.

US6,682,911 offenbart Laminin 12 und Präparationen von isoliertem Laminin 12 und von Untereinheiten davon.

Vor diesem Hintergrund besteht Bedarf an einem Nachweis für das Anti-p200-Pemphigoid mit höherer diagnostischer Zuverlässigkeit, insbesondere Sensitivität. Dabei soll zudem die Differenzierbarkeit zwischen den verschiedenen blasenbildenden Autoimmunkrankheiten der Haut verbessert werden.

In einem ersten Aspekt wird die der Erfindung zugrundeliegende Aufgabe gelöst durch ein Polypeptid umfassend Laminin beta-4 oder eine Variante davon, bevorzugt in rekombinanter und/oder isolierter Form.

In einer bevorzugten Ausführungsform ist das Polypeptid immobilisiert.

In einem zweiten Aspekt wird das Problem gelöst durch einen Träger umfassend das erfindungsgemäße Polypeptid, weiter umfassend wenigstens ein, bevorzugt alle Antigene aus der Gruppe umfassend Laminin gamma-1, BP180, BP230, Desmoglein 1, Desmoglein 3, Envoplakin, desamidiertes Gliadin, Laminin 332 und Kollagen Typ VII oder Varianten davon.

In einer bevorzugten Ausführungsform ist der Träger aus der Gruppe ausgewählt, die einen Glasträger, bevorzugt Objektträger für Mikroskopie, einen Biochip, eine Mikrotiterplatte, eine Lateral Flow-Vorrichtung, einen Teststreifen, eine Membran, bevorzugt einen Blot, bevorzugter ein Linienblot, ein Chromatographiesäulenmaterial und einen Bead umfasst, bevorzugt einen magnetischen oder fluoreszenten Bead.

In einem dritten Aspekt wird das Problem gelöst durch einen therapeutisch nützlichen Träger umfassend das erfindungsgemäße Polypeptid, wobei der Träger das Kontaktieren von menschlichem Blut mit dem Polypeptid, gefolgt von der Rückführung des Blutes in den Körper eines Patienten gestattet, wobei der Träger bevorzugt eine Aphereseapparatur ist.

In einem vierten Aspekt wird das Problem gelöst durch einen Antikörper, bevorzugt Autoantikörper gegen Laminin beta-4, bevorzugt in isolierter Form.

In einem fünften Aspekt wird das Problem gelöst durch die Verwendung des erfindungsgemäßen Polypeptides, Trägers oder Autoantikörpers zur Diagnose einer Krankheit.

In einem sechsten Aspekt wird das Problem gelöst durch ein Verfahren zur Herstellung eines diagnostisch oder therapeutisch nützlichen Trägers, umfassend den Schritt Beschichten des Trägers mit dem erfindungsgemäßen Polypeptid.

In einem siebten Aspekt wird das Problem gelöst durch ein Verfahren zum Aufreinigen eines Autoantikörpers, umfassend die Schritte a) Kontaktieren einer Flüssigkeit umfassend den Autoantikörper mit dem erfindungsgemäßen Polypeptid unter Bedingungen, die die Ausbildung eines Komplexes umfassend den Autoantikörper und das Polypeptid gestatten, b) Isolieren des Komplexes aus Schritt a), und c) optional Nachweisen des Komplexes aus Schritt a) oder Dissoziieren des in Schritt b) isolierten Komplexes, gefolgt von Abtrennen des Autoantikörpers vom Polypeptid.

In einem achten Aspekt wird das Problem gelöst durch ein Verfahren umfassend den Schritt Nachweisen eines Autoantikörpers gegen Laminin beta-4 in einer Probe.

In einem neunten Aspekt wird das Problem gelöst durch eine pharmazeutische Zusammensetzung umfassend das erfindungsgemäße Polypeptid oder eine Variante davon, bevorzugt zusammen mit einem pharmazeutisch akzeptablen Träger.

In einem zehnten Aspekt wird das Problem gelöst durch ein Kit umfassend das erfindungsgemäße Polypeptid oder den erfindungsgemäßen Träger, wobei das Kit ein oder mehr als ein Reagenz, bevorzugt alle Reagenzien aus der Gruppe enthält, die eine Waschlösung, eine Kalibratorlösung, einen Antikörper gegen Laminin beta-4, und ein Mittel zum Nachweisen eines Autoantikörpers gegen Laminin beta-4, bevorzugt einen sekundären Antikörper, umfasst.

In einem elften Aspekt wird das Problem gelöst durch die Verwendung des erfindungsgemäßen Polypeptids, des erfindungsgemäßen Trägers oder eines Antikörpers, bevorzugt Autoantikörpers gegen Laminin beta-4 zur Herstellung eines Kits oder diagnostisch oder therapeutisch nützlichen Trägers.

In einer bevorzugten Ausführungsform wird ein Autoantikörper gegen Laminin beta-4 unter Verwendung einer Methode nachgewiesen, die aus der Gruppe ausgewählt ist, welche Immundiffusion, Immunelektrophorese, Lichtstreuung, Agglutination, Immuntest mit Markierung wie die aus der Gruppe umfassend Immuntest mit radioaktiver Markierung, mit enzymatischer Markierung, bevorzugter ELISA, mit Chemilumineszenz-Markierung, bevorzugter Elektrochemilumineszenz-Markierung und mit Immunfluoreszenz-Markierung, bevorzugt indirekte Immunfluoreszenz, umfasst.

In einem zwölften Aspekt wird das Problem gelöst durch die Verwendung des erfindungsgemäßen Polypeptides oder des erfindungsgemäßen Antikörpers zur Bestimmung der Fähigkeit, bevorzugt Kapazität einer diagnostischen oder therapeutischen Vorrichtung oder eines diagnostischen oder therapeutischen Reagenz, einen Antikörper gegen Laminin beta-4 zu binden.

Die vorliegende Erfindung beruht auf der überraschenden Erkenntnis der Erfinder, dass ein Autoantikörper gegen Laminin beta-4 existiert und spezifisch bei Patienten, die unter Anti-p200-Pemphigoid leiden, nachgewiesen werden kann.

Die Erfinder haben weiterhin gefunden, dass der Nachweis dieses Autoantikörpers die Sensitivität des serologischen diagnostischen Tests für das Anti-p200-Pemphigoid erhöht. Er kann bei einigen Patienten nachgewiesen werden, bei denen der bisher als alleinig entscheidend angesehene Autoantikörper gegen Laminin gamma-1 nicht nachgewiesen werden kann.

Laminine sind trimere Basalmembranproteine, die aus alpha-, beta- und gamma-Ketten bestehen. Bei Laminin beta-4 handelt es sich um eine beta-Kette. Laminine sind an vielen physiologischen Funktionen wie z.B. der Zelladhäsion, Migration, Differenzierung, neuronale Entwicklung und an der Regulation der Genexpression beteiligt.

Choi *et al.* (2015) beobachteten eine reduzierte Expression von Laminin beta-4 in Magen- und Darmkrebsgewebe. Sie identifizierten somatische Laminin beta-4-Mutationen in Krebsgewebe, die möglicherweise zu einer reduzierten Laminin beta-4-Expression beigetragen haben (Choi, M. R., An, C. H., Yoo, N. J., Lee, S. H. Laminin gene LAMB4 is somatically mutated and expressionally altered in gastric and colorectal cancers. APMIS 123: 65-71, 2015.) Reduzierte Laminin beta-4-Level könnten zudem auch eine Rolle bei der Divertikulitits spielen (Coble JL, Sheldon KE, Yue F, Salameh TJ, Harris LR III, Deiling S, Ruggiero FM, Eshelman MA, Yochum GS, Koltun WA, Gerhard GS, Broach JR. Identification of a rare LAMB4 variant associated with familial diverticulitis through exome sequencing. Hum Mol Genet. 2017;26(16):3212-3220).

Die vorliegende Erfindung betrifft ein Polypeptid umfassend ein Säugetier-Laminin beta-4, bevorzugt aus dem Menschen, Affen, dem Rind, dem Schaf oder Schwein und besonders bevorzugt aus dem Menschen.

In einer bevorzugteren Ausführungsform ist Laminin beta-4 das Polypeptid, das durch die Datenbank-Codes (BC140804), bevorzugt SEQ ID NO27, kodiert wird, oder eine Variante davon. In dieser Anmeldung stehen sämtliche zitierte Datenbank-Codes für die Uniprot-Datenbank oder sonstige Datenbanken, genauer für deren Version am Einreichungstag dieser Anmeldung oder ihrer frühesten Prioritätsanmeldung. In einer weiteren bevorzugten Ausführungsform weisen die weiteren Antigene die jeweils in Klammern angegebene Sequenz auf: Laminin gamma-1 (LAMC1 P11047), Laminin 332 ausgewählt aus der Gruppe umfassend LAMA3 (Q16787), LAMB3 (Q13751) und LAMC2 (Q13753), BP180 (Q9UMD9), BP230 (SEQ ID NO8 aus EP3260864, hier aufgeführt als SEQ ID NO 28), Desmoglein 1 (Q02413), Desmoglein 3 (P32926), Envoplakin (Q92817), Gliadin, bevorzugt ausgewählt aus der Gruppe umfassend LGQQQPFPPQQPYPQPQPFPSQQPY (SEQ ID NO 29), QLQPFPQPELPYPQPQS (SEQ ID NO 30) und QQLPQPEQPQQSFPEQERPF (SEQ ID NO 31) und Kollagen Typ VII (Q02388) oder Varianten davon.

Die erfindungsgemäße Lehre kann jedoch nicht nur unter Verwendung von Polypeptiden oder Nukleinsäuren, genauer einem Polypeptid umfassend die Wildtypsequenz von Laminin beta-4 oder einer dafür kodierenden Nukleinsäure, die die genauen Sequenzen aufweisen, die in dieser Anmeldung ausdrücklich oder implizit aufgeführt werden, sondern auch unter Verwendung von Varianten solcher Polypeptide oder Nukleinsäuren ausgeführt werden.

In einer bevorzugten Ausführungsform, kann der Begriff "Variante", wie hierin verwendet werden, wenigstens ein Fragment oder eine Volllängen-Sequenz bezeichnen, genauer eine oder mehr als eine Aminosäuresequenz oder Nukleinsäurensequenz, die relativ zur Volllängen-Sequenz an einem oder beiden Enden um wenigstens eine oder mehr als eine Aminosäure trunkiert ist. Ein solches Fragment umfasst oder kodiert für ein Peptid mit wenigstens 6, 7, 8, 10, 12, 15, 20, 25, 50, 75, 100, 300, 400, 600, 800, 1000 oder 1200 aufeinander folgenden Aminosäuren der Originalsequenz oder einer Variante davon. Die Gesamtlänge der Variante kann wenigstens 6, 7, 8, 9, 10, 11, 12, 20, 25, 30, 40, 50, 60, 70, 80, 90, 100, 200, 300, 400, 500, 600, 800, 1000, 1200, 1500, 1800 oder mehr Aminosäuren betragen. Ein besonders bevorzugtes Fragment ist aus der Gruppe ausgewählt, die SEQ ID NO 8, SEQ ID NO 9, SEQ ID NO 10, SEQ ID NO 11, SEQ ID NO 12, SEQ ID NO 13 und SEQ ID NO 14 und Varianten davon umfasst.

In einer weiteren bevorzugten Ausführungsform bezeichnet der Begriff "Variante" nicht nur wenigstens ein Fragment, sondern auch ein Polypeptid oder Fragment davon, das Aminosäurensequenzen umfasst, die zu wenigstens 40, 50, 60, 70, 75, 80, 85, 90, 92, 94, 95, 96, 97, 98 oder 99 % identisch zur Aminosäurensequenz oder des Fragments davon sind, auf die oder das ausdrücklich Bezug genommen wurde, wobei andere Aminosäuren als die für die biologische Aktivität essenziellen, beispielsweise die Fähigkeiten eines Antigens, an den (Auto)Antikörper zu binden, oder die Faltung oder Struktur des Polypeptides, deletiert oder substituiert wurden und/oder eine oder mehr als eine solche essenzielle Aminosäure auf konservative Weise ersetzt und/oder Aminosäuren derart hinzugefügt wurden, dass die biologische Aktivität des Polypeptides beibehalten wird. Der Stand der Technik umfasst verschiedene Verfahren, die verwendet werden können, um zwei gegebene Nukleinsäuren- oder Aminosäuren-Sequenzen zu alignen und das Ausmaß ihrer Identität zu berechnen, siehe zum Beispiel Arthur Lesk (2008), Introduction to bioinformatics, Oxford University Press, 2008, 3rd edition. In einer bevorzugten Ausführungsform wird die ClustalW- Software unter Verwendung der Grundeinstellungen eingesetzt (Larkin, M. A., Blackshields, G., Brown, N. P., Chenna, R., McGettigan, P. A., McWilliam, H., Valentin, F., Wallace, I. M., Wilm, A., Lopez, R., Thompson, J. D., Gibson, T. J., Higgins, D. G. (2007). Clustal W and Clustal X version 2.0. Bioinformatics, 23, 2947-2948).

In einer bevorzugten Ausführungsform ist die Variante ein lineares, nicht gefaltetes Polypeptid, das optional denaturiert ist.

In einer bevorzugten Ausführungsform können das Polypeptid und Varianten davon zusätzlich chemische Modifikationen enthalten, zum Beispiel isotopische Markierungen oder kovalente Modifikationen wie eine Glykosylierung, Phosphorylierung, Acetylierung, Decarboxylierung, Citrullinierung, Methylierung, Hydroxylierung und dergleichen. Der Fachmann ist mit Verfahren vertraut, um Polypeptide zu modifizieren. Jegliche Modifikation wird derart gestaltet, dass sie die biologische Aktivität der Variante nicht aufhebt.

Darüber hinaus können Varianten auch durch N-terminale und/oder C-terminale Fusion mit anderen Polypeptiden, Peptiden oder Aminosäuren oder Varianten davon generiert werden und aktive Teile oder Domänen umfassen, zum Beispiel mit einer Sequenzidentität von wenigstens 70, 75, 80, 85, 90, 92, 94, 95, 96, 97, 98 oder 99 %, wenn sie mit dem aktiven Teil der Wildtypsequenz aligned werden, wobei der Begriff "aktiver Teil" wie hierin verwendet, die Aminosäurensequenz bezeichnet, die kürzer als die Vollängen-Aminosäurensequenz ist, oder, im Falle einer Nukleinsäurensequenz, für weniger als die Volllängen-Aminosäurensequenz kodiert, und/oder eine Variante der natürlichen Sequenz ist, aber wenigstens ein Teil der biologischen Aktivität behält. Anfusionierte Sequenzen sind so ausgestaltet, dass sie die Bindung des Autoantikörpers an das Laminin beta-4 oder die Variante davon nicht stören, insbesondere nicht an die Epitope, die die Sequenzen SEQ ID NO 8, SEQ ID NO 9, SEQ ID NO 10, SEQ ID NO 11, SEQ ID NO 12, SEQ ID NO 13 und SEQ ID NO 14 und Varianten davon umfassen. Geeignet ist z.B. ein Tag, beispielsweise aus der Gruppe umfassend GST, His, MBP und FLAG, das direkt oder über einen flexiblen Linker N-terminal und/oder C-terminal anfusioniert ist.

In einer bevorzugten Ausführungsform umfasst der Begriff "Variante" Nukleinsäuren des komplementären Stranges, der unter stringenten Bedingungen, an die Referenz- oder Wildtyp- Nucleinsäure hybridisiert. Die Stringenz von Hybridisierungsreaktionen kann von dem Fachmann leicht bestimmt werden und ist im Allgemeinen eine empirische Berechnung, die von der Sondenlänge, den Waschtemperaturen und der Salzkonzentration abhängt. Im Allgemeinen gilt, dass längere Sonden höhere Temperaturen zum spezifischen Annealen benötigen, wohingegen kürzere Sonden weniger hohe Temperatur benötigen. Die Hybridisierung hängt im Allgemeinen von der Fähigkeit einer denaturierten DNA ab, erneut an den komplementären Strang in einer Umgebung unterhalb ihrer Schmelztemperatur zu hybridisieren. Je höher das Ausmaß der erforderlichen Homologie zwischen der Sonde und der hybridisierfähigen Sequenz, desto höher die relative Temperatur die verwendet werden kann. Als Ergebnis würden höhere relative Temperaturen dazu tendieren, die Reaktionsbedingungen stringenter zu gestalten, während eine geringere Temperatur dies in einem geringeren Grad bewirkt. Für zusätzliche Details und Erklärungen zur Stringenz und Hybridisierungsreaktion siehe Ausubel, F. M. (1995), Current Protocols in Molecular Biology. John Wiley & Sons, Inc. Darüber hinaus kann der Fachmann den Instruktionen folgen, die im Manual von Boehringer Mannheim GmbH (1993) (The DIG System Users Guide for Filter Hybridization, Boehringer Mannheim GmbH, Mannheim, Germany) and in Liebl, W., Ehrmann, M., Ludwig, W., and Schleifer, K. H. (1991) International Journal of Systematic Bacteriology 41: 255-260) zur Frage, wie DNA-Sequenzen mittels Hybridisierung identifiziert werden können. In einer bevorzugten Ausführungsform werden stringente Bedingungen für jegliche Hybridisierung angewandt, d.h. eine Hybridisierung tritt auf, wenn die Sonde zu 70% oder mehr zur Zielsequenz identisch ist. Sonden mit einem geringeren Ausmaß an Identität mit Hinblick auf die Zielsequenz können hybridisieren, aber solche Hybride sind instabil und werden in einem Waschschritt unter stringenten Bedingungen entfernt, zum Beispiel durch Erniedrigen der Salzkonzentration auf 2 x SSC oder optional und dann nachfolgend 0,5 x SSC, während die Temperatur, mit zunehmender Bevorzugtheit, ungefähr 50°C - 68°C, ungefähr 52°C - 68°C, ungefähr 54°C - 68°C, ungefähr 56°C - 68°C, ungefähr 58°C - 68°C, ungefähr 60°C - 68°C, ungefähr 62°C - 68°C, ungefähr 64°C - 68°C, ungefähr 66°C - 68°C beträgt. In einer besonderen bevorzugten Ausführungsform beträgt die Temperatur ungefähr 64°C - 68°C oder bevorzugt 66°C - 68°C. Es ist möglich, die Salzkonzentration auf 0.2 x SSC oder selbst 0.1 x SSC anzupassen. Nukleinsäuresequenzen mit einem Identitätsausmaß mit Hinblick auf die Referenz- oder Wildtyp Sequenz von wenigstens 70, 80, 90, 91, 92, 93, 94, 95, 96, 97, 98, 99 % können isoliert werden. In einer bevorzugten Ausführungsform bezeichnet der Begriff "Variante einer Nukleinsäuresequenz", wie hierin verwendet, eine jegliche Nukleinsäurensequenz, die für die gleiche Aminosäuresequenz und Varianten davon wie die Referenz-Nukleinsäurensequenz kodiert, entsprechend der Degeneriertheit des genetischen Codes.

Die Variante des Polypeptides weist biologische Aktivität auf. In einer bevorzugten Ausführungsform handelt es sich dabei um die Fähigkeit, an einen Autoantikörper gegen Laminin beta-4 zu binden, wie er in einem Patienten gefunden wird, der an einer Autoimmunkrankheit leidet, die mit einem solchen Autoantikörper assoziiert ist, bevorzugt Anti-p200-Pemphigoid. Ob z.B. eine Variante von Laminin beta-4 eine solche biologische Aktivität hat, kann durch den Nachweis geklärt werden, ob sie an einen Autoantikörper aus einer Patientenprobe bindet, bevorzugt durch Westernblotting und/oder indirekte Immunfluoreszenz, bevorzugt indirekte Immunfluoreszenz, unter Verwendung von rekombinanten Proteinen wie in den Beispielen beschrieben. Die Erfinder haben zudem die gegenüber einem Autoantikörper gegen Laminin beta-4 reaktive Sequenzen SEQ ID NO 8, SEQ ID NO 9, SEQ ID NO 10, SEQ ID NO 11, SEQ ID NO 12, SEQ ID NO 13 und SEQ ID NO 14 identifiziert. Varianten von Laminin beta-4, an die ein solcher Autoantikörper bindet, lassen sich also leicht erzeugen, indem ein Epitop aus der Laminin beta-4-Sequenz generiert wird, das wenigstens eine dieser Sequenzen umfasst, wobei optional zusätzliche Varianten durch konservative Substitutionen von Aminosäureresten erzeugt werden können.

Das erfindungsgemäße Polypeptid kann in jeglicher Form und jeglicher Aufreinigungsstufe bereitgestellt werden, von flüssigen Proben, Geweben oder Zellen, die das Polypeptid in einer endogenen Form umfassen, bevorzugter Zellen, die das Polypeptid überexprimieren, kruden oder angereicherten Lysaten solcher Zellen, bis zu gereinigtem und/oder isoliertem Polypeptid, das optional im Wesentlichen rein ist. In einer bevorzugten Ausführungsform ist das Polypeptid ein natives Polypeptid, wobei der Begriff "natives Polypeptid", wie hierin verwendet, ein gefaltetes Polypeptid, bevorzugter ein gefaltetes Polypeptid aufgereinigt aus Geweben oder Zellen, bevorzugter aus Säugetierzellen oder -geweben, optional aus nichtrekombinanten Geweben oder Zellen darstellt. In einer weiteren bevorzugten Ausführungsform ist das Polypeptid ein rekombinantes Protein, wobei der Begriff "rekombinant", wie hierin verwendet, ein Polypeptid bedeutet, das unter Verwendung von gentechnischen Methoden bei einer beliebigen Phase des Herstellungsprozesses hergestellt wurde, zum Beispiel durch Fusionieren einer Nukleinsäure, die für das Polypeptid kodiert, an einen starken Promotor zur Überexpression in Zellen oder Geweben oder durch Verändern der Polypeptid-Sequenz selbst. Der Fachmann ist mit Verfahren zum gentechnischen Verändern von Nukleinsäuren und von ihnen kodierten Polypeptiden vertraut (zum Beispiel beschrieben in Sambrook, J., Fritsch, E. F. and Maniatis, T. (1989), Molecular Cloning, CSH or in Brown T. A. (1986), Gene Cloning - an introduction, Chapman & Hall), sowie mit Methoden zum Produzieren und Aufreinigen nativer oder rekombinanter Proteine (zum Beispiel Handbooks "Strategies for Protein Purification", "Antibody Purification", "Purifying Challenging Proteins" (2009/2010), published by GE Healthcare Life Sciences, and in Burgess, R. R., Deutscher, M. P. (2009), Guide to Protein Purification). In einer bevorzugten Ausführungsform ist ein Polypeptid rein, wenn wenigstens 60, 70, 80, 90, 95 oder 99 % des Gesamtpolypeptides in der entsprechenden Probe aus dem Polypeptid besteht, wie mittels SDS-Polyacrylamid-Gelelektrophorese gefolgt von Coomassie Blau-Färbung und Abschätzung mit dem bloßen Auge beurteilt wird. Falls das erfindungsgemäße Polypeptid in Form eines Gewebe bereitgestellt wird, ist bevorzugt, dass das Gewebe ein SäugetierGewebe ist, zum Beispiel Gewebe vom Menschen, vom Primaten, vom Esel, von der Ziege, vom Pferd, vom Schaf, vom Schwein, oder von der Kuh, bevorzugt Hautgewebe. Wenn das Polypeptid in Form einer rekombinanten Zelle bereitgestellt wird, ist bevorzugt, dass die rekombinante Zelle eine eukaryotische Zelle wie eine Hefezelle ist, bevorzugter eine Zelle aus einem multizellulären Eukaryonten wie einer Pflanze, einem Säugetier, Frosch oder Insekt, am bevorzugtesten aus einem Säugetier, zum Beispiel Ratte, Mensch, Primat, Esel, Maus, Ziege, Pferd, Schaf, Schwein oder Kuh.

Das Polypeptid zum Ausführen der erfindungsgemäßen Lehre ist bevorzugt derart beschaffen, dass es wenigstens ein Epitop umfasst, das von dem Autoantikörper erkannt wird, der an Laminin beta-4 bindet, oder dass es an ihn spezifisch bindet. Das Epitop ist bevorzugt ein Epitop, das nur von solch einem Autoantikörper erkannt wird und nicht von anderen Antikörpern, wie Autoantikörpern gegen Laminin gamma-1, BP180, BP230, Desmoglein 1, Desmoglein 3, Envoplakin, Gliadin, Laminin 332 oder Kollagen Typ VII. In einer solchen Ausführungsform umfasst ein solches Epitop einen Abschnitt von 6, 7, 8, 9, 10, 11, 12, 20, 25, 30, 40, 50, 60, 70, 80, 90, 100 oder mehr, bevorzugt wenigstens 9, aber nicht mehr als 16, aufeinanderfolgenden Aminosäuren aus Laminin beta-4. Der Fachmann ist vertraut mit Richtlinien zum Entwerfen von Peptiden, die ausreichend Immunogenizität haben, zum Beispiel wie sie in Jackson *et al.* (Jackson, D. C., Fitzmaurice, C. J., Brown, L. E., Zeng, W. (1999), Preparation and properties of totally synthetic immunogenes, Vaccine Volume 18, Issues 3-4, September 1999, Pages 355-361; and Black, M., Trent, A., Tirrell, M. and Olive, C. (2010), Advances in the design and delivery of peptide subunit vaccines with a focus on Toll-like receptor agonists, Expert Rev Vaccines, 2010 February; 9(2): 157-173) beschrieben sind.

Das erfindungsgemäße Polypeptid, das Laminin beta-4 oder eine Variante davon umfasst, kann zur Ausführung der vorliegenden Erfindung in jeglicher Konformation bereitgestellt werden. Zum Beispiel kann das Polypeptid ein im Wesentlichen ungefaltetes, oder teilweise oder voll gefaltetes Polypeptid darstellen. In einer bevorzugten Ausführungsform ist das Polypeptid in den Sinne gefaltet, dass das oder die für das Binden an den erfindungsgemäßen Autoantikörper erforderliche Epitop bzw. erforderlichen Epitope in seiner oder ihrer Gesamtheit, die Faltung nimmt bzw. annehmen, die das native Protein in seiner natürlichen Umgebung annimmt. Der Fachmann auf dem Gebiet ist mit Verfahren vertraut, die zum Bestimmen eingesetzt werden können, ob das Polypeptid gefaltet ist, und wenn es gefaltet ist, welche Struktur es annimmt, zum Beispiel limitierte Proteolyse, NMR-Spektroskopie, CD-Spektroskopie oder Röntgen-Kristallografie (siehe zum Beispiel Banaszak L. J. (2008), Foundations of Structural Biology, Academics Press, or Teng Q. (2013), Structural Biology: Practical Applications, Springer), wobei bevorzugt CD-Spektroskopie verwendet wird.

Das erfindungsgemäße Polypeptid kann ein Fusionsprotein sein, das C-terminal und/oder N-terminal andere Aminosäuren und/oder -sequenzen als die aus Laminin beta-4 umfasst, insbesondere ein C-terminales oder N-terminales Tag, bevorzugt ein C-terminales Tag, das in einer bevorzugten Ausführungsform, wie hierin verwendet wird, ein zusätzliches Sequenzmotiv oder ein Polypeptid umfasst, die irgendeine biologische oder physikalische Funktion aufweist, und zum Beispiel umgesetzt werden kann, um das erfindungsgemäße Polypeptid aufzureinigen, zu immobilisieren, zu präzipitieren oder zu identifizieren. In einer bevorzugteren Ausführungsform ist der Tag eine Sequenz oder Domäne, der in der Lage ist, spezifisch an einen Liganden zu binden, zum Beispiel ein Tag ausgewählt aus der Gruppe umfassend His-Tags, Thioredoxin, Maltose bindendes Protein, Glutathion-S-Transferase, ein Fluoreszenz Tag, zum Beispiel aus der Gruppe umfassend grünes fluoreszierendes Protein (GFP).

Das erfindungsgemäße Polypeptid kann ein immobilisiertes Polypeptid sein. In einer bevorzugten Ausführungsform bezeichnet der Begriff "immobilisiert", wie hierin verwendet, ein Molekül, das an einen festen Träger gebunden ist, der in einer wässrigen Lösung unlöslich ist, bevorzugter über eine kovalente Bindung, elektrostatische Interaktion, Einkapselung oder Einschluss, zum Beispiel durch Denaturierung eines globulären Polypeptides in einem Gel, oder über hydrophobe Interaktion, am bevorzugtesten über eine oder mehr als eine kovalente Bindung. Verschiedene geeignete Träger, zum Beispiel Papier, Polystyrol, Metall, Silikon oder Glasoberflächen, mikrofluidische Kanäle, Membranen, Beads wie magnetische Beads, Chromatographie-Säulenmedien, Biochips, Polyacrylamid Gele und dergleichen sind in der Literatur beschrieben, zum Beispiel in Kim, D., and Herr, A. E. (2013), Protein immobilization techniques for microfluidic assays, Biomicrofluidics 7(4), 041501. Auf diese Weise kann das immobilisierte Molekül zusammen mit dem unlöslichen Träger von einer wässrigen Lösung auf einfache Weise getrennt werden, zum Beispiel durch Filtrierung, Zentrifugieren oder Dekantieren. Ein immobilisiertes Molekül kann reversibel oder irreversibel immobilisiert sein. Zum Beispiel ist die Immobilisierung reversibel, wenn das Molekül mit dem Träger über eine ionische Interaktion interagiert, die durch Hinzufügen einer hohen Konzentration eines Salzes maskiert werden kann, oder wenn das Molekül über eine spaltbare kovalente Bindung wie eine Disulfidbrücke gebunden ist, die durch Hinzufügen von Thiol enthaltenen Reagenzien gespalten werden kann. Im Gegensatz dazu ist die Immobilisierung irreversibel, wenn das Molekül an dem Träger über eine kovalente Bindung befestigt ist, die nicht in wässriger Lösung gespalten werden kann, zum Beispiel eine Bindung, die durch Reaktion einer Epoxidgruppe und einer Aminogruppe gebildet wird, wie sie häufig verwendet wird, um Lysinseitenketten an Affinitätssäulen zu binden. Das Protein kann indirekt immobilisiert werden, zum Beispiel durch Immobilisieren eines Antikörpers oder einer anderen Einheit mit Affinität an das Molekül, gefolgt von Bildung eines Komplexes mit der Wirkung, dass der Molekül-Antikörper Komplex immobilisiert ist. Verschiedene Möglichkeiten zum Immobilisieren von Molekülen sind in der Literatur beschrieben, zum Beispiel in Kim, D., Herr, and A. E. (2013), Protein immobilization techniques for microfluidic assays, Biomicrofluidics 7(4), 041501. Zusätzlich sind verschiedene Reagenzien und Kits für Immobilisierungsreaktionen im Handel erhältlich, zum Beispiel von Pierce Biotechnology.

Es ist notwendig, dass die zur Diagnose im Wege des Nachweises von Autoantikörpern gemäß der vorliegenden Erfindung eingesetzte Probe Antikörper aufweist, die auch als Immunglobuline bezeichnet werden. Typischerweise umfasst die Probe einer Körperflüssigkeit eine repräsentative Auswahl der Gesamtheit der Immunglobuline des Patienten. Nach dem Bereitstellen kann die Probe jedoch weiteren Prozessierungsschritten unterworfen werden, die Fraktionierung, Zentrifugation, Anreichung oder Isolierung der Gesamtheit der Immunglobuline oder einer bestimmten Immunglobulinklasse des Patienten umfassen können und die relative Verteilung der Immunglobuline der verschiedenen Klassen beeinflussen können.

Die Reagenzien, Vorrichtungen, Verfahren und Verwendungen, die in dieser Anmeldung beschrieben sind, können zur Diagnose einer Krankheit verwendet werden. In einer bevorzugten Ausführungsform ist die Krankheit eine Autoimmunkrankheit, noch bevorzugter eine Autoimmunkrankheit der Haut und/oder des Auges. Solche Krankheiten zeichnen sich durch ein überlappendes Spektrum an Symptomen aus, darunter Blasen, Rötungen und Blutungen sowie Folgeerkrankungen wie eine Infektion der betroffenen Stellen. Dementsprechend ist auch eine Diagnose, diagnostische Differenzierung von oder indirekte Diagnose einer Infektionen der Haut, des Auges oder einer Allergie möglich. Bei der Autoimmunkrankheit handelt es sich besonders bevorzugt um eine aus der Gruppe umfassend Anti-p200-Pemphigoid, Anti-Laminin 332 Schleimhautpemphigoid, Epidermolysis bullosa acquisita, BP und PV.

In einer bevorzugten Ausführungsform bezeichnet der Begriff "Diagnose", wie hierin verwendet, eine jegliche Prozedur, die darauf abzielt, Informationen zu erhalten, die für die Einschätzung, ob ein Patient an einer bestimmten Krankheit oder Störung in der Vergangenheit, zur Zeit der Diagnose oder in der Zukunft leidet bzw. mit höherer Wahrscheinlichkeit als die durchschnittliche Vergleichsperson, bevorzugt mit ähnlichen Symptomen, leiden wird, um herauszufinden, wie die Krankheit fortschreitet oder wahrscheinlich in der Zukunft fortschreiten wird, oder um das Ansprechen eines Patienten auf eine bestimmte Behandlung zu evaluieren, beispielsweise die Verabreichung von Immunsuppressiva. In anderen Worten umfasst der Begriff "Diagnose" nicht nur das Diagnostizieren, sondern auch das Prognostizieren und/oder das Überwachen des Verlaufs einer Krankheit oder Störung.

In vielen Fällen ist das bloße Nachweisen, in anderen Worten das Bestimmen, ob nachweisbare Konzentrationen des Antikörpers in der Probe vorhanden sind oder nicht, für die Diagnose ausreichend. Wenn der Autoantikörper nachgewiesen werden kann, ist dies eine Information, die für die Diagnose des Arztes wichtig ist und anzeigt, dass seine erhöhte Wahrscheinlichkeit besteht, dass der Patient an der Krankheit leidet. Bevorzugt kann diese Information oder ein Signal, das diese Information beinhaltet, an einen Patienten oder den ihn behandelnden Arzt schriftlich, telefonisch oder elektronisch kommuniziert werden, beispielsweise über das Internet, z.B. als Email. Die ermöglicht den Arzt in einer besonders bevorzugten Ausführungsform, eine diagnostisch nützliche Schlussfolgerung zu ziehen und optional bei der Behandlung des Patienten zu berücksichtigen. In einer bevorzugten Ausführungsform gilt der Autoantikörper als nachweisbar, wenn er unter Verwendung von einem oder mehr als einem Verfahren ausgewählt aus der Gruppe umfassend Immunpräzipitation, indirekte Immunfluoreszenz, ELISA oder Blot, bevorzugt Blot nachgewiesen werden kann. Die experimentellen Details sind wie in dem experimentellen Teil dieser Anmeldung beschrieben oder wie in Lehrbüchern oder praktischen Protokollen beschrieben, wie sie zum frühesten Prioritätstag dieser Anmeldung verfügbar waren. In einer bevorzugten Ausführungsform, wird die relative Konzentration des Antikörpers im Serum bestimmt, im Vergleich zur Konzentration, die in einer durchschnittlichen gewöhnlichen Person gefunden werden. Während es in vielen Fällen ausreichend ist, festzustellen, ob die Autoantikörper in der Probe vorhanden oder nachweisbar sind, kann das Verfahren zum Erhalten nützlicher Informationen für die Diagnose umfassen, dass bestimmt wird, ob die Konzentration wenigstens 0.1, bevorzugt 0.2, 0.5, 1, 2, 5, 10, 20, 25, 50, 100, 200, 500, 1000, 10000 oder 100000 Mal höher als die Konzentration ist, die in der gewöhnlichen gesunden Person gefunden wird. In einer bevorzugten Ausführungsform, wird die relative Konzentration des Autoantikörpers unter Verwendung von einem oder mehr als einem Verfahren bestimmt, das aus der Gruppe umfassend semi-quantitative Immunpräzipitation, semi-quantitative indirekte Immunfluoreszenz, ELISA oder Line Blot, bevorzugt ELISA ausgewählt ist.

Die vorliegende Erfindung betrifft ein Komplex umfassend einen Antikörper, bevorzugt Autoantikörper, der gegen das erfindungsgemäße Polypeptid bindet. Ein solcher Komplex kann verwendet oder als Teil eines Verfahrens zum Diagnostizieren einer Krankheit nachgewiesen werden. Es kann eine flüssige Probe umfassend Antikörper von einem Patienten verwendet werden, um das Verfahren auszuführen, bei dem ein Autoantikörper gegen Laminin beta-4 nachzuweisen ist. Eine solche flüssige Probe kann irgendeine Körperflüssigkeit umfassend eine repräsentative Auswahl an Antikörpern aus der Person umfassen, bevorzugt eine Probe umfassend Antikörper der Immunglobulinklasse IgG von der Person. Zum Beispiel kann eine Probe Liquor, Blut oder Blutserum, Lymphflüssigkeit, interstitielle Flüssigkeit sein und ist bevorzugt Serum oder Liquor, bevorzugter Serum.

Der Schritt Kontaktieren einer flüssigen Probe umfassend Antikörper mit dem erfindungsgemäßen Polypeptid kann durch Inkubieren einer immobilisierten Form des Polypeptides in Anwesenheit der Probe, der Antikörper umfassenden Probe unter Bedingungen ausgeführt werden, die mit der Bildung eines Komplexes umfassend das Polypeptid und einen Antikörper, bevorzugt Autoantikörper, kompatibel sind, der oder die an das erfindungsgemäße Polypeptid bindet. Die flüssige Probe, in der Antikörper, die gegen das oder die erfindungsgemäßen Polypeptid(e) binden, abgereichert sind, kann anschließend entfernt werden, gefolgt von einem oder mehr als einem Waschschritt. Schließlich kann der Komplex umfassend den Antikörper oder die Antikörper und das Polypeptid oder die Polypeptide nachgewiesen werden. In einer bevorzugten Ausführungsform bezeichnet der Begriff "Bedingungen, die mit der Bildung des Komplexes kompatibel sind" Bedingungen, die das Ausbilden der spezifischen Antigen-Antikörper Interaktionen zum Aufbau des Komplexes umfassend das Polypeptid und den Antikörper gestatten. In einer bevorzugten Ausführungsform können solche Bedingungen das Inkubieren des Polypeptides in einer Probe verdünnt 1:100 in PBS-Puffer 30 Minuten lang bei 25 °C umfassen. In einer bevorzugten Ausführungsform bedeutet der Begriff "Autoantikörper", wie hierin verwendet, einen Antikörper, der spezifisch an ein endogenes Molekül des Tieres, bevorzugt Säugetieres, bindet, das den Autoantikörper selbst herstellt, wobei die Konzentration des Autoantikörpers bevorzugt gegenüber dem Durchschnitt irgendwelcher Antikörper, die gegen ein solches endogenes Molekül binden, bevorzugt gegenüber einer gesunden Person, erhöht ist. In einer bevorzugtesten Ausführungsform ist der Autoantikörper ein Autoantikörper gegen Laminin beta-4.

Das erfindungsgemäße Verfahren ist bevorzugt ein *in vitro*-Verfahren.

In einer bevorzugten Ausführungsform umfasst der Nachweis des Komplexes für die Prognose, Diagnose, Verfahren oder den Testkit gemäß der vorliegenden Erfindung die Verwendung eines Verfahrens ausgewählt aus der Gruppe umfassend Immundiffusions-Tests, immunoelektrophoretische Tests, Lichtstreuungs-Immuntests, Agglutinations-Tests, Immuntests mit Markierungen wie die aus der Gruppe umfassend radiomarkierte Immuntests, Enzymimmuntests, bevorzugt ELISA, Chemilumineszenz Immuntests und Immunfluoreszenztests, bevorzugt indirekte Immunfluoreszenztests. Der Fachmann ist mit solchen Verfahren vertraut, und sie sind im Stand der Technik beschrieben, zum Beispiel in Zane, H. D. (2001), Immunology - Theoretical & Practical Concepts in Laboratory Medicine, W. B. Saunders Company, besonders in Kapitel 14.

Alternativ kann eine Gewebeprobe, d.h. bevorzugt eine Probe umfassend Gewebe, umfassend das erfindungsgemäße Polypeptid statt einer flüssigen Probe verwendet werden, insbesondere Spalthaut, die hergestellt werden kann, wie im Stand der Technik beschrieben ist (Zillikens, D., Kawahara, Y., Ishiko, A., Shimizu, H., Mayer, J., Rank, C. V., Liu, Z., Giudice, G. J., Tran, H. H., Marinkovich, M. P., Brocker, E. B., and Hashimoto, T., A novel subepidermal blistering disease with autoantibodies to a 200-kDa antigen of the basement membrane zone. J Invest Dermatol, 1996. 106(6): p. 1333-8.). Die Gewebeprobe stammt bevorzugt aus einem Gewebe umfassend endogenes Laminin beta-4, bevorzugt exprimierend das Polypeptid in einem erhöhten Ausmaß gegenüber dem durchschnittlichen Gewebe in dem entsprechenden Organismus, bevorzugt dem menschlichen Körper. Eine solche Probe, die dann in der Form eines Gewebeschnittes fixiert auf einem Träger, zum Beispiel einem Objektträger für mikroskopische Analysen, vorliegen kann, kann dann mit dem erfindungsgemäßen Antikörper, bevorzugt Autoantikörper, der gegen das erfindungsgemäße Polypeptid bindet, kontaktiert werden. Der Antikörper wird bevorzugt markiert, um die Unterscheidung von endogenem Autoantikörper zu ermöglichen, der gegen das erfindungsgemäße Polypeptid bindet, sodass neu gebildete Komplexe mit Autoantikörpern aus einer nachfolgend kontaktierten Probe, die mit dem ersten Antikörper konkurrieren, nachgewiesen und optional quantifiziert werden können. Wenn die Zahl der neu gebildeten Komplexe höher ist als die Zahl, die mit einer Probe von einer gesunden Person gefunden wird, ist es wahrscheinlich, dass die Person, von der die untersuchte Probe erhalten wurde, an der Krankheit leidet.

Jegliche Daten, die die Anwesenheit oder Abwesenheit des Komplexes umfassend den Antikörper und das erfindungsgemäße Polypeptid zeigen, können mit Referenzdaten verglichen werden. Zum Beispiel zeigt das Nachweisen des Komplexes an, dass der Patient, von dem die analysierte Probe erhalten wurde, an einer Krankheit litt, an ihr leidet oder in der Zukunft wahrscheinlich an der Krankheit leidet. Wenn ein Patient zuvor diagnostiziert wurde und das Verfahren zum Erhalten diagnostisch relevanter Informationen wieder durchgeführt wird, kann die Menge des Komplexes, die bei beiden Durchgängen erhalten wurden, korreliert werden, um das Fortschreiten der Krankheit und/oder den Erfolg einer Behandlung herauszufinden. Wird zum Beispiel gefunden, dass die Menge des Komplexes zugenommen hat, legt dies nahe, dass die Krankheit fortschreitet, sich wahrscheinlich in der Zukunft manifestieren wird und/oder das eine versuchte Behandlung nicht erfolgreich ist und umgekehrt. Der erstmalige Nachweis des Komplexes zeigt an, dass der Patient, von dem die Probe stammt, unter einem hohen oder erhöhten Risiko leidet, die Krankheit in Zukunft zu entwickeln.

In einer bevorzugten Ausführungsform wird eine Mikrotiterplatte, eine Membran, ein Blot wie ein Dotblot oder Linienblot eingesetzt, um das diagnostische Verfahren gemäß der Erfindung auszuführen. Der Fachmann ist mit dem experimentellen Aufbau vertraut, und dieser ist im Stand der Technik beschrieben (Raoult, D., and Dasch, G. A. (1989), The line blot: an immunoassay for monoclonal and other antibodies. Its application to the serotyping of gramnegative bacteria. J. Immunol. Methods, 125 (1- 2), 57-65; WO2013041540).

In einer weiteren bevorzugten Ausführungsform ist für die Prognose, Diagnose, die Verfahren oder der Kit gemäß der erfindungsgemäßen Lehre die Verwendung von indirekter Immunfluoreszenz vorgesehen. Der Fachmann ist mit derartigen Techniken und der Zubereitung geeigneter Proben vertraut, und diese sind im Stand der Technik beschrieben (US4647543; Voigt, J., Krause, C., Rohwäder, E, Saschenbrecker, S., Hahn, M., Danckwardt, M., Feirer, C., Ens, K, Fechner, K, Barth, E, Martinetz, T., and Stöcker, W. (2012), Automated Indirect Immunofluorescence Evaluation of Antinuclear Autoantibodies on HEp-2 Cells," Clinical and Developmental Immunology, vol. 2012, doi:10.1155/2012/65105; Bonilla, E., Francis, L., Allam, F., et al., Immuno-fluorescence microscopy is superior to fluorescent beads for detection of antinuclear antibody reactivity in systemic lupus erythematosus patients, Clinical Immunology, vol. 124, no. 1, pp. 18-21, 2007). Geeignete Reagenzien, Vorrichtungen und Softwarepakete sind im Handel erhältlich, zum Beispiel von EUROIMMUN, Lübeck, Deutschland.

Gemäß der erfindungsgemäßen Lehre, wird ein Antikörper, bevorzugt ein Autoantikörper, der gegen das erfindungsgemäße Polypeptid bindet, das für die Diagnose einer Krankheit verwendet wird, bereitgestellt. Der Fachmann auf dem Gebiet ist vertraut mit Verfahren zum Aufreinigen von Antikörpern, zum Beispiel denjenigen, die in Hermanson, G. T., Mallia, A. K., and Smith, P. K. (1992), Immobilized Affinity Ligand Techniques, San Diego: Academic Press, beschrieben sind. Kurz gesagt wird ein Antigen, das spezifisch gegen den interessierenden Autoantikörper bindet, wobei das Antigen das erfindungsgemäße Polypeptid ist, immobilisiert und verwendet, um über Affinitätschromatografie den interessierenden Autoantikörper aus einer geeigneten Quelle aufzureinigen. Eine flüssige Probe umfassend Antikörper aus einem Patienten, der an neurologischer Störung beziehungsweise Krankheit leidet, die mit dem Auftreten des Autoantikörpers assoziiert ist, kann als Quelle herangezogen werden.

Erfindungsgemäß wird ein Antikörper, zum Beispiel ein Autoantikörper bereitgestellt, der in der Lage ist, spezifisch an das erfindungsgemäße Polypeptid zu binden. In einer bevorzugten Ausführungsform bezeichnet der Begriff "Antikörper", wie hierin verwendet, jegliche Immunglobulin-basierte bindende Einheiten, bevorzugt eine Einheit umfassend wenigstens eine schwere Kette und eine leichte Kette eines Immunglobulins, umfassend, wenn nicht begrenzt auf monoklonale und polyklonale Antikörper wie auch Varianten eines Antikörpers, insbesondere Fragmente, wobei die bindenden Einheiten in der Lage sind, gegen das entsprechende Antigen zu binden, bevorzugter spezifisch an es zu binden. In einer bevorzugten Ausführungsform bedeutet der Begriff "spezifisch binden", wie hierin verwendet, dass die Bindung stärker als eine Bindungsreaktion ist, die durch eine Dissoziationskonstante gekennzeichnet ist, die 1 x 10⁻⁵ M, bevorzugter 1 x 10⁻⁷ M, bevorzugter 1 x 10⁻⁸ M, bevorzugter 1 x 10⁻⁹ M, bevorzugter 1 x 10⁻¹⁰ M, bevorzugter 1 x 10⁻¹¹ M, bevorzugter 1 x 10⁻¹² M beträgt, wie durch Surface Plasmon Resonanz unter Verwendung von Biacore-Ausrüstung bei 25°C in PBS Buffer bei pH 7 bestimmt wird. Der Antikörper ist bevorzugt rekombinant und/oder isoliert. Der erfindungsgemäß nachgewiesene oder bereitgestellte Autoantikörper bindet bevorzugt spezifisch an ein Epitop aus der Sequenz SEQ ID NO 27 umfassend eine oder mehr als eine Sequenz aus der Gruppe umfassend die Sequenzen SEQ ID NO 8, SEQ ID NO 9, SEQ ID NO 10, SEQ ID NO 11, SEQ ID NO 12, SEQ ID NO 13 und SEQ ID NO 14. Der Autoantikörper ist bevorzugt ein IgG-Antikörper.

Der Antikörper kann Teil einer Autoantikörperzubereitung sein, die heterogen ist, oder kann ein homogener Autoantikörper sein, wobei eine heterogene Zubereitung eine Vielzahl von verschiedenen Autoantikörper-Spezies umfasst, wie sie durch Zubereitung aus den Seren von menschlichen Spendern erhalten werden kann, beispielsweise durch Affinitätschromatografie unter Verwendung des immobilisierten Antigens, zum Aufreinigen irgendeines Autoantikörpers, der in der Lage ist, an das Antigen zu binden. Der Antikörper kann glykosyliert oder nicht glykosyliert sein. Der Fachmann auf dem Gebiet ist mit Verfahren vertraut, die zur Identifizierung, Produktion und Aufreinigung von Antikörpern und Varianten davon eingesetzt werden können, zum Beispiel diejenigen, die in EP 2 423 226 A2 und Referenzen darin beschrieben sind. Der Antikörper kann selbst oder in Kombination als diagnostisches Agens eingesetzt werden, zum Beispiel im Komplex mit dem erfindungsgemäßen Polypeptid.

Die vorliegende Erfindung stellt ein Verfahren zum Isolieren eines Antikörpers bereit, bevorzugt eines Autoantikörpers, der gegen das erfindungsgemäße Polypeptid bindet, umfassend die Schritte a) Kontaktieren einer Probe umfassend den Antikörper mit dem erfindungsgemäßen Polypeptid, derart, dass ein Komplex gebildet wird, b) Isolieren des Komplexes, der in a) gebildet wird, c) Dissoziieren des Komplexes, der in Schritt b) isoliert wurde, und d) Abtrennen des Antikörpers von dem erfindungsgemäßen Polypeptid. Eine Probe von einem Patienten, der an Anti-p200-Pemphigoid leidet, kann als Quelle für den Autoantikörper herangezogen werden. Geeignete Methoden sind im Stand der Technik beschrieben, zum Beispiel in den Handbüchern "Affinitätschromatografie", "Strategies for Protein Purification" and "Antibody Purification" (2009/2010), veröffentlicht von GE Healthcare Life Sciences, und in Philips, Terry, M., Analytical techniques in immunochemistry, 1992, Marcel Dekker, Inc.

Die Erfindung stellt eine pharmazeutische Zusammensetzung umfassend das erfindungsgemäße Polypeptid bereit, wobei die Zusammensetzung bevorzugt zur Verabreichung an ein Subjekt geeignet ist, bevorzugt ein Säugetier-Subjekt, bevorzugter ein Mensch. Eine derartige pharmazeutische Zusammensetzung kann einen pharmazeutisch akzeptablen Träger umfassen. Die pharmazeutische Zusammensetzung kann zum Beispiel oral, parenteral, über ein Inhalationsspray, topisch, über Augentropfen, rektal, nasal, bukkal, vaginal oder über ein implantiertes Reservoir verabreicht werden, wobei der Begriff "parenteral", wie hierin verwendet, subkutane, intrakutane, intravenöse, intramuskuläre, intraartikuläre, intrasynoviale, intrasternale, intrathekale, intralesionale und intrakraniale Injektions- oder Infusionstechniken umfasst. Die pharmazeutische Zusammensetzung kann in geeigneten Dosierungsformen bereitgestellt werden, zum Beispiel Kapseln, Tabletten und wässrigen Suspensionen und Lösungen, bevorzugt in steriler Form. Sie kann bei einem Behandlungsverfahren eingesetzt werden, wobei das Verfahren das Verabreichen einer wirksamen Menge des erfindungsgemäßen Polypeptides an ein Subjekt umfasst.

Im Schutzbereich der vorliegenden Erfindung wird eine medizinische oder diagnostische Vorrichtung umfassend, bevorzugt beschichtet mit einem Reagenz zum Detektieren des erfindungsgemäßen (Auto)Antikörpers und/oder das erfindungsgemäße Polypeptid bereitgestellt. Bevorzugt umfasst eine solche medizinische oder diagnostische Vorrichtung das erfindungsgemäße Polypeptid in einer Form, die das Kontaktieren des Polypeptides mit einer wässrigen Lösung, bevorzugter der flüssigen menschlichen Probe, in einer einfachen Weise gestattet. Insbesondere kann das erfindungsgemäße Polypeptid auf der Oberfläche eines Trägers immobilisiert werden, bevorzugt ausgewählt aus der Gruppe umfassend Glasplatten oder -träger, Objektträger, Biochips, Mikrotiterplatten, Beads, zum Beispiel magnetische Beads, Apherese-Vorrichtungen, Chromatographie-Säulen, Membranen oder dergleichen. Beispielhafte medizinische Vorrichtungen umfassen Linienblots, Mikrotiterplatten, Glasträger für Mikroskopie, Beads, bevorzugt magnetische Beads, und Biochips. Zusätzlich zum erfindungsgemäßen Polypeptid kann die diagnostische Vorrichtung weitere Polypeptide umfassen, zum Beispiel Positiv- oder Negativ-Kontrollen wie Proben, die einen Antikörper gegen das interessierende Polypeptid enthalten bzw. nicht enthalten, oder andere bekannte Antigene, die gegen Autoantikörper von diagnostischem Wert binden, insbesondere betreffend andere Krankheiten, die mit einem oder mehr als einem identischen oder ähnlichen Symptom assoziiert sind.

Im Schutzbereich des vorliegenden Erfindung wird auch eine therapeutische Vorrichtung umfassend, bevorzugt beschichtet mit Laminin beta-4 oder einer Variante davon bereitgestellt, die so beschaffen ist, dass der Autoantikörper gegen Laminin beta-4 aus dem Blut eines Patienten entfernt werden kann, wobei die Blutzusammensetzung möglichst wenig verändert und insbesondere möglichst wenig essentielle Komponenten entfernt werden. Dabei kann es sich um eine Apherese-Vorrichtung handeln, bevorzugt zur Plasmapherese. Geeignete Reagenzien und Verfahren sind in der EP17001759.4 und in der WO2007/085240 beschrieben, die hierin in ihrer Gesamtheit durch Bezugnahme aufgenommen sind. Im Stand der Technik ist bereits beschrieben, dass die spezifische Immunadsorption zur Behandlung von bullösen Pemphigoid vielversprechend ist (Mersmann et al., Arch Dermatol Res 2015: "Immunoadsorber for specific apheresis of autoantibodies in the treatment of bullous pemphigoid"). Bevorzugt ist die Vorrichtung in dem Sinne sterilisiert, dass ein- und austretendes Blut nur mit sterilisierten Komponenten in Kontakt tritt.

Gemäß der erfindungsgemäßen Lehre wird ein Kit bereitgestellt, bevorzugt zum Diagnostizieren einer Krankheit. Ein solches Kit kann Instruktionen umfassen, die im Detail beschreiben, wie das Kit zu benutzen ist, sowie den erfindungsgemäßen Träger als Mittel zum Kontaktieren des erfindungsgemäßen Polypeptides mit einer Körperflüssigkeitsprobe aus einem Subjekt, bevorzugt einem menschlichen Subjekt. Weiterhin kann das Kit eine Positivkontrolle umfassen, zum Beispiel eine Charge Autoantikörper oder rekombinanten Antikörper, von dem bekannt ist, dass er gegen das Peptid gemäß der vorliegenden Erfindung bindet, oder eine verdünnte Probe eines Patienten umfassend den Autoantikörper. Das Kit kann eine Negativkontrolle enthalten, zum Beispiel ein Protein, das keine nachweisbare Affinität zum erfindungsgemäßen Polypeptid hat, wie Rinderserumalbumin, oder eine Probe von einem Gesunden. Ein solches Kit kann eine oder mehr als eine Standardlösung des Antikörpers zum Erstellen einer Kalibrationskurve umfassen, auch bezeichnet als Kalibrator, wobei der Antikörper ein rekombinanter und/oder monoklonaler Antikörper oder der verdünnte Antikörper aus einer Probe eines Patienten sein kann, bevorzugt gegen ein Epitop aus der Gruppe umfassend SEQ ID NO 8, SEQ ID NO 9, SEQ ID NO 10, SEQ ID NO 11, SEQ ID NO 12, SEQ ID NO 13 und SEQ ID NO 14. Das Kit kann weiterhin einen sekundären Antikörper, bevorzugt mit einer detektierbaren Markierung, aufweisen, der einen Autoantikörper gegen Laminin beta-4 erkennt, bevorzugt einen sekundären Antikörper, der IgG-Antikörper erkennt.

In einer bevorzugten Ausführungsform umfasst das Kit ein Mittel zum Detektieren eines Autoantikörpers, der gegen das erfindungsgemäße Polypeptid bindet, bevorzugt durch Nachweisen eines Komplexes umfassend das erfindungsgemäße Polypeptid und einen Antikörper, der gegen das erfindungsgemäße Polypeptid bindet. Ein solches Mittel ist bevorzugt ein Agens, das gegen den Komplex bindet und den Komplex modifiziert oder eine Markierung trägt, die den Komplex nachweisbar macht. Zum Beispiel kann ein solches Mittel ein markierter Antikörper sein, der gegen das Polypeptid an einer Bindungsstelle bindet, die sich von der Bindungsstelle unterscheidet, an die der primäre Autoantikörper bindet, oder der gegen eine konstante Region des primären Antikörpers bindet. Alternativ kann das Agens ein sekundärer Antikörper gegen Laminin beta-4 sein, der gegen die konstante Region des Autoantikörpers bindet, bevorzugt ein sekundärer Antikörper, der für Säugetier-Antikörper der IgG-Klasse spezifisch ist. Eine Vielzahl von Verfahren und Mitteln zum Nachweisen eines solchen Komplexes ist im Stand der Technik beschrieben, zum Beispiel in Philips, Terry, M., Analytical techniques in immunochemistry, 1992, Marcel Dekker, Inc.

Das Polypeptid umfassend Laminin beta-4 oder eine Variante davon kann hergestellt oder bereitgestellt werden in Form einer Zelle, die eine Nukleinsäure kodierend das Polypeptid umfasst und/oder exprimiert. Wenn eine Nukleinsäure umfassend eine Sequenz, die für das erfindungsgemäße Polypeptid oder eine Variante davon kodiert, verwendet wird, kann eine solche Nukleinsäure eine unmodifizierte Nukleinsäure sein. In einer bevorzugten Ausführungsform ist die Nukleinsäure eine Nukleinsäure, die als solche nicht in der Natur vorkommt und verglichen zu einer natürlichen Nukleinsäure wenigstens eine Modifizierung umfasst, zum Beispiel ein Isotop enthält oder eine chemische Modifizierung, zum Beispiel eine Methylierung, eine Sequenzmodifizierung, eine Markierung oder dergleichen, die den synthetischen Ursprung anzeigt. In einer bevorzugten Ausführungsform ist die Nukleinsäure eine rekombinante Nukleinsäure oder, in einer bevorzugteren Ausführungsform, Teil eines Vektors, in dem sie funktionell verbunden mit einem Promotor ist, der die Expression, bevorzugt Überexpression der Nukleinsäure gestattet. Der Fachmann ist mit einer Vielzahl von geeigneten Vektoren vertraut, die kommerziell erhältlich sind, zum Beispiel von *Origene.* Zum Beispiel kann ein Vektor kodierend für Fusionskonstrukte mit einem C-terminalen GFP verwendet werden. Der Vektor kann einen bakteriellen Replikationsursprung und einen Promotor zur Expression in eukaryontischen, bevorzugt Säugetierzellen aufweisen, beispielsweise einen SV40-Promotor oder Chicken beta-actin Promotor. Die Zelle kann eine eukaryotische oder prokaryotische Zelle sein, bevorzugt eine eukaryotische Zelle, wie beispielweise eine Hefezelle, und ist bevorzugter eine Säugetierzelle, noch bevorzugter eine menschliche Zelle wie eine HEK293-Zelle. Beispiele für eine Säugetierzelle umfassen HEK293, CHO oder COS-7-Zellen. Die Zelle umfassend die Nukleinsäure kodierend für das erfindungsgemäße Polypeptid kann eine rekombinante Zelle sein oder eine isolierte Zelle, wobei der Begriff "isoliert" bedeutet, dass die Zelle derart angereichert ist, dass in ihrer Umgebung im Vergleich zum Wildtyp der Zelle oder dessen Umgebung weniger Zellen anderer Differenzierung oder Spezies oder tatsächlich gar keine anderen solcher Zellen anwesend sind. Die Zelle oder der Vektor können zur Expression des Polypeptids verwendet werden, gefolgt von dessen Verwendung zu Herstellung einer Zusammensetzung oder eines Kits zur Diagnose einer Autoimmunkrankheit. Die Zelle kann lebende oder eine fixierte, nicht mehr stoffwechselaktive Zelle sein. Das Fixieren von Zellen ist durch Behandlung mit Aceton oder Formalin möglich, wie in Probst *et al.* beschrieben ist (Probst, C. Saschenbrecker, S., Stoecker, W., und Komorowski, L (2014) Anti-neuronal autoantibodies: Current diagnostic challenges, Multiple Sclerosis and Related Disorders 3, 303-320).

Die erfindungsgemäße Lehre kann nicht nur für eine Diagnose aber auch zum Verhindern oder zum Behandeln einer Krankheit eingesetzt werden, genauer für ein Verfahren zum Verhindern oder Behandeln einer Krankheit, umfassend die Schritte a) Verringern der Konzentration von Autoantikörpern, die gegen das erfindungsgemäße Polypeptid binden, im Blut eines Subjekts und/oder b) Verabreichen von einer oder mehr als einer immunsuppressiven pharmazeutischen Substanz, bevorzugt aus der Gruppe umfassend Rituximab, Prednisone, Methylprednisolon, Cyclophosphamid, Mycophenolatemofetil, intravenöse Immunoglobuline, FK506, Cyclosporin, Methotrexat, Clobetasolpropionat, Dapson, Tetracyclin, Nicotinamid, Colchicin und Azathioprin.

In einer bevorzugten Ausführungsform stellt die vorliegende Erfindung eine Verwendung eines Mittels oder Reagenz für die Detektion eines Antikörpers gegen Laminin beta-4 oder einer Nukleinsäure umfassend eine Sequenz kodierend Laminin beta-4 oder eine Variante davon oder eines Vektors umfassend diese Sequenz oder einer Zelle umfassend den Vektor oder diese Nukleinsäure zur Herstellung eines Kits für die Diagnose einer Autoimmunkrankheit bereit.

In einer bevorzugten Ausführungsform kann jegliches Verfahren oder jegliche Verwendung gemäß der vorliegenden Erfindung für einen nicht-diagnostischen Zweck beabsichtigt sein, insbesondere Bestimmen der Anwesenheit eines Autoantikörpers gegen Laminin beta-4 für eine andere Verwendung als das Diagnostizieren eines Patienten. Zum Beispiel kann das Verfahren oder die Verwendung zum *in vitro*-Testbestimmen der Effizienz einer medizinischen Vorrichtung bestimmt sein, die dazu gestaltet ist, einen Autoantikörper aus dem Blut eines Patienten zu entfernen, wobei das Testen mit einer Flüssigkeit durchgeführt wird, die nicht Patientenblut ist. In einer bevorzugten Ausführungsform kann ein beliebiges Verfahren und eine beliebige Verwendung gemäß der vorliegenden Erfindung mit der Absicht verwendet werden, ein Autoantikörper-Profil zu generieren, bevorzugt zum Nachweisen einer Krankheit in einem Säugetier, bevorzugt einem Menschen. In einer bevorzugten Ausführungsform kann ein Verfahren oder eine Verwendung zum Nachweisen krankheits-assoziierter Marker in einer Probe aus Patienten verwendet werden, die unter einer Hautkrankheit leiden.

In einer bevorzugten Ausführungsform ist das Verfahren gemäß der vorliegenden Erfindung ein Verfahren zum Kalibrieren eines diagnostischen Testsystems oder zum Bestätigen der Zuverlässigkeit und/oder ausreichenden Kapazität eines solchen Testsystems oder eines therapeutischen Systems zum Entfernen von Autoantikörpern aus dem Blut eines Patienten. Im Falle eines diagnostischen Testsystems, werden Autoantikörper nicht in der Probe eines Patienten nachgewiesen, der zu diagnostizieren ist, aber in einer künstlichen Lösung mit bekannter Zusammensetzung, insbesondere mit einer definierten, bekannten Konzentration des Antikörpers oder eines rekombinanten Antikörpers von bekannter Konzentration, der gegen das erfindungsgemäße Polypeptid bindet. Das diagnostische System kann jegliches System sein, das den Nachweis von Autoantikörpern in einer Probe gestattet, beispielsweise eine medizinische oder diagnostische Vorrichtung gemäß der vorliegenden Erfindung.

Im Falle eines therapeutischen Systems, z.B. eines Apparates zur Apherese, kann das Verfahren verwendet werden, um ein solches System zu entwickeln und seine Zuverlässigkeit und/oder Effizienz und/oder Kapazität zu testen. Zum Beispiel kann nach einem Aphereselauf oder davor eine Lösung umfassend eine bekannte Konzentration eines Antikörpers der gegen das erfindungsgemäße Polypeptid bindet, mit dem System kontaktiert werden, und das Verfahren gemäß der vorliegenden Erfindung kann eingesetzt werden, um zu bestätigen, dass das System in der Lage ist, den Antikörper aus der Lösung zu entfernen.

In einer bevorzugten Ausführungsform wird erfindungsgemäß ein Apparat zum Analysieren einer Probe von einem Patienten bereitgestellt, um einen oder mehr als einen Autoantikörper gegen Laminin beta-4 zu detektieren, wobei der oder die Autoantikörper eine erhöhte Wahrscheinlichkeit anzeigt, dass der Patient unter einer Autoimmunkrankheit leidet, wobei der Apparat folgendes umfasst:
a. einen Träger, der ein Mittel zum Einfangen von wenigstens einem Autoantikörper in der Probe umfasst, wenn die Probe mit dem Träger kontaktiert wird,
b. ein detektierbares Mittel, das in der Lage ist, an den eingefangenen Antikörper zu binden, wenn das detektierbare Mittel mit dem Träger kontaktiert wird, wobei das detektierbare Mittel bevorzugt ein markierter sekundärer Antikörper ist, der in der Lage ist, an den Antikörper zu binden, der auf dem Träger eingefangen ist,
c. optional ein Mittel zum Entfernen einer Probe von dem Träger and zum Entfernen des detektierbaren Mittels, bevorzugt durch Waschen;
d. eine Detektiervorrichtung zum Detektieren der Anwesenheit des detektierbaren Mittels und zum Umwandeln der Ergebnisse in ein elektrisches Signal, und
e. optional ein Mittel zum Empfangen des elektrischen Signals von der Detektiervorrichtung und Bestimmen, ob die Stärke des Signals eine erhöhte Wahrscheinlcihkeit einer Autoimmunkrankheit anzeigt, durch Vergleichen der Stärke des Signals mit der Stärke eines Hintergrundsignals oder mit einem eingegebenen Referenzwert, der mit proben von gesunden Personen erhalten wurde.

Die vorliegende Anmeldung umfasst eine Reihe von Sequenzen neuer Nucleinsäuren oder Polypeptide:
**SEQ ID NO 1: Primer sense LAMB4:**
   ATAGGTCTCACATGCAATTTCAACTGACCCTTTTTTTGCACCTTG
**SEQ ID NO 2: Primer asense LAMB4:**
   ATAGGTCTCGTCGAGGCTATAGCACCTAGCATATTTTTTTTCTTG
**SEQ ID NO 3: Primer asense LAMB4-Stop:**
   ATAGGTCTCCTCGAGCTAGCTATAGCACCTAGCATATTTTTTTTCTTG

SEQ ID NO 4_pTriEx-1 (Standard-Vektor)
GGGGAATTGTGAGCGGATAACAATTCCCCGGAGTTAATCCGGGACCTTTAATTCAACCC
AACACAATATATTATAGTTAAATAAGAATTATTATCAAATCATTTGTATATTAATTAAAATA
CTATACTGTAAATTACATTTTATTTACAATCAAAGGAGATATACCATGGCGATATCCCGG
GAGCTCGTGGATCCGAATTCTCAGATCTCGGCGCGCCTGCAGGTCGACGGTACCGGTT
CGAAGCTTGCGGCCGCACAGCTGTATACACGTGCAAGCCAGCCAGAACTCGCCCCGG
AAGACCCCGAGGATCTCGAGCACCACCATCACCATCACCATCACTAAGTGATTAACCTC
AGGTGCAGGCTGCCTATCAGAAGGTGGTGGCTGGTGTGGCCAATGCCCTGGCTCACA
AATACCACTGAGATCGATCTTTTTCCCTCTGCCAAAAATTATGGGGACATCATGAAGCC
CCTTGAGCATCTGACTTCTGGCTAATAAAGGAAATTTATTTTCATTGCAATAGTGTGTTG
GAATTTTTTGTGTCTCTCACTCGGAAGGACATATGGGAGGGCAAATCATTTAAAACATC
AGAATGAGTATTTGGTTTAGAGTTTGGCAACATATGCCCATATGTAACTAGCATAACCCC
TTGGGGCCTCTAAACGGGTCTTGAGGGGTTTTTTGCTGAAAGCATGCGGAGGAAATTC
TCCTTGAAGTTTCCCTGGTGTTCAAAGTAAAGGAGTTTGCACCAGACGCACCTCTGTTC
ACTGGTCCGGCGTATTAAAACACGATACATTGTTATTAGTACATTTATTAAGCGCTAGAT
TCTGTGCGTTGTTGATTTACAGACAATTGTTGTACGTATTTTAATAATTCATTAAATTTAT
AATCTTTAGGGTGGTATGTTAGAGCGAAAATCAAATGATTTTCAGCGTCTTTATATCTGA
ATTTAAATATTAAATCCTCAATAGATTTGTAAAATAGGTTTCGATTAGTTTCAAACAAGGG
TTGTTTTTCCGAACCGATGGCTGGACTATCTAATGGATTTTCGCTCAACGCCACAAAACT
TGCCAAATCTTGTAGCAGCAATCTAGCTTTGTCGATATTCGTTTGTGTTTTGTTTTGTAA
TAAAGGTTCGACGTCGTTCAAAATATTATGCGCTTTTGTATTTCTTTCATCACTGTCGTT
AGTGTACAATTGACTCGACGTAAACACGTTAAATAGAGCTTGGACATATTTAACATCGG
GCGTGTTAGCTTTATTAGGCCGATTATCGTCGTCGTCCCAACCCTCGTCGTTAGAAGTT
GCTTCCGAAGACGATTTTGCCATAGCCACACGACGCCTATTAATTGTGTCGGCTAACAC
GTCCGCGATCAAATTTGTAGTTGAGCTTTTTGGAATTATTTCTGATTGCGGGCGTTTTTG
GGCGGGTTTCAATCTAACTGTGCCCGATTTTAATTCAGACAACACGTTAGAAAGCGATG
GTGCAGGCGGTGGTAACATTTCAGACGGCAAATCTACTAATGGCGGCGGTGGTGGAG

CTGATGATAAATCTACCATCGGTGGAGGCGCAGGCGGGGCTGGCGGCGGAGGCGGA
GGCGGAGGTGGTGGCGGTGATGCAGACGGCGGTTTAGGCTCAAATGTCTCTTTAGGC
AACACAGTCGGCACCTCAACTATTGTACTGGTTTCGGGCGCCGTTTTTGGTTTGACCGG
TCTGAGACGAGTGCGATTTTTTTCGTTTCTAATAGCTTCCAACAATTGTTGTCTGTCGTC
TAAAGGTGCAGCGGGTTGAGGTTCCGTCGGCATTGGTGGAGCGGGCGGCAATTCAGA
CATCGATGGTGGTGGTGGTGGAGGCGCTGGAATGTTAGGCACGGGAGAAGGTGG
TGGCGGCGGTGCCGCCGGTATAATTTGTTCTGGTTTAGTTTGTTCGCGCACGATTGTG
GGCACCGGCGCAGGCGCCGCTGGCTGCACAACGGAAGGTCGTCTGCTTCGAGGCAG
CGCTTGGGGTGGTGGCAATTCAATATTATAATTGGAATACAAATCGTAAAATCTGCTAT
AAGCATTGTAATTTCGCTATCGTTTACCGTGCCGATATTTAACAACCGCTCAATGTAAGC
AATTGTATTGTAAAGAGATTGTCTCAAGCTCGGAACGCTGCGCTCGGTCGTTCGGCTG
CGGCGAGCGGTATCAGCTCACTCAAAGGCGGTAATACGGTTATCCACAGAATCAGGGG
ATAACGCAGGAAAGAACATGTGAGCAAAAGGCCAGCAAAAGGCCAGGAACCGTAAAAA
GGCCGCGTTGCTGGCGTTTTTCCATAGGCTCCGCCCCCCTGACGAGCATCACAAAAAT
CGACGCTCAAGTCAGAGGTGGCGAAACCCGACAGGACTATAAAGATACCAGGCGTTTC
CCCCTGGAAGCTCCCTCGTGCGCTCTCCTGTTCCGACCCTGCCGCTTACCGGATACCT
GTCCGCCTTTCTCCCTTCGGGAAGCGTGGCGCTTTCTCAATGCTCACGCTGTAGGTAT
CTCAGTTCGGTGTAGGTCGTTCGCTCCAAGCTGGGCTGTGTGCACGAACCCCCCGTTC
AGCCCGACCGCTGCGCCTTATCCGGTAACTATCGTCTTGAGTCCAACCCGGTAAGACA
CGACTTATCGCCACTGGCAGCAGCCACTGGTAACAGGATTAGCAGAGCGAGGTATGTA
GGCGGTGCTACAGAGTTCTTGAAGTGGTGGCCTAACTACGGCTACACTAGAAGGACAG
TATTTGGTATCTGCGCTCTGCTGAAGCCAGTTACCTTCGGAAAAAGAGTTGGTAGCTCT
TGATCCGGCAAACAAACCACCGCTGGTAGCGGTGGTTTTTTTGTTTGCAAGCAGCAGAT
TACGCGCAGAAAAAAAGGATCTCAAGAAGATCCTTTGTTACCAATGCTTAATCAGTGAG
GCACCTATCTCAGCGATCTGTCTATTTCGTTCATCCATAGTTGCCTGACTCCCCGTCGT
GTAGATAACTACGATACGGGAGGGCTTACCATCTGGCCCCAGTGCTGCAATGATACCG
CGAGACCCACGCTCACCGGCTCCAGATTTATCAGCAATAAACCAGCCAGCCGGAAGGG
CCGAGCGCAGAAGTGGTCCTGCAACTTTATCCGCCTCCATCCAGTCTATTAATTGTTGC
CGGGAAGCTAGAGTAAGTAGTTCGCCAGTTAATAGTTTGCGCAACGTTGTTGCCATTGC
TACAGGCATCGTGGTGTCACGCTCGTCGTTTGGTATGGCTTCATTCAGCTCCGGTTCC
CAACGATCAAGGCGAGTTACATGATCCCCCATGTTGTGCAAAAAAGCGGTTAGCTCCTT
CGGTCCTCCGATCGTTGTCAGAAGTAAGTTGGCCGCAGTGTTATCACTCATGGTTATGG
CAGCACTGCATAATTCTCTTACTGTCATGCCATCCGTAAGATGCTTTTCTGTGACTGGT
GAGTACTCAACCAAGTCATTCTGAGAATAGTGTATGCGGCGACCGAGTTGCTCTTGCCC
GGCGTCAATACGGGATAATACCGCGCCACATAGCAGAACTTTAAAAGTGCTCATCATTG

GAAAACGTTCTTCGGGGCGAAAACTCTCAAGGATCTTACCGCTGTTGAGATCCAGTTCG
ATGTAACCCACTCGTGCACCCAACTGATCTTCAGCATCTTTTACTTTCACCAGCGTTTCT
GGGTGAGCAAAAACAGGAAGGCAAAATGCCGCAAAAAAGGGAATAAGGGCGACACGG
AAATGTTGAATACTCATACTCTTCCTTTTTCAATATTATTGAAGCATTTATCAGGGTTATT
GTCTCATGTCCGCGCGTTTCCTGCATCTTTTAATCAAATCCCAAGATGTGTATAAACCAC
CAAACTGCCAAAAAATGAAAACTGTCGACAAGCTCTGTCCGTTTGCTGGCAACTGCAAG
GGTCTCAATCCTATTTGTAATTATTGAATAATAAAACAATTATAAATGTCAAATTTGTTTTT
TATTAACGATACAAACCAAACGCAACAAGAACATTTGTAGTATTATCTATAATTGAAAAC
GCGTAGTTATAATCGCTGAGGTAATATTTAAAATCATTTTCAAATGATTCACAGTTAATTT
GCGACAATATAATTTTATTTTCACATAAACTAGACGCCTTGTCGTCTTCTTCTTCGTATTC
CTTCTCTTTTTCATTTTTCTCTTCATAAAAATTAACATAGTTATTATCGTATCCATATATGT
ATCTATCGTATAGAGTAAATTTTTTGTTGTCATAAATATATATGTCTTTTTTAATGGGGTG
TATAGTACCGCTGCGCATAGTTTTTCTGTAATTTACAACAGTGCTATTTTCTGGTAGTTC
TTCGGAGTGTGTTGCTTTAATTATTAAATTTATATAATCAATGAATTTGGGATCGTCGGT
TTTGTACAATATGTTGCCGGCATAGTACGCAGCTTCTTCTAGTTCAATTACACCATTTTT
TAGCAGCACCGGATTAACATAACTTTCCAAAATGTTGTACGAACCGTTAAACAAAAACAG
TTCACCTCCCTTTTCTATACTATTGTCTGCGAGCAGTTGTTTGTTGTTAAAAATAACAGC
CATTGTAATGAGACGCACAAACTAATATCACAAACTGGAAATGTCTATCAATATATAGTT
GCTCTAGTTATTAATAGTAATCAATTACGGGGTCATTAGTTCATAGCCCATATATGGAGT
TCCGCGTTACATAACTTACGGTAAATGGCCCGCCTGGCTGACCGCCCAACGACCCCCG
CCCATTGACGTCAATAATGACGTATGTTCCCATAGTAACGCCAATAGGGACTTTCCATT
GACGTCAATGGGTGGACTATTTACGGTAAACTGCCCACTTGGCAGTACATCAAGTGTAT
CATATGCCAAGTACGCCCCCTATTGACGTCAATGACGGTAAATGGCCCGCCTGGCATTA
TGCCCAGTACATGACCTTATGGGACTTTCCTACTTGGCAGTACATCTACGTATTAGTCAT
CGCTATTACCATGCATGGTCGAGGTGAGCCCCACGTTCTGCTTCACTCTCCCCATCTCC
CCCCCCTCCCCACCCCCAATTTTGTATTTATTTATTTTTTAATTATTTTGTGCAGCGATGG
GGGCGGGGGGGGGGGGGGGCGCGCGCCAGGCGGGGCGGGGCGGGGCGAGGGG
CGGGGCGGGGCGAGGCGGAGAGGTGCGGCGGCAGCCAATCAGAGCGGCGCGCTCC
GAAAGTTTCCTTTTATGGCGAGGCGGCGGCGGCGGCGGCCCTATAAAAAGCGAAGCG
CGCGGCGGGCGGGAGTCGCTGCGACGCTGCCTTCGCCCCGTGCCCCGCTCCGCCGC
CGCCTCGCGCCGCCCGCCCCGGCTCTGACTGACCGCGTTACTCCCACAGGTGAGCGG
GCGGGACGGCCCTTCTCCTTCGGGCTGTAATTAGCGCTTGGTTTAATGACGGCTTGTT
TCTTTTCTGTGGCTGCGTGAAAGCCTTGAGGGGCTCCGGGAGGGCCCTTTGTGCGGG
GGGAGCGGCTCGGGGCTGTCCGCGGGGGGACGGCTGCCTTCGGGGGGGACGGGGC
AGGGCGGGGTTCGGCTTCTGGCGTGTGACCGGCGGCTCTAGAGCCTCTGCTAACCAT

GTTCATGCCTTCTTCTTTTCCTACAGCTCCTGGGCAACGTGCTGGTTATTGTGCTGTC
TCATCATTTTGGCAAAGAATTGGATCGGACCGAAATTAATACGACTCACTATA

**SEQ ID NO 5_pTriEx-1-LAMB4(IF1)[human] - kodiert für - Humanes Laminin beta-4 ohne His-Tag** 
GGAGTCGCTGCGACGCTGCCTTCGCCCCGTGCCCCGCTCCGCCGCCGCCTCGCGCC
GCCCGCCCCGGCTCTGACTGACCGCGTTACTCCCACAGGTGAGCGGGCGGGACGGC
CCTTCTCCTTCGGGCTGTAATTAGCGCTTGGTTTAATGACGGCTTGTTTCTTTTCTGTG
GCTGCGTGAAAGCCTTGAGGGGCTCCGGGAGGGCCCTTTGTGCGGGGGGAGCGGCT
CGGGGCTGTCCGCGGGGGGACGGCTGCCTTCGGGGGGGACGGGGCAGGGCGGGGT
TCGGCTTCTGGCGTGTGACCGGCGGCTCTAGAGCCTCTGCTAACCATGTTCATGCCTT
CTTCTTTTTCCTACAGCTCCTGGGCAACGTGCTGGTTATTGTGCTGTCTCATCATTTTGG
CAAAGAATTGGATCGGACCGAAATTAATACGACTCACTATAGGGGAATTGTGAGCGGAT
AACAATTCCCCGGAGTTAATCCGGGACCTTTAATTCAACCCAACACAATATATTATAGTT
AAATAAGAATTATTATCAAATCATTTGTATATTAATTAAAATACTATACTGTAAATTACATT
TTATTTACAATCAAAGGAGATATACCATGCAATTTCAACTGACCCTTTTTTTGCACCTTG
GGTGGCTCAGTTACTCAAAAGCTCAAGATGACTGCAACAGGGGTGCCTGTCATCCCAC
CACTGGTGATCTCCTGGTGGGCAGGAACACGCAGCTTATGGCTTCTTCTACCTGTGGG
CTGAGCAGAGCCCAGAAATACTGCATCCTCAGTTACCTGGAGGGGGAACAAAAATGCT
TCATCTGTGACTCTAGATTTCCATATGATCCGTATGACCAACCCAACAGCCACACCATTG
AGAATGTCATTGTAAGTTTTGAACCAGACAGAGAAAAGAAATGGTGGCAATCTGAAAAT
GGTCTTGATCATGTCAGCATCAGACTGGACTTAGAGGCATTATTTCGGTTCAGCCACCT
TATCCTGACCTTTAAGACTTTTCGGCCTGCTGCAATGTTAGTTGAACGTTCCACAGACTA
TGGACACAACTGGAAAGTGTTCAAATATTTTGCAAAAGACTGTGCCACTTCCTTTCCTAA
CATCACATCTGGCCAGGCCCAGGGAGTGGGAGACATTGTTTGTGACTCCAAATACTCG
GATATTGAACCCTCAACAGGTGGAGAGGTTGTTTTAAAAGTTTTGGATCCCAGTTTTGA
AATTGAAAACCCTTATAGCCCCTACATCCAAGACCTTGTGACATTGACAAACCTGAGGA
TAAACTTTACCAAGCTCCACACCCTTGGGGATGCTTTGCTTGGAAGGAGGCAAAATGAT
TCCCTTGATAAATACTACTATGCTCTGTACGAGATGATTGTTCGGGGAAGCTGCTTTTG
CAATGGCCATGCTAGCGAATGTCGCCCTATGCAGAAGATGCGGGGAGATGTTTTCAGC
CCTCCTGGAATGGTTCACGGTCAGTGTGTGTGTCAGCACAATACAGATGGTCCGAACT
GTGAGAGATGCAAGGACTTCTTCCAGGATGCTCCTTGGAGGCCAGCTGCAGACCTCCA
GGACAACGCTTGCAGATCGTGCAGCTGTAACAGCCACTCCAGCCGCTGTCACTTTGAC
ATGACTACGTACCTGGCAAGCGGTGGCCTCAGCGGGGGCGTGTGTGAAGACTGCCAG
CACAACACTGAGGGGCAGCACTGCGACCGCTGCAGACCCCTCTTCTACAGGGACCCG
 
CTCAAGACCATCTCAGATCCCTACGCGTGCATTCCTTGTGAATGTGACCCCGATGGGA
CCATATCTGGTGGCATTTGTGTGAGCCACTCTGATCCTGCCTTAGGGTCTGTGGCTGG
CCAGTGCCTTTGTAAAGAGAACGTGGAAGGAGCCAAATGCGACCAGTGCAAACCCAAC
CACTATGGACTAAGCGCCACCGACCCCCTGGGCTGCCAGCCCTGCGACTGTAACCCCC
TTGGGAGTCTGCCATTCTTGACCTGTGATGTGGATACAGGCCAATGCTTGTGCCTGTCA
TATGTCACCGGAGCACACTGCGAAGAATGCACTGTTGGATACTGGGGCCTGGGAAATC
ATCTCCATGGGTGTTCTCCCTGTGACTGTGATATTGGAGGTGCTTATTCTAACGTGTGC
TCACCCAAGAATGGGCAGTGTGAATGCCGCCCACATGTCACTGGCCGTAGCTGCTCTG
AACCAGCCCCTGGCTACTTCTTTGCTCCTTTGAATTTCTATCTCTACGAGGCAGAGGAA
GCCACAACACTCCAAGGACTGGCGCCTTTGGGCTCGGAGACGTTTGGCCAGAGTCCT
GCTGTTCACGTTGTTTAGGAGAGCCAGTTCCTGGGAACCCTGTTACATGGACTGGAC
CTGGATTTGCCAGGGTTCTCCCTGGGGCTGGCTTGAGATTTGCTGTCAACAACATTCC
CTTTCCTGTGGACTTCACCATTGCCATTCACTATGAAACCCAGTCTGCAGCTGACTGGA
CTGTCCAGATTGTGGTGAACCCCCCTGGAGGGAGTGAGCACTGCATACCCAAGACTCT
ACAGTCAAAGCCTCAGTCTTTTGCCTTACCAGCGGCTACGAGAATCATGCTGCTTCCCA
CACCCATCTGTTTAGAACCAGATGTACAATATTCCATAGATGTCTATTTTTCTCAGCCTT
TGCAAGGAGAGTCCCACGCTCATTCACATGTCCTGGTGGACTCTCTTGGCCTTATTCCC
CAAATCAATTCATTGGAGAATTTCTGCAGCAAGCAGGACTTAGATGAGTATCAGCTTCA
CAACTGTGTTGAAATTGCCTCAGCAATGGGACCTCAAGTGCTCCCGGGTGCCTGTGAA
AGGCTGATCATCAGCATGTCTGCCAAGCTGCATGATGGGGCTGTGGCCTGCAAGTGTC
ACCCCCAGGGCTCAGTCGGATCCAGCTGCAGCCGACTTGGAGGCCAGTGCCAGTGTA
AACCTCTTGTGGTCGGGCGCTGCTGTGACAGGTGCTCAACTGGAAGCTATGATTTGGG
GCATCACGGCTGTCACCCATGTCACTGCCATCCTCAAGGATCAAAGGACACTGTATGTG
ACCAAGTAACAGGACAGTGCCCCTGCCATGGAGAGGTGTCTGGCCGCCGCTGTGATC
GCTGCCTGGCAGGCTACTTTGGATTTCCCAGCTGCCACCCTTGCCCTTGTAATAGGTTT
GCTGAACTTTGTGATCCTGAGACAGGGTCATGCTTCAATTGTGGAGGCTTTACAACTGG
CAGAAACTGTGAAAGGTGTATTGATGGTTACTATGGAAATCCTTCTTCAGGACAGCCCT
GTCGTCCTTGCCTGTGTCCAGATGATCCCTCAAGCAATCAGTATTTTGCCCATTCCTGT
TATCAGAATCTGTGGAGCTCAGATGTAATCTGCAATTGTCTTCAAGGTTATACGGGTAC
TCAGTGTGGAGAATGCTCTACTGGTTTCTATGGAAATCCAAGAATTTCAGGAGCACCTT
GCCAACCATGTGCCTGCAACAACAACATAGATGTAACCGATCCAGAGTCCTGCAGCCG
GGTAACAGGGGAGTGCCTTCGATGTTTGCACAACACTCAGGGCGCAAACTGCCAGCTC
TGCAAACCAGGTCACTATGGATCAGCCCTCAATCAGACCTGCAGAAGATGCTCCTGCC
ATGCTTCCGGCGTGAGTCCCATGGAGTGTCCCCCTGGTGGGGGAGCTTGCCTCTGTG
ACCCTGTCACTGGTGCATGTCCTTGTCTGCCGAATGTCACAGGCCTGGCCTGTGACCG
 
TTGTGCTGATGGATACTGGAATCTGGTCCCTGGCAGAGGATGTCAGTCATGTGACTGT
GACCCTAGGACCTCTCAAAGTAGCCACTGTGACCAGCTTACAGGCCAGTGTCCGTGTA
AATTAGGTTACGGCGGGAAACGTTGCAGTGAGTGCCAGGAAAATTATTATGGTGATCCA
CCTGGGCGATGCATTCCATGTGATTGTAACAGGGCAGGTACCCAGAAGCCCATCTGTG
ATCCAGACACAGGCATGTGCCGCTGCCGGGAGGGTGTCAGCGGCCAGAGATGTGATC
GCTGTGCCCGGGGACACAGCCAGGAATTCCCTACTTGTCTTCAATGTCACTTGTGCTTT
GATCAGTGGGACCACACCATTTCTTCCCTCTCCAAAGCGGTGCAAGGGTTAATGAGACT
GGCTGCTAACATGGAAGATAAAAGAGAGACCCTGCCTGTCTGTGAGGCAGACTTCAAA
GACCTCAGAGGGAACGTGTCTGAAATAGAAAGGATTTTGAAACATCCTGTTTTCCCATC
TGGGAAATTCTTAAAAGTCAAGGATTATCATGACTCTGTTAGAAGACAAATCATGCAGCT
AAATGAACAACTGAAAGCAGTGTATGAATTTCAAGATCTGAAAGATACAATAGAAAGAGC
AAAGAATGAAGCAGACCTCTTACTTGAAGACCTTCAGGAAGAAATTGATTTGCAATCCA
GTGTCCTTAATGCAAGCATTGCGGACTCCTCAGAAAACATCAAGAAATATTATCACATAT
CATCATCTGCTGAAAAGAAAATTAATGAAACTAGTTCCACCATTAATACCTCTGCAAATA
CAAGGAATGACTTACTTACCATCTTAGATACACTAACCTCAAAAGGAAACTTGTCATTGG
AAAGATTAAAGCAGATTAAGATACCAGATATCCAAATATTGAATGAAAAGGTGTGCGGA
GATCCAGGAAATGTGCCATGTGTGCCCTTGCCCTGTGGCGGTGCTCTCTGCACGGGC
CGGAAGGGGCACAGGAAGTGTAGGGGTCCCGGCTGTCACGGCTCCCTGACCCTCTCA
ACGAATGCCCTCCAAAAAGCCCAGGAAGCAAAATCCATTATTCGTAATTTGGACAAACA
GGTTCGTGGGTTGAAAAATCAGATCGAAAGTATAAGTGAACAGGCAGAAGTCTCCAAAA
ACAATGCCTTACAGCTGAGGGAAAAACTGGGAAATATAAGAAACCAAAGTGACTCTGAA
GAAGAAAACATCAATCTTTTCATCAAAAAAGTGAAAAACTTTTTGTTAGAGGAAAACGTG
CCTCCAGAAGACATCGAGAAGGTTGCGAATGGTGTGCTTGACATTCACCTACCAATTCC
ATCCCAAAATCTAACCGATGAACTTGTCAAAATACAGAAACATATGCAACTCTGTGAGGA
TTACAGGACAGATGAAAACAGGTTAAATGAAGAAGCAGATGGAGCCCAAAAGCTTTTGG
TGAAGGCCAAAGCAGCTGAGAAAGCAGCAAATATTCTATTAAATCTTGACAAAACATTGA
ACCAGTTACAACAAGCTCAAATCACTCAAGGACGGGCAAACTCTACCATTACACAGCTG
ACTGCCAATATAACAAAAATAAAAAAGAATGTGCTGCAGGCTGAAAATCAAACCAGGGA
AATGAAGAGTGAGCTGGAGTTAGCAAAGCAGCGATCAGGGCTGGAGGATGGACTTTCC
CTGCTGCAGACCAAGTTGCAAAGGCATCAAGACCACGCTGTCAATGCGAAAGTTCAGG
CTGAATCTGCCCAACACCAGGCTGGGAGTCTTGAGAAGGAATTTGTTGAGCTGAAAAAA
CAATATGCTATTCTCCAACGTAAGACAAGCACTACAGGACTAACAAAGGAGACATTAGG
AAAAGTTAAACAGCTAAAAGATGCGGCAGAAAAATTGGCTGGAGATACAGAGGCCAAG
ATAAGAAGAATAACAGATTTAGAAAGGAAAATCCAAGATTTGAATCTAAGTAGACAAGCA
AAAGCTGATCAACTGAGAATATTGGAAGATCAAGTTGTTGCCATTAAAAATGAAATTGTT
 
GAACAAGAAAAAAATATGCTAGGTGCTATAGCCTCGAGCACCACCATCACCATCACCA
TCACTAAGTGATTAACCTCAGGTGCAGGCTGCCTATCAGAAGGTGGTGGCTGGTGTGG
CCAATGCCCTGGCTCACAAATACCACTGAGATCGATCTTTTTCCCTCTGCCAAAAATTAT
GGGGACATCATGAAGCCCCTTGAGCATCTGACTTCTGGCTAATAAAGGAAATTTATTTT
CATTGCAATAGTGTGTTGGAATTTTTTGTGTCTCTCACTCGGAAGGACATATGGGAGGG
CAAATCATTTAAAACATCAGAATGAGTATTTGGTTTAGAGTTTGGCAACATATGCCCATA
TGTAACTAGCATAACCCCTTGGGGCCTCTAAACGGGTCTTGAGGGGTTTTTTGCTGAAA
GCATGCGGAGGAAATTCTCCTTGAAGTTTCCCTGGTGTTCAAAGTAAAGGAGTTTGCAC
CAGACGCACCTCTGTTCACTGGTCCGGCGTATTAAAACACGATACATTGTTATTAGTAC
ATTTATTAAGCGCTAGATTCTGTGCGTTGTTGATTTACAGACAATTGTTGTACGTATTTTA
ATAATTCATTAAATTTATAATCTTTAGGGTGGTATGTTAGAGCGAAAATCAAATGATTTTC
AGCGTCTTTATATCTGAATTTAAATATTAAATCCTCAATAGATTTGTAAAATAGGTTTCGA
TTAGTTTCAAACAAGGGTTGTTTTTCCGAACCGATGGCTGGACTATCTAATGGATTTTCG
CTCAACGCCACAAAACTTGCCAAATCTTGTAGCAGCAATCTAGCTTTGTCGATATTCGTT
TGTGTTTTGTTTTGTAATAAAGGTTCGACGTCGTTCAAAATATTATGCGCTTTTGTATTTC
TTTCATCACTGTCGTTAGTGTACAATTGACTCGACGTAAACACGTTAAATAGAGCTTGGA
CATATTTAACATCGGGCGTGTTAGCTTTATTAGGCCGATTATCGTCGTCGTCCCAACCC
TCGTCGTTAGAAGTTGCTTCCGAAGACGATTTTGCCATAGCCACACGACGCCTATTAAT
TGTGTCGGCTAACACGTCCGCGATCAAATTTGTAGTTGAGCTTTTTGGAATTATTTCTGA
TTGCGGGCGTTTTTGGGCGGGTTTCAATCTAACTGTGCCCGATTTTAATTCAGACAACA
CGTTAGAAAGCGATGGTGCAGGCGGTGGTAACATTTCAGACGGCAAATCTACTAATGG
CGGCGGTGGTGGAGCTGATGATAAATCTACCATCGGTGGAGGCGCAGGCGGGGCTGG
CGGCGGAGGCGGAGGCGGAGGTGGTGGCGGTGATGCAGACGGCGGTTTAGGCTCAA
ATGTCTCTTTAGGCAACACAGTCGGCACCTCAACTATTGTACTGGTTTCGGGCGCCGTT
TTTGGTTTGACCGGTCTGAGACGAGTGCGATTTTTTTCGTTTCTAATAGCTTCCAACAAT
TGTTGTCTGTCGTCTAAAGGTGCAGCGGGTTGAGGTTCCGTCGGCATTGGTGGAGCG
GGCGGCAATTCAGACATCGATGGTGGTGGTGGTGGTGGAGGCGCTGGAATGTTAGGC
ACGGGAGAAGGTGGTGGCGGCGGTGCCGCCGGTATAATTTGTTCTGGTTTAGTTTGTT
CGCGCACGATTGTGGGCACCGGCGCAGGCGCCGCTGGCTGCACAACGGAAGGTCGT
CTGCTTCGAGGCAGCGCTTGGGGTGGTGGCAATTCAATATTATAATTGGAATACAAATC
GTAAAAATCTGCTATAAGCATTGTAATTTCGCTATCGTTTACCGTGCCGATATTTAACAA
CCGCTCAATGTAAGCAATTGTATTGTAAAGAGATTGTCTCAAGCTCGGAACGCTGCGCT
CGGTCGTTCGGCTGCGGCGAGCGGTATCAGCTCACTCAAAGGCGGTAATACGGTTATC
CACAGAATCAGGGGATAACGCAGGAAAGAACATGTGAGCAAAAGGCCAGCAAAAGGCC
AGGAACCGTAAAAAGGCCGCGTTGCTGGCGTTTTTCCATAGGCTCCGCCCCCCTGACG
 
AGCATCACAAAAATCGACGCTCAAGTCAGAGGTGGCGAAACCCGACAGGACTATAAAG
ATACCAGGCGTTTCCCCCTGGAAGCTCCCTCGTGCGCTCTCCTGTTCCGACCCTGCCG
CTTACCGGATACCTGTCCGCCTTTCTCCCTTCGGGAAGCGTGGCGCTTTCTCAATGCTC
ACGCTGTAGGTATCTCAGTTCGGTGTAGGTCGTTCGCTCCAAGCTGGGCTGTGTGCAC
GAACCCCCCGTTCAGCCCGACCGCTGCGCCTTATCCGGTAACTATCGTCTTGAGTCCA
ACCCGGTAAGACACGACTTATCGCCACTGGCAGCAGCCACTGGTAACAGGATTAGCAG
AGCGAGGTATGTAGGCGGTGCTACAGAGTTCTTGAAGTGGTGGCCTAACTACGGCTAC
ACTAGAAGGACAGTATTTGGTATCTGCGCTCTGCTGAAGCCAGTTACCTTCGGAAAAAG
AGTTGGTAGCTCTTGATCCGGCAAACAAACCACCGCTGGTAGCGGTGGTTTTTTTGTTT
GCAAGCAGCAGATTACGCGCAGAAAAAAAGGATCTCAAGAAGATCCTTTGTTACCAATG
CTTAATCAGTGAGGCACCTATCTCAGCGATCTGTCTATTTCGTTCATCCATAGTTGCCTG
ACTCCCCGTCGTGTAGATAACTACGATACGGGAGGGCTTACCATCTGGCCCCAGTGCT
GCAATGATACCGCGAGACCCACGCTCACCGGCTCCAGATTTATCAGCAATAAACCAGC
CAGCCGGAAGGGCCGAGCGCAGAAGTGGTCCTGCAACTTTATCCGCCTCCATCCAGTC
TATTAATTGTTGCCGGGAAGCTAGAGTAAGTAGTTCGCCAGTTAATAGTTTGCGCAACG
TTGTTGCCATTGCTACAGGCATCGTGGTGTCACGCTCGTCGTTTGGTATGGCTTCATTC
AGCTCCGGTTCCCAACGATCAAGGCGAGTTACATGATCCCCCATGTTGTGCAAAAAAGC
GGTTAGCTCCTTCGGTCCTCCGATCGTTGTCAGAAGTAAGTTGGCCGCAGTGTTATCAC
TCATGGTTATGGCAGCACTGCATAATTCTCTTACTGTCATGCCATCCGTAAGATGCTTTT
CTGTGACTGGTGAGTACTCAACCAAGTCATTCTGAGAATAGTGTATGCGGCGACCGAG
TTGCTCTTGCCCGGCGTCAATACGGGATAATACCGCGCCACATAGCAGAACTTTAAAAG
TGCTCATCATTGGAAAACGTTCTTCGGGGCGAAAACTCTCAAGGATCTTACCGCTGTTG
AGATCCAGTTCGATGTAACCCACTCGTGCACCCAACTGATCTTCAGCATCTTTTACTTTC
ACCAGCGTTTCTGGGTGAGCAAAAACAGGAAGGCAAAATGCCGCAAAAAAGGGAATAA
GGGCGACACGGAAATGTTGAATACTCATACTCTTCCTTTTTCAATATTATTGAAGCATTT
ATCAGGGTTATTGTCTCATGTCCGCGCGTTTCCTGCATCTTTTAATCAAATCCCAAGATG
TGTATAAACCACCAAACTGCCAAAAAATGAAAACTGTCGACAAGCTCTGTCCGTTTGCT
GGCAACTGCAAGGGTCTCAATCCTATTTGTAATTATTGAATAATAAAACAATTATAAATGT
CAAATTTGTTTTTTATTAACGATACAAACCAAACGCAACAAGAACATTTGTAGTATTATCT
ATAATTGAAAACGCGTAGTTATAATCGCTGAGGTAATATTTAAAATCATTTTCAAATGATT
CACAGTTAATTTGCGACAATATAATTTTATTTTCACATAAACTAGACGCCTTGTCGTCTTC
TTCTTCGTATTCCTTCTCTTTTTCATTTTTCTCTTCATAAAAATTAACATAGTTATTATCGT
ATCCATATATGTATCTATCGTATAGAGTAAATTTTTTGTTGTCATAAATATATATGTCTTTT
TTAATGGGGTGTATAGTACCGCTGCGCATAGTTTTTCTGTAATTTACAACAGTGCTATTT
TCTGGTAGTTCTTCGGAGTGTGTTGCTTTAATTATTAAATTTATATAATCAATGAATTTGG
 
GATCGTCGGTTTTGTACAATATGTTGCCGGCATAGTACGCAGCTTCTTCTAGTTCAATTA
CACCATTTTTTAGCAGCACCGGATTAACATAACTTTCCAAAATGTTGTACGAACCGTTAA
ACAAAAACAGTTCACCTCCCTTTTCTATACTATTGTCTGCGAGCAGTTGTTTGTTGTTAA
AAATAACAGCCATTGTAATGAGACGCACAAACTAATATCACAAACTGGAAATGTCTATCA
ATATATAGTTGCTCTAGTTATTAATAGTAATCAATTACGGGGTCATTAGTTCATAGCCCA
TATATGGAGTTCCGCGTTACATAACTTACGGTAAATGGCCCGCCTGGCTGACCGCCCA
ACGACCCCCGCCCATTGACGTCAATAATGACGTATGTTCCCATAGTAACGCCAATAGGG
ACTTTCCATTGACGTCAATGGGTGGACTATTTACGGTAAACTGCCCACTTGGCAGTACA
TCAAGTGTATCATATGCCAAGTACGCCCCCTATTGACGTCAATGACGGTAAATGGCCCG
CCTGGCATTATGCCCAGTACATGACCTTATGGGACTTTCCTACTTGGCAGTACATCTAC
GTATTAGTCATCGCTATTACCATGCATGGTCGAGGTGAGCCCCACGTTCTGCTTCACTC
TCCCCATCTCCCCCCCCTCCCCACCCCCAATTTTGTATTTATTTATTTTTTAATTATTTTG
TGCAGCGATGGGGGCGGGGGGGGGGGGGGCGCGCGCCAGGCGGGGCGGGGCG
GGGCGAGGGGCGGGGCGGGGCGAGGCGGAGAGGTGCGGCGGCAGCCAATCAGAGC
GGCGCGCTCCGAAAGTTTCCTTTTATGGCGAGGCGGCGGCGGCGGCGGCCCTATAAA
AAGCGAAGCGCGCGGCGGGCG
**SEQ ID NO 6_pTriEx-1-LAMB4(IF1)[human](dHis) - kodiert für - Humanes Laminin beta-4 mit His-Tag** 
GGAGTCGCTGCGACGCTGCCTTCGCCCCGTGCCCCGCTCCGCCGCCGCCTCGCGCC
GCCCGCCCCGGCTCTGACTGACCGCGTTACTCCCACAGGTGAGCGGGCGGGACGGC
CCTTCTCCTTCGGGCTGTAATTAGCGCTTGGTTTAATGACGGCTTGTTTCTTTTCTGTG
GCTGCGTGAAAGCCTTGAGGGGCTCCGGGAGGGCCCTTTGTGCGGGGGGAGCGGCT
CGGGGCTGTCCGCGGGGGGACGGCTGCCTTCGGGGGGGACGGGGCAGGGCGGGGT
TCGGCTTCTGGCGTGTGACCGGCGGCTCTAGAGCCTCTGCTAACCATGTTCATGCCTT
CTTCTTTTTCCTACAGCTCCTGGGCAACGTGCTGGTTATTGTGCTGTCTCATCATTTTGG
CAAAGAATTGGATCGGACCGAAATTAATACGACTCACTATAGGGGAATTGTGAGCGGAT
AACAATTCCCCGGAGTTAATCCGGGACCTTTAATTCAACCCAACACAATATATTATAGTT
AAATAAGAATTATTATCAAATCATTTGTATATTAATTAAAATACTATACTGTAAATTACATT
TTATTTACAATCAAAGGAGATATACCATGCAATTTCAACTGACCCTTTTTTGCACCTTG
GGTGGCTCAGTTACTCAAAAGCTCAAGATGACTGCAACAGGGGTGCCTGTCATCCCAC
CACTGGTGATCTCCTGGTGGGCAGGAACACGCAGCTTATGGCTTCTTCTACCTGTGGG
CTGAGCAGAGCCCAGAAATACTGCATCCTCAGTTACCTGGAGGGGGAACAAAAATGCT
TCATCTGTGACTCTAGATTTCCATATGATCCGTATGACCAACCCAACAGCCACACCATTG
AGAATGTCATTGTAAGTTTTGAACCAGACAGAGAAAAGAAATGGTGGCAATCTGAAAAT
 
GGTCTTGATCATGTCAGCATCAGACTGGACTTAGAGGCATTATTTCGGTTCAGCCACCT
TATCCTGACCTTTAAGACTTTTCGGCCTGCTGCAATGTTAGTTGAACGTTCCACAGACTA
TGGACACAACTGGAAAGTGTTCAAATATTTTGCAAAAGACTGTGCCACTTCCTTTCCTAA
CATCACATCTGGCCAGGCCCAGGGAGTGGGAGACATTGTTTGTGACTCCAAATACTCG
GATATTGAACCCTCAACAGGTGGAGAGGTTGTTTTAAAAGTTTTGGATCCCAGTTTTGA
AATTGAAAACCCTTATAGCCCCTACATCCAAGACCTTGTGACATTGACAAACCTGAGGA
TAAACTTTACCAAGCTCCACACCCTTGGGGATGCTTTGCTTGGAAGGAGGCAAAATGAT
TCCCTTGATAAATACTACTATGCTCTGTACGAGATGATTGTTCGGGGAAGCTGCTTTTG
CAATGGCCATGCTAGCGAATGTCGCCCTATGCAGAAGATGCGGGGAGATGTTTTCAGC
CCTCCTGGAATGGTTCACGGTCAGTGTGTGTCAGCACAATACAGATGGTCCGAACT
GTGAGAGATGCAAGGACTTCTTCCAGGATGCTCCTTGGAGGCCAGCTGCAGACCTCCA
GGACAACGCTTGCAGATCGTGCAGCTGTAACAGCCACTCCAGCCGCTGTCACTTTGAC
ATGACTACGTACCTGGCAAGCGGTGGCCTCAGCGGGGGCGTGTGTGAAGACTGCCAG
CACAACACTGAGGGGCAGCACTGCGACCGCTGCAGACCCCTCTTCTACAGGGACCCG
CTCAAGACCATCTCAGATCCCTACGCGTGCATTCCTTGTGAATGTGACCCCGATGGGA
CCATATCTGGTGGCATTTGTGTGAGCCACTCTGATCCTGCCTTAGGGTCTGTGGCTGG
CCAGTGCCTTTGTAAAGAGAACGTGGAAGGAGCCAAATGCGACCAGTGCAAACCCAAC
CACTATGGACTAAGCGCCACCGACCCCCTGGGCTGCCAGCCCTGCGACTGTAACCCCC
TTGGGAGTCTGCCATTCTTGACCTGTGATGTGGATACAGGCCAATGCTTGTGCCTGTCA
TATGTCACCGGAGCACACTGCGAAGAATGCACTGTTGGATACTGGGGCCTGGAAATC
ATCTCCATGGGTGTTCTCCCTGTGACTGTGATATTGGAGGTGCTTATTCTAACGTGTGC
TCACCCAAGAATGGGCAGTGTGAATGCCGCCCACATGTCACTGGCCGTAGCTGCTCTG
AACCAGCCCCTGGCTACTTCTTTGCTCCTTTGAATTTCTATCTCTACGAGGCAGAGGAA
GCCACAACACTCCAAGGACTGGCGCCTTTGGGCTCGGAGACGTTTGGCCAGAGTCCT
GCTGTTCACGTTGTTTAGGAGAGCCAGTTCCTGGGAACCCTGTTACATGGACTGGAC
CTGGATTTGCCAGGGTTCTCCCTGGGGCTGGCTTGAGATTTGCTGTCAACAACATTCC
CTTTCCTGTGGACTTCACCATTGCCATTCACTATGAAACCCAGTCTGCAGCTGACTGGA
CTGTCCAGATTGTGGTGAACCCCCCTGGAGGGAGTGAGCACTGCATACCCAAGACTCT
ACAGTCAAAGCCTCAGTCTTTTGCCTTACCAGCGGCTACGAGAATCATGCTGCTTCCCA
CACCCATCTGTTTAGAACCAGATGTACAATATTCCATAGATGTCTATTTTTCTCAGCCTT
TGCAAGGAGAGTCCCACGCTCATTCACATGTCCTGGTGGACTCTCTTGGCCTTATTCCC
CAAATCAATTCATTGGAGAATTTCTGCAGCAAGCAGGACTTAGATGAGTATCAGCTTCA
CAACTGTGTTGAAATTGCCTCAGCAATGGGACCTCAAGTGCTCCCGGGTGCCTGTGAA
AGGCTGATCATCAGCATGTCTGCCAAGCTGCATGATGGGGCTGTGGCCTGCAAGTGTC
ACCCCCAGGGCTCAGTCGGATCCAGCTGCAGCCGACTTGGAGGCCAGTGCCAGTGTA
 
AACCTCTTGTGGTCGGGCGCTGCTGTGACAGGTGCTCAACTGGAAGCTATGATTTGGG
GCATCACGGCTGTCACCCATGTCACTGCCATCCTCAAGGATCAAAGGACACTGTATGTG
ACCAAGTAACAGGACAGTGCCCCTGCCATGGAGAGGTGTCTGGCCGCCGCTGTGATC
GCTGCCTGGCAGGCTACTTTGGATTTCCCAGCTGCCACCCTTGCCCTTGTAATAGGTTT
GCTGAACTTTGTGATCCTGAGACAGGGTCATGCTTCAATTGTGGAGGCTTTACAACTGG
CAGAAACTGTGAAAGGTGTATTGATGGTTACTATGGAAATCCTTCTTCAGGACAGCCCT
GTCGTCCTTGCCTGTGTCCAGATGATCCCTCAAGCAATCAGTATTTTGCCCATTCCTGT
TATCAGAATCTGTGGAGCTCAGATGTAATCTGCAATTGTCTTCAAGGTTATACGGGTAC
TCAGTGTGGAGAATGCTCTACTGGTTTCTATGGAAATCCAAGAATTTCAGGAGCACCTT
GCCAACCATGTGCCTGCAACAACAACATAGATGTAACCGATCCAGAGTCCTGCAGCCG
GGTAACAGGGGAGTGCCTTCGATGTTTGCACAACACTCAGGGCGCAAACTGCCAGCTC
TGCAAACCAGGTCACTATGGATCAGCCCTCAATCAGACCTGCAGAAGATGCTCCTGCC
ATGCTTCCGGCGTGAGTCCCATGGAGTGTCCCCCTGGTGGGGAGCTTGCCTCTGTG
ACCCTGTCACTGGTGCATGTCCTTGTCTGCCGAATGTCACAGGCCTGGCCTGTGACCG
TTGTGCTGATGGATACTGGAATCTGGTCCCTGGCAGAGGATGTCAGTCATGTGACTGT
GACCCTAGGACCTCTCAAAGTAGCCACTGTGACCAGCTTACAGGCCAGTGTCCGTGTA
AATTAGGTTACGGCGGGAAACGTTGCAGTGAGTGCCAGGAAAATTATTATGGTGATCCA
CCTGGGCGATGCATTCCATGTGATTGTAACAGGGCAGGTACCCAGAAGCCCATCTGTG
ATCCAGACACAGGCATGTGCCGCTGCCGGGAGGGTGTCAGCGGCCAGAGATGTGATC
GCTGTGCCCGGGGACACAGCCAGGAATTCCCTACTTGTCTTCAATGTCACTTGTGCTTT
GATCAGTGGGACCACACCATTTCTTCCCTCTCCAAAGCGGTGCAAGGGTTAATGAGACT
GGCTGCTAACATGGAAGATAAAGAGAGACCCTGCCTGTCTGTGAGGCAGACTTCAAA
GACCTCAGAGGGAACGTGTCTGAAATAGAAAGGATTTTGAAACATCCTGTTTTCCCATC
TGGGAAATTCTTAAAAGTCAAGGATTATCATGACTCTGTTAGAAGACAAATCATGCAGCT
AAATGAACAACTGAAAGCAGTGTATGAATTTCAAGATCTGAAAGATACAATAGAAAGAGC
AAAGAATGAAGCAGACCTCTTACTTGAAGACCTTCAGGAAGAAATTGATTTGCAATCCA
GTGTCCTTAATGCAAGCATTGCGGACTCCTCAGAAAACATCAAGAAATATTATCACATAT
CATCATCTGCTGAAAAGAAAATTAATGAAACTAGTTCCACCATTAATACCTCTGCAAATA
CAAGGAATGACTTACTTACCATCTTAGATACACTAACCTCAAAAGGAAACTTGTCATTGG
AAAGATTAAAGCAGATTAAGATACCAGATATCCAAATATTGAATGAAAAGGTGTGCGGA
GATCCAGGAAATGTGCCATGTGTGCCCTTGCCCTGTGGCGGTGCTCTCTGCACGGGC
CGGAAGGGGCACAGGAAGTGTAGGGGTCCCGGCTGTCACGGCTCCCTGACCCTCTCA
ACGAATGCCCTCCAAAAAGCCCAGGAAGCAAAATCCATTATTCGTAATTTGGACAAACA
GGTTCGTGGGTTGAAAAATCAGATCGAAAGTATAAGTGAACAGGCAGAAGTCTCCAAAA
ACAATGCCTTACAGCTGAGGGAAAAACTGGGAAATATAAGAAACCAAAGTGACTCTGAA
 
GAAGAAAACATCAATCTTTTCATCAAAAAAGTGAAAAACTTTTTGTTAGAGGAAAACGTG
CCTCCAGAAGACATCGAGAAGGTTGCGAATGGTGTGCTTGACATTCACCTACCAATTCC
ATCCCAAAATCTAACCGATGAACTTGTCAAAATACAGAAACATATGCAACTCTGTGAGGA
TTACAGGACAGATGAAAACAGGTTAAATGAAGAAGCAGATGGAGCCCAAAAGCTTTTGG
TGAAGGCCAAAGCAGCTGAGAAAGCAGCAAATATTCTATTAAATCTTGACAAAACATTGA
ACCAGTTACAACAAGCTCAAATCACTCAAGGACGGGCAAACTCTACCATTACACAGCTG
ACTGCCAATATAACAAAAATAAAAAAGAATGTGCTGCAGGCTGAAAATCAAACCAGGGA
AATGAAGAGTGAGCTGGAGTTAGCAAAGCAGCGATCAGGGCTGGAGGATGGACTTTCC
CTGCTGCAGACCAAGTTGCAAAGGCATCAAGACCACGCTGTCAATGCGAAAGTTCAGG
CTGAATCTGCCCAACACCAGGCTGGGAGTCTTGAGAAGGAATTTGTTGAGCTGAAAAAA
CAATATGCTATTCTCCAACGTAAGACAAGCACTACAGGACTAACAAAGGAGACATTAGG
AAAAGTTAAACAGCTAAAAGATGCGGCAGAAAAATTGGCTGGAGATACAGAGGCCAAG
ATAAGAAGAATAACAGATTTAGAAAGGAAAATCCAAGATTTGAATCTAAGTAGACAAGCA
AAAGCTGATCAACTGAGAATATTGGAAGATCAAGTTGTTGCCATTAAAAATGAAATTGTT
GAACAAGAAAAAAAATATGCTAGGTGCTATAGCTAGCTCGAGCACCACCATCACCATCA
CCATCACTAAGTGATTAACCTCAGGTGCAGGCTGCCTATCAGAAGGTGGTGGCTGGTG
TGGCCAATGCCCTGGCTCACAAATACCACTGAGATCGATCTTTTTCCCTCTGCCAAAAA
TTATGGGGACATCATGAAGCCCCTTGAGCATCTGACTTCTGGCTAATAAAGGAAATTTA
TTTTCATTGCAATAGTGTGTTGGAATTTTTTGTGTCTCTCACTCGGAAGGACATATGGGA
GGGCAAATCATTTAAAACATCAGAATGAGTATTTGGTTTAGAGTTTGGCAACATATGCCC
ATATGTAACTAGCATAACCCCTTGGGGCCTCTAAACGGGTCTTGAGGGGTTTTTTGCTG
AAAGCATGCGGAGGAAATTCTCCTTGAAGTTTCCCTGGTGTTCAAAGTAAAGGAGTTTG
CACCAGACGCACCTCTGTTCACTGGTCCGGCGTATTAAAACACGATACATTGTTATTAG
TACATTTATTAAGCGCTAGATTCTGTGCGTTGTTGATTTACAGACAATTGTTGTACGTAT
TTTAATAATTCATTAAATTTATAATCTTTAGGGTGGTATGTTAGAGCGAAAATCAAATGAT
TTTCAGCGTCTTTATATCTGAATTTAAATATTAAATCCTCAATAGATTTGTAAAATAGGTT
TCGATTAGTTTCAAACAAGGGTTGTTTTTCCGAACCGATGGCTGGACTATCTAATGGATT
TTCGCTCAACGCCACAAAACTTGCCAAATCTTGTAGCAGCAATCTAGCTTTGTCGATATT
CGTTTGTGTTTTGTTTTGTAATAAAGGTTCGACGTCGTTCAAAATATTATGCGCTTTTGT
ATTTCTTTCATCACTGTCGTTAGTGTACAATTGACTCGACGTAAACACGTTAAATAGAGC
TTGGACATATTTAACATCGGGCGTGTTAGCTTTATTAGGCCGATTATCGTCGTCGTCCC
AACCCTCGTCGTTAGAAGTTGCTTCCGAAGACGATTTTGCCATAGCCACACGACGCCTA
TTAATTGTGTCGGCTAACACGTCCGCGATCAAATTTGTAGTTGAGCTTTTTGGAATTATT
TCTGATTGCGGGCGTTTTTGGGCGGGTTTCAATCTAACTGTGCCCGATTTTAATTCAGA
CAACACGTTAGAAAGCGATGGTGCAGGCGGTGGTAACATTTCAGACGGCAAATCTACT
 
AATGGCGGCGGTGGTGGAGCTGATGATAAATCTACCATCGGTGGAGGCGCAGGCGGG
GCTGGCGGCGGAGGCGGAGGCGGAGGTGGTGGCGGTGATGCAGACGGCGGTTTAG
GCTCAAATGTCTCTTTAGGCAACACAGTCGGCACCTCAACTATTGTACTGGTTTCGGGC
GCCGTTTTTGGTTTGACCGGTCTGAGACGAGTGCGATTTTTTTCGTTTCTAATAGCTTC
CAACAATTGTTGTCTGTCGTCTAAAGGTGCAGCGGGTTGAGGTTCCGTCGGCATTGGT
GGAGCGGGCGGCAATTCAGACATCGATGGTGGTGGTGGTGGAGGCGCTGGAAT
GTTAGGCACGGGAGAAGGTGGTGGCGGCGGTGCCGCCGGTATAATTTGTTCTGGTTT
AGTTTGTTCGCGCACGATTGTGGGCACCGGCGCAGGCGCCGCTGGCTGCACAACGGA
AGGTCGTCTGCTTCGAGGCAGCGCTTGGGGTGGTGGCAATTCAATATTATAATTGGAA
TACAAATCGTAAAAATCTGCTATAAGCATTGTAATTTCGCTATCGTTTACCGTGCCGATA
TTTAACAACCGCTCAATGTAAGCAATTGTATTGTAAAGAGATTGTCTCAAGCTCGGAACG
CTGCGCTCGGTCGTTCGGCTGCGGCGAGCGGTATCAGCTCACTCAAAGGCGGTAATA
CGGTTATCCACAGAATCAGGGGATAACGCAGGAAAGAACATGTGAGCAAAAGGCCAGC
AAAAGGCCAGGAACCGTAAAAAGGCCGCGTTGCTGGCGTTTTTCCATAGGCTCCGCCC
CCCTGACGAGCATCACAAAAATCGACGCTCAAGTCAGAGGTGGCGAAACCCGACAGGA
CTATAAAGATACCAGGCGTTTCCCCCTGGAAGCTCCCTCGTGCGCTCTCCTGTTCCGA
CCCTGCCGCTTACCGGATACCTGTCCGCCTTTCTCCCTTCGGGAAGCGTGGCGCTTTC
TCAATGCTCACGCTGTAGGTATCTCAGTTCGGTGTAGGTCGTTCGCTCCAAGCTGGGC
TGTGTGCACGAACCCCCCGTTCAGCCCGACCGCTGCGCCTTATCCGGTAACTATCGTC
TTGAGTCCAACCCGGTAAGACACGACTTATCGCCACTGGCAGCAGCCACTGGTAACAG
GATTAGCAGAGCGAGGTATGTAGGCGGTGCTACAGAGTTCTTGAAGTGGTGGCCTAAC
TACGGCTACACTAGAAGGACAGTATTTGGTATCTGCGCTCTGCTGAAGCCAGTTACCTT
CGGAAAAAGAGTTGGTAGCTCTTGATCCGGCAAACAAACCACCGCTGGTAGCGGTGGT
TTTTTTGTTTGCAAGCAGCAGATTACGCGCAGAAAAAAAGGATCTCAAGAAGATCCTTT
GTTACCAATGCTTAATCAGTGAGGCACCTATCTCAGCGATCTGTCTATTTCGTTCATCCA
TAGTTGCCTGACTCCCCGTCGTGTAGATAACTACGATACGGGAGGGCTTACCATCTGG
CCCCAGTGCTGCAATGATACCGCGAGACCCACGCTCACCGGCTCCAGATTTATCAGCA
ATAAACCAGCCAGCCGGAAGGGCCGAGCGCAGAAGTGGTCCTGCAACTTTATCCGCCT
CCATCCAGTCTATTAATTGTTGCCGGGAAGCTAGAGTAAGTAGTTCGCCAGTTAATAGT
TTGCGCAACGTTGTTGCCATTGCTACAGGCATCGTGGTGTCACGCTCGTCGTTTGGTAT
GGCTTCATTCAGCTCCGGTTCCCAACGATCAAGGCGAGTTACATGATCCCCCATGTTGT
GCAAAAAAGCGGTTAGCTCCTTCGGTCCTCCGATCGTTGTCAGAAGTAAGTTGGCCGC
AGTGTTATCACTCATGGTTATGGCAGCACTGCATAATTCTCTTACTGTCATGCCATCCGT
AAGATGCTTTTCTGTGACTGGTGAGTACTCAACCAAGTCATTCTGAGAATAGTGTATGC
GGCGACCGAGTTGCTCTTGCCCGGCGTCAATACGGGATAATACCGCGCCACATAGCAG
 
AACTTTAAAAGTGCTCATCATTGGAAAACGTTCTTCGGGGCGAAAACTCTCAAGGATCT
TACCGCTGTTGAGATCCAGTTCGATGTAACCCACTCGTGCACCCAACTGATCTTCAGCA
TCTTTTACTTTCACCAGCGTTTCTGGGTGAGCAAAAACAGGAAGGCAAAATGCCGCAAA
AAAGGGAATAAGGGCGACACGGAAATGTTGAATACTCATACTCTTCCTTTTTCAATATTA
TTGAAGCATTTATCAGGGTTATTGTCTCATGTCCGCGTTTCCTGCATCTTTTAATCAA
ATCCCAAGATGTGTATAAACCACCAAACTGCCAAAAAATGAAAACTGTCGACAAGCTCT
GTCCGTTTGCTGGCAACTGCAAGGGTCTCAATCCTATTTGTAATTATTGAATAATAAAAC
AATTATAAATGTCAAATTTGTTTTTTATTAACGATACAAACCAAACGCAACAAGAACATTT
GTAGTATTATCTATAATTGAAAACGCGTAGTTATAATCGCTGAGGTAATATTTAAAATCAT
TTTCAAATGATTCACAGTTAATTTGCGACAATATAATTTTATTTTCACATAAACTAGACGC
CTTGTCGTCTTCTTCTTCGTATTCCTTCTCTTTTTCATTTTTCTCTTCATAAAAATTAACAT
AGTTATTATCGTATCCATATATGTATCTATCGTATAGAGTAAATTTTTTGTTGTCATAAAT
ATATATGTCTTTTTTAATGGGGTGTATAGTACCGCTGCGCATAGTTTTTCTGTAATTTAC
AACAGTGCTATTTTCTGGTAGTTCTTCGGAGTGTGTTGCTTTAATTATTAAATTTATATAA
TCAATGAATTTGGGATCGTCGGTTTTGTACAATATGTTGCCGGCATAGTACGCAGCTTC
TTCTAGTTCAATTACACCATTTTTTAGCAGCACCGGATTAACATAACTTTCCAAAATGTTG
TACGAACCGTTAAACAAAAACAGTTCACCTCCCTTTTCTATACTATTGTCTGCGAGCAGT
TGTTTGTTGTTAAAAATAACAGCCATTGTAATGAGACGCACAAACTAATATCACAAACTG
GAAATGTCTATCAATATATAGTTGCTCTAGTTATTAATAGTAATCAATTACGGGGTCATTA
GTTCATAGCCCATATATGGAGTTCCGCGTTACATAACTTACGGTAAATGGCCCGCCTGG
CTGACCGCCCAACGACCCCCGCCCATTGACGTCAATAATGACGTATGTTCCCATAGTAA
CGCCAATAGGGACTTTCCATTGACGTCAATGGGTGGACTATTTACGGTAAACTGCCCAC
TTGGCAGTACATCAAGTGTATCATATGCCAAGTACGCCCCCTATTGACGTCAATGACGG
TAAATGGCCCGCCTGGCATTATGCCCAGTACATGACCTTATGGGACTTTCCTACTTGGC
AGTACATCTACGTATTAGTCATCGCTATTACCATGCATGGTCGAGGTGAGCCCCACGTT
CTGCTTCACTCTCCCCATCTCCCCCCCCTCCCCACCCCCAATTTTGTATTTATTTATTTT
TTAATTATTTTGTGCAGCGATGGGGGCGGGGGGGGGGGGGGCGCGCGCCAGGCG
GGGCGGGGCGGGGCGAGGGGCGGGGCGGGGCGAGGCGGAGAGGTGCGGCGGCAG
CCAATCAGAGCGGCGCGCTCCGAAAGTTTCCTTTTATGGCGAGGCGGCGGCGGCGGC
GGCCCTATAAAAAGCGAAGCGCGCGGCGGGCG
**SEQ ID NO7:>LAMB4-His - Humanes Laminin beta-4 mit His-Tag** 
MQFQLTLFLHLGWLSYSKAQDDCNRGACHPTTGDLLVGRNTQLMASSTCGLSRAQKYCIL
SYLEGEQKCFICDSRFPYDPYDQPNSHTIENVIVSFEPDREKKWWQSENGLDHVSIRLDLEA
LFRFSHLILTFKTFRPAAMLVERSTDYGHNWKVFKYFAKDCATSFPNITSGQAQGVGDIVCD
 
SKYSDIEPSTGGEVVLKVLDPSFEIENPYSPYIQDLVTLTNLRINFTKLHTLGDALLGRRQNDS
LDKYYYALYEMIVRGSCFCNGHASECRPMQKMRGDVFSPPGMVHGQCVCQHNTDGPNCE
RCKDFFQDAPWRPAADLQDNACRSCSCNSHSSRCHFDMTTYLASGGLSGGVCEDCQHNT
EGQHCDRCRPLFYRDPLKTISDPYACIPCECDPDGTISGGICVSHSDPALGSVAGQCLCKEN
VEGAKCDQCKPNHYGLSATDPLGCQPCDCNPLGSLPFLTCDVDTGQCLCLSYVTGAHCEE
CTVGYWGLGNHLHGCSPCDCDIGGAYSNVCSPKNGQCECRPHVTGRSCSEPAPGYFFAP
LNFYLYEAEEATTLQGLAPLGSETFGQSPAVHVVLGEPVPGNPVTWTGPGFARVLPGAGL
RFAVNNIPFPVDFTIAIHYETQSAADWTVQIVVNPPGGSEHCIPKTLQSKPQSFALPAATRIM
LLPTPICLEPDVQYSIDVYFSQPLQGESHAHSHVLVDSLGLIPQINSLENFCSKQDLDEYQLH
NCVEIASAMGPQVLPGACERLIISMSAKLHDGAVACKCHPQGSVGSSCSRLGGQCQCKPLV
VGRCCDRCSTGSYDLGHHGCHPCHCHPQGSKDTVCDQVTGQCPCHGEVSGRRCDRCLA
GYFGFPSCHPCPCNRFAELCDPETGSCFNCGGFTTGRNCERCIDGYYGNPSSGQPCRPC
LCPDDPSSNQYFAHSCYQNLWSSDVICNCLQGYTGTQCGECSTGFYGNPRISGAPCQPCA
CNNNIDVTDPESCSRVTGECLRCLHNTQGANCQLCKPGHYGSALNQTCRRCSCHASGVSP
MECPPGGGACLCDPVTGACPCLPNVTGLACDRCADGYWNLVPGRGCQSCDCDPRTSQS
SHCDQLTGQCPCKLGYGGKRCSECQENYYGDPPGRCIPCDCNRAGTQKPICDPDTGMCR
CREGVSGQRCDRCARGHSQEFPTCLQCHLCFDQWDHTISSLSKAVQGLMRLAANMEDKR
ETLPVCEADFKDLRGNVSEIERILKHPVFPSGKFLKVKDYHDSVRRQIMQLNEQLKAVYEFQ
DLKDTIERAKNEADLLLEDLQEEIDLQSSVLNASIADSSENIKKYYHISSSAEKKINETSSTINT
SANTRNDLLTILDTLTSKGNLSLERLKQIKIPDIQILNEKVCGDPGNVPCVPLPCGGALCTGRK
GHRKCRGPGCHGSLTLSTNALQKAQEAKSIIRNLDKQVRGLKNQIESISEQAEVSKNNALQL
REKLGNIRNQSDSEEENINLFIKKVKNFLLEENVPPEDIEKVANGVLDIHLPIPSQNLTDELVKI
QKHMQLCEDYRTDENRLNEEADGAQKLLVKAKAAEKAANILLNLDKTLNQLQQAQITQGRA
NSTITQLTANITKIKKNVLQAENQTREMKSELELAKQRSGLEDGLSLLQTKLQRHQDHAVNA
KVQAESAQHQAGSLEKEFVELKKQYAILQRKTSTTGLTKETLGKVKQLKDAAEKLAGDTEAK
IRRITDLERKIQDLNLSRQAKADQLRILEDQVVAIKNEIVEQEKKYARCYSLEHHHHHHHH
**SEQ ID NO 8: Autoantikörper-Epitop 1**
   EFQDL
**SEQ ID NO 9: Autoantikörper-Epitop 2**
   ADLLLEDLQE
**SEQ ID NO 10: Autoantikörper-Epitop 3**
   ADLLLEDLQEEIDLQS
**SEQ ID NO 11: Autoantikörper-Epitop 4**
   DLLTILDTLTSK
**SEQ ID NO 12: Autoantikörper-Epitop 5**
   QIKIPDIQILNEK
**SEQ ID NO 13: Autoantikörper-Epitop 6**
   VRGLKNQIESISE
**SEQ ID NO 14: Autoantikörper-Epitop 7**
   LLEENVPPEDI

SEQ ID NO 15: Teilfragment 1
MQDDCNRGACHPTTGDLLVGRNTQLMASSTCGLSRAQKYCILSYLEGEQKCFICDSRFPY
DPYDQPNSHTIENVIVSFEPDREKKWWQSENGLDHVSIRLDLEALFRFSHLILTFKTFRPAAM
LVERSTDYGHNWKVFKYFAKDCATSFPNITSGQAQGVGDIVCDSKYSDIEPSTGGEVVLKV
LDPSFEIENPYSPYIQDLVTLTNLRINFTKLHTLGDALLGRRQNDSLDKYYYALYEMIVRGSCF
CNGHASECRPMQKMRGDVFSPPGMVHGQCVCQHNTDGPNCERCKDFFQDAPW

SEQ ID NO 16: Teilfragment 2
MPNCERCKDFFQDAPWRPAADLQDNACRSCSCNSHSSRCHFDMTTYLASGGLSGGVCED
CQHNTEGQHCDRCRPLFYRDPLKTISDPYACIPCECDPDGTISGGICVSHSDPALGSVAGQ
CLCKENVEGAKCDQCKPNHYGLSATDPLGCQPCDCNPLGSLPFLTCDVDTGQCLCLSYVT
GAHCEECTVGYWGLGNHLHGCSPCDCDIGGAYSNVCSPKNGQCECRPHVTGRSCSEPAP
GYFFAPLNFYLYEAEEATTLQGLAPLGSETFGQSPAVHVVLGEPVPGNPVTWTGPGFARVL
W

SEQ ID NO 17: Teilfragment 3
MGNPVTWTGPGFARVLPGAGLRFAVNNIPFPVDFTIAIHYETQSAADWTVQIVVNPPGGSE
HCIPKTLQSKPQSFALPAATRIMLLPTPICLEPDVQYSIDVYFSQPLQGESHAHSHVLVDSLG
LIPQINSLENFCSKQDLDEYQLHNCVEIASAMGPQVLPGACERLIISMSAKLHDGAVACKCHP
QGSVGSSCSRLGGQCQCKPLVVGRCCDRCSTGSYDLGHHGCHPCHCHPQGSKDTVCDQ
VTGQCPCHGEVSGRRCDRCLAGYFGFPSCHPCPCNRFAELCDPETGSCFNCGGFTTGRN
CERCIDGYYGNWGW

SEQ ID NO 18: Teilfragment 4
MTGRNCERCIDGYYGNPSSGQPCRPCLCPDDPSSNQYFAHSCYQNLWSSDVICNCLQGY
TGTQCGECSTGFYGNPRISGAPCQPCACNNNIDVTDPESCSRVTGECLRCLHNTQGANCQ
LCKPGHYGSALNQTCRRCSCHASGVSPMECPPGGGACLCDPVTGACPCLPNVTGLACDR
CADGYWNLVPGRGCQSCDCDPRTSQSSHCDQLTGQCPCKLGYGGKRCSECQENYYGDP
PGRCIPCDCNRAGTQKPICDPDTGMCRCREGVSGQRCDRCARGHSQEWGW

SEQ ID NO 19: Teilfragment 5
MSGQRCDRCARGHSQEFPTCLQCHLCFDQWDHTISSLSKAVQGLMRLAANMEDKRETLP
VCEADFKDLRGNVSEIERILKHPVFPSGKFLKVKDYHDSVRRQIMQLNEQLKAVYEFQDLKD
TIERAKNEADLLLEDLQEEIDLQSSVLNASIADSSENIKKYYHISSSAEKKINETSSTINTSANT
RNDLLTILDTLTSKGNLSLERLKQIKIPDIQILNEKVCGDPGNVPCVPLPCGGALCTGRKGHR
KCRGPGCHGSLTLSTNALQKAQEAKSIIRNLDKQVRGLKNQIESISEQAEVSKNNALQLREK
LGNIWGWGW

SEQ ID NO 20: Teilfragment 6
MSKNNALQLREKLGNIRNQSDSEEENINLFIKKVKNFLLEENVPPEDIEKVANGVLDIHLPIPS
QNLTDELVKIQKHMQLCEDYRTDENRLNEEADGAQKLLVKAKAAEKAANILLNLDKTLNQLQ
QAQITQGRANSTITQLTANITKIKKNVLQAENQTREMKSELELAKQRSGLEDGLSLLQTKLQR
HQDHAVNAKVQAESAQHQAGSLEKEFVELKKQYAILQRKTSTTGLTKETLGKVKQLKDAAE
KLAGDTEAKIRRITDLERKIQDLNLSRQAKADQLRILEDQVVAIKNEIVEQEKKYARCYSWGW
GWGW

**SEQ ID NO 21: Teilfragment 1, mit vier Tryptophanen und C-terminalem His-Tag (AMB4-TF1-4W-His)** 
MQDDCNRGACHPTTGDLLVGRNTQLMASSTCGLSRAQKYCILSYLEGEQKCFICDSRFPY
DPYDQPNSHTIENVIVSFEPDREKKWWQSENGLDHVSIRLDLEALFRFSHLILTFKTFRPAAM
LVERSTDYGHNWKVFKYFAKDCATSFPNITSGQAQGVGDIVCDSKYSDIEPSTGGEVVLKV
LDPSFEIENPYSPYIQDLVTLTNLRINFTKLHTLGDALLGRRQNDSLDKYYYALYEMIVRGSCF
CNGHASECRPMQKMRGDVFSPPGMVHGQCVCQHNTDGPNCERCKDFFQDAPWLEHHH
HHH
**SEQ ID NO 22: Teilfragment 2, mit vier Tryptophanen und C-terminalem His-Tag (LAMB4-TF2-4W-His)** 
MPNCERCKDFFQDAPWRPAADLQDNACRSCSCNSHSSRCHFDMTTYLASGGLSGGVCED
CQHNTEGQHCDRCRPLFYRDPLKTISDPYACIPCECDPDGTISGGICVSHSDPALGSVAGQ
CLCKENVEGAKCDQCKPNHYGLSATDPLGCQPCDCNPLGSLPFLTCDVDTGQCLCLSYVT
GAHCEECTVGYWGLGNHLHGCSPCDCDIGGAYSNVCSPKNGQCECRPHVTGRSCSEPAP
GYFFAPLNFYLYEAEEATTLQGLAPLGSETFGQSPAVHVVLGEPVPGNPVTWTGPGFARVL
WLEHHHHHH
**SEQ ID NO 23: Teilfragment 3, mit vier Tryptophanen und C-terminalem His-Tag (LAMB4-TF3-4W-His)** 
MGNPVTWTGPGFARVLPGAGLRFAVNNIPFPVDFTIAIHYETQSAADWTVQIVVNPPGGSE
HCIPKTLQSKPQSFALPAATRIMLLPTPICLEPDVQYSIDVYFSQPLQGESHAHSHVLVDSLG
LIPQINSLENFCSKQDLDEYQLHNCVEIASAMGPQVLPGACERLIISMSAKLHDGAVACKCHP
QGSVGSSCSRLGGQCQCKPLVVGRCCDRCSTGSYDLGHHGCHPCHCHPQGSKDTVCDQ
VTGQCPCHGEVSGRRCDRCLAGYFGFPSCHPCPCNRFAELCDPETGSCFNCGGFTTGRN
CERCIDGYYGNWGWLEHHHHHH
**SEQ ID NO 24: Teilfragment 4, mit vier Tryptophanen und C-terminalem His-Tag (LAMB4-TF4-4W-His)** 
MTGRNCERCIDGYYGNPSSGQPCRPCLCPDDPSSNQYFAHSCYQNLWSSDVICNCLQGY
TGTQCGECSTGFYGNPRISGAPCQPCACNNNIDVTDPESCSRVTGECLRCLHNTQGANCQ
LCKPGHYGSALNQTCRRCSCHASGVSPMECPPGGGACLCDPVTGACPCLPNVTGLACDR
CADGYWNLVPGRGCQSCDCDPRTSQSSHCDQLTGQCPCKLGYGGKRCSECQENYYGDP
PGRCIPCDCNRAGTQKPICDPDTGMCRCREGVSGQRCDRCARGHSQEWGWLEHHHHHH
**SEQ ID NO 25: Teilfragment 5, mit vier Tryptophanen und C-terminalem His-Tag (LAMB4-TF5-4W-His)** 
MSGQRCDRCARGHSQEFPTCLQCHLCFDQWDHTISSLSKAVQGLMRLAANMEDKRETLP
VCEADFKDLRGNVSEIERILKHPVFPSGKFLKVKDYHDSVRRQIMQLNEQLKAVYEFQDLKD
TIERAKNEADLLLEDLQEEIDLQSSVLNASIADSSENIKKYYHISSSAEKKINETSSTINTSANT
RNDLLTILDTLTSKGNLSLERLKQIKIPDIQILNEKVCGDPGNVPCVPLPCGGALCTGRKGHR
KCRGPGCHGSLTLSTNALQKAQEAKSIIRNLDKQVRGLKNQIESISEQAEVSKNNALQLREK
LGNIWGWGWLEHHHHHH
**SEQ ID NO 26: Teilfragment 6, mit vier Tryptophanen und C-terminalem His-Tag (LAMB4-TF6-4W-His)** 
MSKNNALQLREKLGNIRNQSDSEEENINLFIKKVKNFLLEENVPPEDIEKVANGVLDIHLPIPS
QNLTDELVKIQKHMQLCEDYRTDENRLNEEADGAQKLLVKAKAAEKAANILLNLDKTLNQLQ
QAQITQGRANSTITQLTANITKIKKNVLQAENQTREMKSELELAKQRSGLEDGLSLLQTKLQR
HQDHAVNAKVQAESAQHQAGSLEKEFVELKKQYAILQRKTSTTGLTKETLGKVKQLKDAAE
KLAGDTEAKIRRITDLERKIQDLNLSRQAKADQLRILEDQVVAIKNEIVEQEKKYARCYSWGW
GWGWLEHHHHHH
**SEQ ID NO 27: Laminin beta-4** 
MQFQLTLFLHLGWLSYSKAQDDCNRGACHPTTGDLLVGRNTQLMASSTCGLSRAQKYCIL
SYLEGEQKCFICDSRFPYDPYDQPNSHTIENVIVSFEPDREKKWWQSENGLDHVSIRLDLEA
LFRFSHLILTFKTFRPAAMLVERSTDYGHNWKVFKYFAKDCATSFPNITSGQAQGVGDIVCD
SKYSDIEPSTGGEVVLKVLDPSFEIENPYSPYIQDLVTLTNLRINFTKLHTLGDALLGRRQNDS
LDKYYYALYEMIVRGSCFCNGHASECRPMQKMRGDVFSPPGMVHGQCVCQHNTDGPNCE
RCKDFFQDAPWRPAADLQDNACRSCSCNSHSSRCHFDMTTYLASGGLSGGVCEDCQHNT
EGQHCDRCRPLFYRDPLKTISDPYACIPCECDPDGTISGGICVSHSDPALGSVAGQCLCKEN
VEGAKCDQCKPNHYGLSATDPLGCQPCDCNPLGSLPFLTCDVDTGQCLCLSYVTGAHCEE
CTVGYWGLGNHLHGCSPCDCDIGGAYSNVCSPKNGQCECRPHVTGRSCSEPAPGYFFAP
LNFYLYEAEEATTLQGLAPLGSETFGQSPAVHVVLGEPVPGNPVTWTGPGFARVLPGAGL
RFAVNNIPFPVDFTIAIHYETQSAADWTVQIVVNPPGGSEHCIPKTLQSKPQSFALPAATRIM
LLPTPICLEPDVQYSIDVYFSQPLQGESHAHSHVLVDSLGLIPQINSLENFCSKQDLDEYQLH
NCVEIASAMGPQVLPGACERLIISMSAKLHDGAVACKCHPQGSVGSSCSRLGGQCQCKPLV
VGRCCDRCSTGSYDLGHHGCHPCHCHPQGSKDTVCDQVTGQCPCHGEVSGRRCDRCLA
GYFGFPSCHPCPCNRFAELCDPETGSCFNCGGFTTGRNCERCIDGYYGNPSSGQPCRPC
LCPDDPSSNQYFAHSCYQNLWSSDVICNCLQGYTGTQCGECSTGFYGNPRISGAPCQPCA
CNNNIDVTDPESCSRVTGECLRCLHNTQGANCQLCKPGHYGSALNQTCRRCSCHASGVSP
MECPPGGGACLCDPVTGACPCLPNVTGLACDRCADGYWNLVPGRGCQSCDCDPRTSQS
SHCDQLTGQCPCKLGYGGKRCSECQENYYGDPPGRCIPCDCNRAGTQKPICDPDTGMCR
CREGVSGQRCDRCARGHSQEFPTCLQCHLCFDQWDHTISSLSKAVQGLMRLAANMEDKR
ETLPVCEADFKDLRGNVSEIERILKHPVFPSGKFLKVKDYHDSVRRQIMQLNEQLKAVYEFQ
DLKDTIERAKNEADLLLEDLQEEIDLQSSVLNASIADSSENIKKYYHISSSAEKKINETSSTINT
SANTRNDLLTILDTLTSKGNLSLERLKQIKIPDIQILNEKVCGDPGNVPCVPLPCGGALCTGRK
GHRKCRGPGCHGSLTLSTNALQKAQEAKSIIRNLDKQVRGLKNQIESISEQAEVSKNNALQL
REKLGNIRNQSDSEEENINLFIKKVKNFLLEENVPPEDIEKVANGVLDIHLPIPSQNLTDELVKI
 
QKHMQLCEDYRTDENRLNEEADGAQKLLVKAKAAEKAANILLNLDKTLNQLQQAQITQGRA
NSTITQLTANITKIKKNVLQAENQTREMKSELELAKQRSGLEDGLSLLQTKLQRHQDHAVNA
KVQAESAQHQAGSLEKEFVELKKQYAILQRKTSTTGLTKETLGKVKQLKDAAEKLAGDTEAK
IRRITDLERKIQDLNLSRQAKADQLRILEDQVVAIKNEIVEQEKKYARCYS
**SEQ ID NO 28: SEQ ID NO 8 aus** EP3260864 
MDCTFKPDFEMTVKECQHSGELSSRNTGHLHPTPRSPLLRWTQEPQPLEEKWQHRVVEQI
PKEVQFQPPGAPLEKEKSQQCYSEYFSQTSTELQITFDETNPITRLSEIEKIRDQALNNSRPP
VRYQDNACEMELVKVLTPLEIAKNKQYDMHTEVTTLKQEKNPVPSAEEWMLEGCRASGGL
KKGDFLKKGLEPETFQNFDGDHACSVRDDEFKFQGLRHTVTARQLVEAKLLDMRTIEQLRL
GLKTVEEVQKTLNKFLTKATSIAGLYLESTKEKISFASAAERIIIDKMVALAFLEAQAATGFIIDP
ISGQTYSVEDAVLKGVVDPEFRIRLLEAEKAAVGYSYSSKTLSVFQAMENRMLDRQKGKHIL
EAQIASGGVIDPVRGIRVPPEIALQQGLLNNAILQFLHEPSSNTRVFPNPNNKQALYYSELLR
MCVFDVESQCFLFPFGERNISNLNVKKTHRISVVDTKTGSELTVYEAFQRNLIEKSIYLELSG
QQYQWKEAMFFESYGHSSHMLTDTKTGLHFNINEAIEQGTIDKALVKKYQEGLITLTELADS
LLSRLVPKKDLHSPVAGYWLTASGERISVLKASRRNLVDRITALRCLEAQVSTGGIIDPLTGK
KYRVAEALHRGLVDEGFAQQLRQCELVITGIGHPITNKMMSVVEAVNANIINKEMGIRCLEFQ
YLTGGLIEPQVHSRLSIEEALQVGIIDVLIATKLKDQKSYVRNIICPQTKRKLTYKEALEKADFD
FHTGLKLLEVSEPLMTGISSLYYSSLLE
**SEQ ID NO 29: Gliadin-Peptid**
   LGQQQPFPPQQPYPQPQPFPSQQPY
**SEQ ID NO 30: Gliadin-Peptid**
   QLQPFPQPELPYPQPQS
**SEQ ID NO 31: Gliadin-Peptid**
   QQLPQPEQPQQSFPEQERPF

Im Folgenden wird die Erfindung unter Bezugnahme auf die Figuren anhand von Ausführungsbeispielen erläutert. Die beschriebenen Ausführungsformen sind in jeder Hinsicht lediglich beispielhaft und nicht als einschränkend zu verstehen, und verschiedene Kombinationen der angeführten Merkmale sind vom Umfang der Erfindung umfasst.

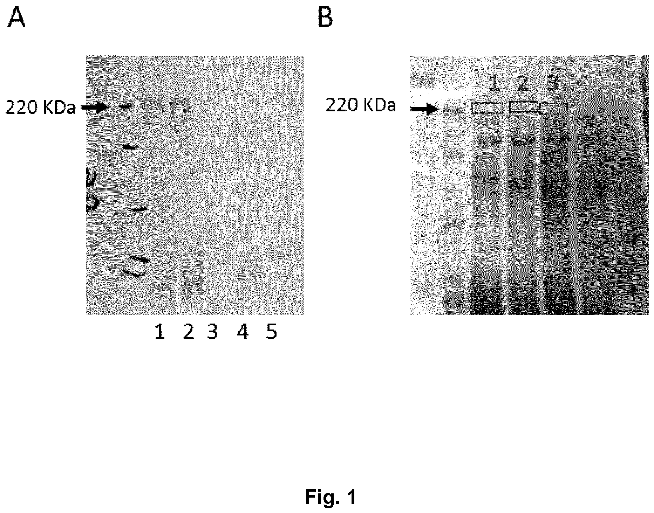

**Fig. 1** zeigt eine Immunpräzipitation mit Anti-p200-Pemphigoid Patienten IgG bzw. Serum ohne LAMC1-Reaktivität und Extrakt humaner Dermis. A: Westernblot-Analyse: Nachweis der immunpräzipitierten Proteine durch Inkubation mit einem Anti-p200-Pemphigoid Serum als Erstantikörper (Verdünnung 1:50). Reihe 1: Anti-p200 IgG (aufgereinigt) ohne LAMC1-cterm Reaktivität, Reihe 2: Anti-p200-Pemphigoid Serum ohne LAMC1-cterm Reaktivität, Reihe 3: normales humanes IgG (aufgereinigt), Reihe 4: normales humanes Serum, Reihe 5: Gammbind G Sepharose alleine B: Silver blue (Coomassie)-Färbung eines Gels mit den gleichen Proben wie in A. Die Banden 1, 2 und 3 wurden ausgeschnitten und einer LC-MS/MS-Analyse unterzogen. Laminin beta-4 wurde als mögliches Autoantigen identifiziert (Banden 1 und 2).

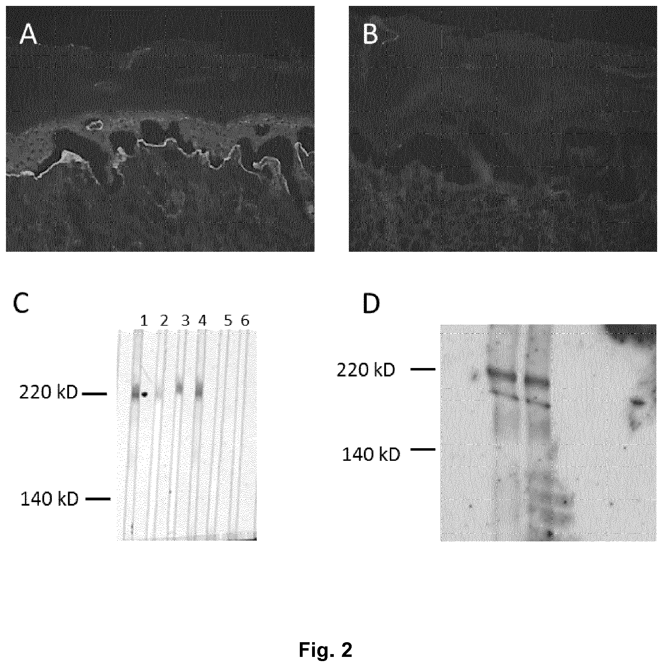

**Fig. 2** zeigt den Nachweis von Laminin beta-4 in der Haut. A, B: Indirekte Immunfluoreszenz Mikroskopie: Färbung von Laminin beta-4 auf der dermalen Seite des artifiziellen Spaltes in salzgespaltener humaner Haut mit Hilfe eines kommerziellen polyklonalen Antikörpers (Cloud-Clone; PAC079Hu01). B. Negativkontrolle. C: Test des Laminin beta-4 Antikörpers in Harnstoffexktrakten humaner Dermis mittels Westernblot-Analyse. Streifen 1 und 2: Anti-200 Pemphigoid Seren, Streifen 3: Laminin gamma 1 Antikörper, Streifen 4: Laminin beta-4 Antikörper, Streifen 5 und 6: normale humane Seren. D: Immunpräzipitation mit Laminin beta-4 Antikörper und Extrakt humaner Dermis gefolgt von Westernblot-Analyse (Anfärbung mit einem Pool aus Anti-p200 Pemphigoid Patienten IgG ohne Laminin gamma 1 Reaktivität). Reihe 1 und 2: Anti-Laminin beta-4 Antikörper (5 bzw 10 µg), Reihe 3: Anti-NC16A IgG (Kontroll-IgG, 10 µg), Reihe 4: normales Kaninchen IgG (10 µg), Reihe 5: Gammabind G Sepharose alleine

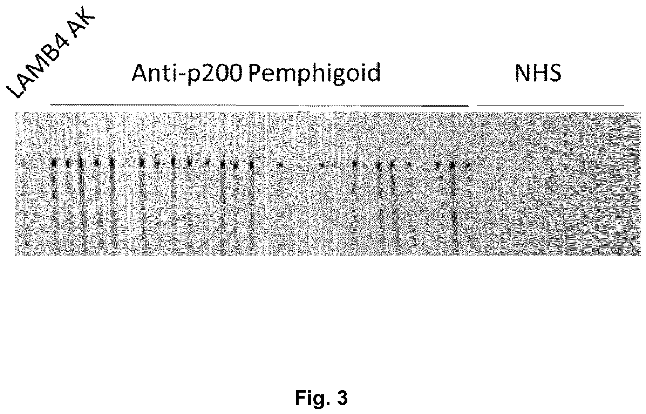

**Fig. 3** zeigt eine repräsentative Westernblot-Analyse mit Laminin beta-4 exprimierenden HEK293 Zellen und Anti-p200-Pemphigoid Seren (n=29). HEK293 Zellen wurden mit LAMB4-pTriEx1 transfiziert (jetPRIME; Polyplus) und nach 48h in RIPA Puffer lysiert. Das Proteinextrakt wurde mittels SDS PAGE aufgetrennt, die Proteine auf eine Nitrozellulosemembran übertragen, die Membran geblockt und mit dem Laminin beta-4 Antikörper (kommerzieller polyklonaler Antikörper von Cloud-Clone; 1:2000), den Anti-p200 Pemphigoid Seren (1:50) und den normalen humanen Seren (NHS, 1:50) gesunder Blutspender über Nacht inkubiert. Als Zweitantikörper wurde ein Anti-human IgG4 HRP Konjugat (1:2000) verwendet. Der Blot wurde für 1 min mit DAB entwickelt. Alle hier gezeigten Anti-p200 Pemphigoid Seren reagierten mit Laminin beta-4, wobei die Kontrollen keine Reaktion aufwiesen.

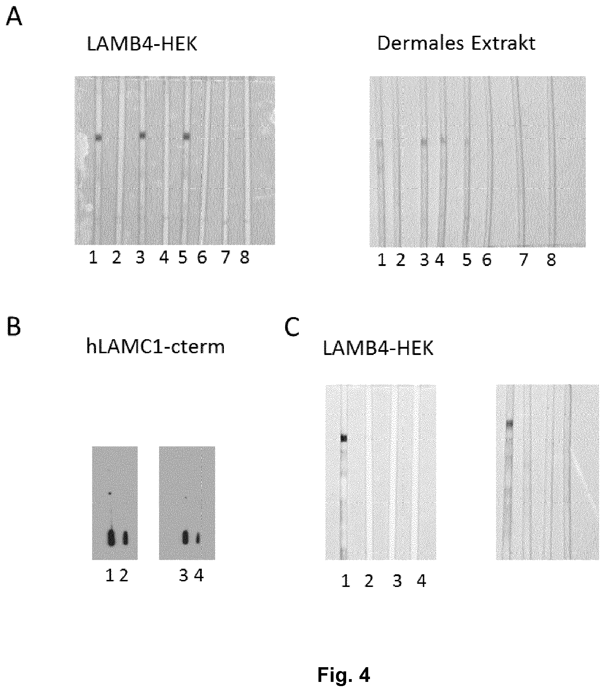

**Fig. 4** zeigt die Ergebnisse der Präadsorptionsstudie. Anti-p200-Pemphigoidseren wurden mit dem HEK293- Laminin beta-4 Extrakt präadsorbiert (ü. Nacht) und dann auf ihre Reaktivität im Westernblot getestet. A: Links: drei Anti-p200-Pemphigoidseren wurden mit HEK293- Laminin beta-4 Extrakt über Nacht präadsorbiert und wiesen im anschließenden Westernblot mit Laminin beta-4 im Vergleich zu den nicht-präadsorbierten Seren (1, 3, 5) kein Signal mehr auf (Streifen 2, 4, 6). Streifen 7, normales humanes Serum; Streifen 8, präadsorbiertes normales humanes Serum. Rechts: die gleichen Proben wurden auch mit Nitrozellulose-Streifen von dermalem Extrakt inkubiert und wiesen das gleiche Ergebnis auf. Nur Serum Nr. 2 reagierte trotz Präadsorption im Westernblot mit dermalem Extrakt. B: Im Westernblot mit rekombinantem hLAMC1-cterm konnte gezeigt werden, dass Serum Nr. 2 auch mit Laminin gamma-1 reagierte. C: Erneute Präadsorption des Serums Nr. 2 mit hLAMC1-cterm und dem Zellextrakt mit Laminin beta-4 Expression. Die Autoantikörperreaktivität mit beiden Extrakten konnte aufgehoben werden. Streifen 1: Anti-p200-Pemphigoid Serum (Nr. 2), Streifen 2: präadsorbiertes Anti-p200-Pemphigoid Serum (Nr. 2), Streifen 3: normales humanes Serum, Streifen 4: präadsorbiertes normales humanes Serum.

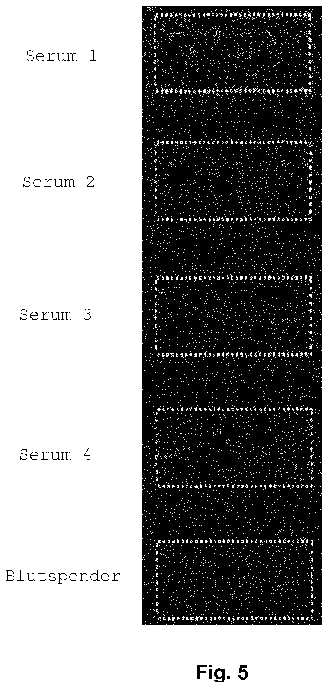

**Fig. 5** zeigt beispielhafte Primärdaten aus dem genauen Epitop-Mapping. Gezeigt wird das Signal von einem Mikroarray nach der Inkubation von vier Patientenseren und einer Probe von einem Blutspender (jeweils 1:10 verdünnt) mit IgG sekundärem Antikörper.

### Beispiel 1: Identifizierung von Laminin beta-4 als diagnostisch relevantes Autoantigen

### Herstellung von dermalem Extrakt

Zur Identifizierung eines weiteren Autoantigens des Anti-p200-Pemphigoids wurde zunächst eine Immunpräzipitation mit Extrakt humaner Dermis und aufgereinigter Patientenantikörper durchgeführt.

Dazu erfolgte zunächst der Herstellung des dermalen Extraktes (Zillikens D, Kawahara Y, Ishiko A, Shimizu H, Mayer J, Rank C V, Liu Z, Giudice G J, Tran H H, Marinkovich M P, Brocker E B, and Hashimoto T, A novel subepidermal blistering disease with autoantibodies to a 200-kDa antigen of the basement membrane zone. J Invest Dermatol, 1996. 106(6): p. 1333-8). Humane Hautstücke wurden für 48h in PBS (phosphate-buffered saline) mit 1M NaCl, 5mM EDTA, 1 mM PMSF und Proteaseinhibitor Cocktail (Set III, Merck) bei 4°C inkubiert. Nach 48h konnten dann die Dermis und die Epidermis voneinander getrennt werden. Um die Proteine aus der Dermis zu extrahieren, wurde die Dermis zunächst mit einem 4 M Harnstoffpuffer (4M Harnstoff, 12,5 mM Tris-HCl pH 6,8, 5 mM EDTA, 1 mM PMSF) für 10 min überschichtet, dann der Puffer entfernt und anschließend mit einem 9 M Harnstoffpuffer (9 M Harnstoff, 12,5 mM Tris-HCl pH 6,8, 2%SDS, 100 mM DTT, 5 mM EDTA und 1 mM PMSF) für 1 Stunde inkubiert. Das resultierende Extrakt wurde mittels Entsalzungssäulen (Zeba™ Spin Desalting Columns, Thermo Scientific) in RIPA Puffer (Radioimmunoprecipitation assay buffer: 0,1% SDS, 1% NP-40, 0,5% Na-deoxycholat, Proteaseinhibitoren (1 Tablette SIGMA*FAST*™ Protease Inhibitor Cocktail Tablets, EDTA-Free, Sigma Aldrich)) umgepuffert und bis zur Verwendung bei -20°C gelagert.

### Affinitätsreinigung von IgG-Antikörpern

Anti-p200-Pemphigoid IgG wurde mittels Protein G Sepharose (Genscript) nach Angaben des Herstellers aufgereinigt. Im Anschluss wurden die hLAMC1-cterm (C-Terminus von humanem Laminin gamma-1, Aminosäuren 1363-1609) spezifischen Autoantikörper mit Hilfe einer Affinitätsaufreinigung isoliert (Vafia K, Groth S, Beckmann T, Hirose M, Dworschak J, Recke A, Ludwig R J, Hashimoto T, Zillikens D, and Schmidt E, Pathogenicity of autoantibodies in anti-p200 pemphigoid. PLoS One, 2012. 7(7): p. e41769). Für die spezifische Aufreinigung der Patientenantikörper wurde zunächst hLAMC1-cterm-pQE40 in *E. coli* Rosetta 2 DE3 (Novagen/Merck, Darmstadt) transformiert, das Protein exprimiert und aufgereinigt (mittels IMAC). Schließlich erfolgte eine kovalente Kopplung des rekombinanten hLAMC1-cterm Proteins an Affigel 15 (BIO RAD) nach Angaben des Herstellers (Groth S, Recke A, Vafia K, Ludwig R J, Hashimoto T, Zillikens D, and Schmidt E, Development of a simple enzyme-linked immunosorbent assay for the detection of autoantibodies in anti-p200 pemphigoid. Br J Dermatol, 2011. 164(1): p. 76-82). Mit Hilfe dieser spezifischen Affinitätschromatographie konnten die hLAMC1-cterm spezifischen Autoantikörper aus den IgG Präparationen der Anti-p200-Pemphigoid Patientenseren entfernt werden.

### Immunpräzipitation

In der Immunpräzipitation wurden das umgepufferte dermale Extrakt als Proteinquelle und die aufgereinigten Patienten IgG ohne hLAMC1-cterm Reaktivität, ein Anti-p200-Pemphigoidserum ohne hLAMC1-cterm Antikörper und die entsprechenden Kontrollen gesunder Blutspender eingesetzt.

Zunächst wurde die Protein G Sepharose (GammaBind G Sepharose, GE Healthcare) mit RIPA Puffer gewaschen und mit Serum eines gesunden Blutspenders präadsorbiert. Nach der einstündigen Präadsorption und Zentrifugation wurde die Sepharose jeweils mit:
1. 30 µg Patienten IgG (Pool) ohne hLAMC1-cterm Reaktivität
2. 20 µl Patientenserum
3. 30 mg NH IgG (normales humanes IgG von gesunden Blutspendern)
4. 20 µl NHS (normales humanes Serum von einem gesunden Blutspender)
5. nur RIPA Puffer
in RIPA Puffer für 1h unter Rotation bei Raumtemperatur inkubiert und anschließen mit RIPA Puffer gewaschen. In jedes Reaktionsgefäß wurden dann 200 µl des umgepuffertem dermalen Extraktes pipettiert und die Reaktionsansätze dann über Nacht auf dem Rotator bei 4°C inkubiert. Nach einem Waschschritt mit RIPA-Puffer erfolgte ein Waschschritt mit Saccharose Puffer (1 M Saccharose, 150 mM NaCl, 10 mM EDTA, 10 mM HNaPO4). Abschließend erfolgte nochmal ein Waschschritt mit RIPA Puffer. Die Proben wurden jeweils mit 20 µl Lämmlipuffer 5 min bei 95°C erhitzt, zentrifugiert und die Proben auf 7,5%ige PROTEAN TGX Protein Gele (BIO RAD) aufgetragen.

Eine Hälfte vom Gel wurde Coomassie (Silver blue) gefärbt und die andere Hälfte wurde nach Transfer der Proteine auf eine Nitrozellulosemembran im Westernblot mit einem Anti-p200-Pemphigoidserum (Verdünnung 1:50, Inkubation über Nacht bei 4°C) analysiert. Bei den verwendeten Puffern handelte es sich um TBS-T mit 5% Milchpulver, zum Absättigen freier Bindungsstellen und TBST mit 5% Milchpulver und 1% BSA als Inkubationspuffer. Als Sekundärantikörper diente ein Anti-human IgG4 HRP Antikörper (1:2000, Klon HP6025, Southern Biotech). Der Blot wurde mit DAB (3,3'-Diaminobenzidin) entwickelt. Es konnte ein deutliches Signal bei ca. 200 kD beobachtet werden (**Fig. 1**). Die entsprechenden Banden aus dem Coomassie-gefärbten Gel wurde einer LC-MS/MS Analyse (Wistar Institute, Philadelphia) unterzogen und konnte als Laminin beta-4 (LAMB4) identifiziert werden.

### Beispiel 2: Überprüfung der Lokalisation von Laminin beta-4

Um die Lokalisation des Laminin beta-4 in der Haut zu überprüfen, wurde zunächst ein kommerziell erhältlicher Anti-Laminin beta-4 Antikörper (polyklonal, Cloud-Clone; PAC079Hu01) in der indirekten Immunfluoreszenz eingesetzt. Im Gegensatz zur Negativkontrolle bindet der Anti-Laminin beta-4 Antikörper, genau wie die Anti-p200-Pemphigoidseren (nicht gezeigt), im Boden der künstlichen Blase in humaner salzgespaltener Haut. Zusätzlich wurde der Anti-Laminin beta-4 Antikörper auch im Westernblot mit dermalem Extrakt getestet (Durchführung siehe oben). Der Westernblot mit dermalem humanem Extrakt ist der derzeitige diagnostische Standard für die Diagnose Anti-p200-Pemphigoid. Wie **Fig. 2** zeigt, reagiert der Anti-Laminin beta-4 Antikörper (Streifen 4) mit einer Proteinbande, die sich auf gleicher Höhe mit dem Signal der Anti-p200 Pemphigoidseren (Streifen 1 und 2) befindet. Das Signal des kommerziellen monoklonalen Laminin gamma-1 Antikörpers (Klon B-4, Santa Cruz) ist leicht nach oben verschoben.

Zusätzlich wurde der kommerzielle Anti-Laminin beta-4 Antikörper für eine Immunpräzipitation verwendet. Als Laminin beta-4 Quelle wurde auch hier wieder das dermale Extrakt verwendet. Für die Immunpräzipitation (Durchführung siehe oben) wurden 5 und 10 µg Anti-Laminin beta-4 Antikörper eingesetzt. Als Kontrollen wurden Anti-NC16A Kaninchen IgG (10 µg), normales Kannichen IgG (10 µg) und die GammaBind G Sepharose alleine eingesetzt. In der anschließenden Westernblot-Analyse erfolgte die Detektion von Laminin beta-4 mit einem Pool aus Anti-p200-Pemphigoid IgG ohne Laminin gamma-1 Reaktivität. In **Fig. 2** kann man deutlich erkennen, dass die Anti-p200-Pemphigoid Patienten Autoantikörper gegen Laminin beta-4 aufweisen.

### Beispiel 3: Klonierung

Laminin beta-4 (LAMB4) wurde als Variante mit C-terminal fusioniertem Polyhistidin-Tag und als authentische Variante rekombinant hergestellt. Der cDNA-Klon IRCBp5005F2212Q, kodierend für die Isoform 1 vom humanen Laminin beta-4, wurde von der Firma Source BioScience (Großbritannien) erworben und als Template für die PCR eingesetzt.

Für die Amplifikation der kodierenden Sequenz von Laminin beta-4 wurden für das Fragment ohne Translations-Stop-Codon am Ende der für Laminin beta-4 kodierenden Sequenz der sense-Primer LAMB4 (SEQ ID NO 1) und der asense-Primer LAMB4 (SEQ ID NO 2) genutzt. Die Amplifikation der Laminin beta-4-cDNA mit Translations-Stop-Codon am Ende der kodierenden Sequenz erfolgte mit dem sense-Primer LAMB4 (SEQ ID NO 1) und dem asense-Primer LAMB4-Stop (SEQ ID NO 3).

Beide PCR-Produkte enthielten an den Enden Schnittstellen für das Restriktionsenzym Bsal. Nach Bsal-Verdau wurden die Laminin beta-4-Amplifikate mit dem NcoI/XhoI-linearisierten Plasmidvektor pTriEx-1 (Merck, Darmstadt, Germany, SEQ ID NO 4) ligiert, so dass sich die Konstrukte gemäß SEQ ID NO 5 bzw. SEQ ID NO 6 ergeben. Die Ligationsansätze wurden in *E. coli* NEB 5-alpha (New England Biolabs GmbH) transformiert und Transformanden durch 100 µg/ml Ampicillin selektiert. Die isolierten Plasmide wurden durch Restriktionsanalysen und DNA-Sequenzierung auf Richtigkeit überprüft und für die Transfektion von HEK293-Zellen eingesetzt.

### Beispiel 4: Transfektion von HEK Zellen und Westernblot-Analyse

Zur Überprüfung, dass es sich bei Laminin beta-4 tatsächlich um ein Zielantigen des Anti-p200 Pemphigoids handelt, wurden HEK293 Zellen mittels jetPRIME (Polyplus transfection) mit dem LAMB4-pTriEx1 (mit His-Tag, SEQ ID NO 7) oder dem Leervektor transfiziert und nach 48h die Expression mittels kommerziellen Anti-Laminin beta-4 Antikörpers (s.o.) im Westernblot überprüft. Insgesamt wurden im Westernblot mit dem Laminin beta-4-HEK293 Extrakt (RIPA-Puffer) 49 Anti-p200-Pemphigoid Seren (Verdünnung 1:50) und 20 Seren gesunder Blutspender (NHS, normale humane Seren, Verdünnung 1:50) auf ihre Reaktivität mit Laminin beta-4 untersucht. Dazu wurde die fertige Nitrozellulosemembran in Streifen geschnitten. Als Positivkontrolle wurde der kommerzielle Anti-Laminin beta-4 Antikörper (Verdünnung 1:2000) eingesetzt. Die Inkubation der Streifen mit den Primärantikörpern erfolgte über Nacht bei 4°C. Auch hier dienten, wie oben beschrieben, ein Anti-human IgG4 HRP und ein Maus Anti-Kaninchen-HRP Antikörper als Sekundärantikörper. Der Blot wurde für 1 min mit DAB entwickelt. Insgesamt reagierten 47 der 49 Anti-p200-Pemphigoidseren mit Laminin beta-4 wobei die Negativkontrollen keine Reaktionen aufwiesen.

### Beispiel 5: Präadsorptionsstudie

Die korrekte Identifizierung von Laminin beta-4 wurde durch eine Präadsorptionsstudie bestätigt. Dazu wurden drei Anti-p200-Pemphigoidseren (ohne Laminin gamma-1 Reaktivität) mit dem Laminin beta-4-HEK293 Extrakt über Nacht bei 4°C präadsorbiert und dann auf ihre verbleibende Reaktivität im Westernblot mit dermalem Extrakt und Laminin beta-4-HEK 293 Extrakt getestet (**Fig. 4**). Die Westernblot-Analyse wurde wie oben beschrieben durchgeführt. Im Vergleich zu den nicht-präadsorbierten Seren (**Fig. 4**, Streifen 1, 3 und 5) wiesen die präadsorbierten Seren keine Reaktivität mehr mit dem Laminin beta-4-HEK293 Extrakt und 2 der 3 präadsorbierten Seren reagierten auch nicht mehr mit dem 200 kDa großen Protein im dermalen Extrakt. Die Reaktion ließ sich also neutralisieren. Nur Serum Nr.2 wies noch eine abgeschwächte Reaktion bei etwa 200 kDa auf. Dieses Anti-p200 Pemphigoidserum sollte eigentlich keine Reaktivität mit Laminin gamma-1 aufwiesen wurde aber trotzdem nochmal mittels hLAMC1-cterm Westernblot untersucht. Es stellte sich heraus, dass Serum Nr.2 tatsächlich Laminin gamma-1 Autoantikörper besaß, wodurch sich die Reaktion im dermalen Extrakt erklären lässt.

Durch eine Präadsorption des Serums Nr. 2 mit hLAMC1-cterm und dem Laminin beta-4-HEK293 Extrakt konnte die Autoantikörperreaktivität mit beiden Extrakten aufgehoben werden. Als Negativkontrolle diente in dieser Studie das Serum eines gesunden Blutspenders (NHS).

### Beispiel 6: Grobes Epitopmapping

Zur Identifizierung grober Epitope wurden sechs Teilfragmente von Laminin beta-4 (SEQ ID NO15, SEQ ID NO16, SEQ ID NO17, SEQ ID NO18, SEQ ID NO19 und SEQ ID NO20) kloniert und im pET24d-Vektor mit C-terminalem His-Tag unter Verwendung von Standardprotokollen exprimiert (SEQ ID NO 21, SEQ ID NO22, SEQ ID NO23, SEQ ID NO24, SEQ ID NO25 und SEQ ID NO26). Sofern die Konstrukte dabei nicht ohnehin vier Tryptophan-Rest aufwiesen, wurden diese in der Zahl künstlich anfusioniert, dass jedes Konstrukt zur Konzentrationsmessung vier Tryptophane aufwies.

Die Konstrukte wurden einer Westernblot-Analyse unterzogen, wie in Beispiel 1 beschrieben ist.

Die Ergebnisse sind in Tabelle 1 zusammengefasst. Im Wesentlichen zeigte sich erhebliche Aktivität gegen Teilfragment (TF) 5 und TF 6. Hingegen zeigten die Seren von Blutspendern (BS) praktisch keine Reaktivität.

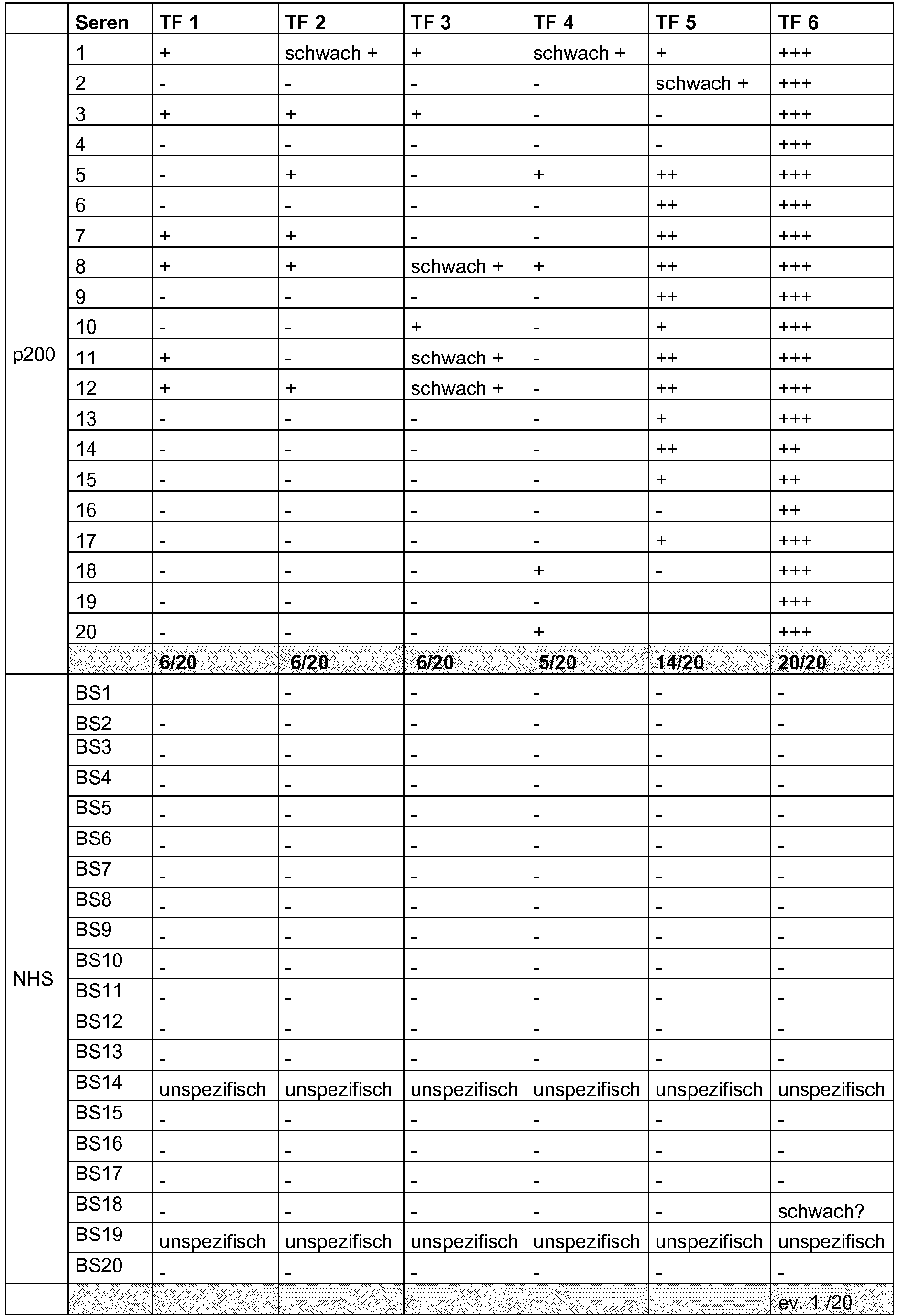

### Beispiel 7: Genaues Epitopmapping

Vier Patientenseren umfassend Autoantikörper gegen Laminin beta-4 wurden für ein Epitopmapping eingesetzt. Das Serum eines Blutspenders wurde zusätzlich inkubiert, um unspezifische Reaktionen erkennen zu können. Es wurden lineare 15mer-Peptide mit einer Überlappung von 14 Aminosäuren eingesetzt, die den C-terminalen Teil, genauer die Teilfragmente 5 (SEQ ID NO19) und 6 (SEQ ID NO20) abbilden. Die Peptide wurden mit den Proben bei einer Verdünnung von 1:100 in Inkubationspuffer (PBS, pH 7.4 mit 0.05% Tween 20 mit 10% Rockland blocking-Puffer MB-070) kontaktiert, 16 h bei 4°C und Schütteln bei 140 upm und in Verdünnungen von 1:500, 1:100 und 1:10 in Inkubationspuffer inkubiert, gefolgt von 45 min. Färben mit sekundären (Maus anti-human IgG (Fc) DyLight680 (0,5 µg/ml) und Kontroll-Antikörpern (Maus monoklonal anti-HA (12CA5) DyLight680 (0,2 µg/ml). Die Auslesung erfolgte mit einem LI-COR Odyssey Imaging System. Die Quantifizierung der Spotintensitäten und die Peptidannotation wurden mit einem PepSlide-Analyzer durchgeführt. Beispielhafte Primärdaten sind in **Fig. 5** zu sehen.

Ergebnis: es wurden geringe bis moderate spezifische Antikörperreaktionen mit Peptiden festgestellt, so dass die Epitope mit den Sequenzen SEQ ID NO 8, SEQ ID NO 9, SEQ ID NO 10, SEQ ID NO 11, SEQ ID NO 12, SEQ ID NO 13 und SEQ ID NO 14 identifiziert werden konnten.

## Patentansprüche

1. Polypeptid umfassend Laminin beta-4 oder eine Variante davon, bevorzugt in rekombinanter und/oder isolierter Form.

2. Polypeptid gemäß Anspruch 1, wobei das Polypeptid immobilisiert ist.

3. Träger umfassend das Polypeptide nach Anspruch 1 oder 2, weiter umfassend wenigstens ein, bevorzugt alle Antigene aus der Gruppe umfassend Laminin gamma-1, Laminin 332, BP180, BP230, Desmoglein 1, Desmoglein 3, Envoplakin, Gliadin und Kollagen Typ VII oder Varianten davon und Spalthaut.

4. Träger nach Anspruch 3, wobei der Träger aus der Gruppe ausgewählt ist, die einen Glasträger, bevorzugt Objektträger für Mikroskopie, noch bevorzugter mit einer eukaryontischen Zelle, die Laminin beta-4 oder eine Variante davon überexprimiert, einen Biochip, eine Mikrotiterplatte, eine Lateral Flow-Vorrichtung, einen Teststreifen, eine Membran, bevorzugt einen Blot, bevorzugter ein Linienblot, ein Chromatographiesäulenmaterial und einen Bead umfasst, bevorzugt einen magnetischen oder fluoreszenten Bead.

5. Therapeutisch nützlicher Träger umfassend das Polypeptid gemäß einem der Ansprüche 1 bis 2, wobei der Träger das Kontaktieren von menschlichem Blut mit dem Polypeptid, gefolgt von der Rückführung des Blutes in den Körper eines Patienten gestattet, wobei der Träger bevorzugt eine Aphereseapparatur ist.

6. Antikörper, bevorzugt Autoantikörper gegen Laminin beta-4, bevorzugt in isolierter Form.

7. Verwendung des Polypeptides, Trägers oder Autoantikörpers nach einem der Ansprüche 1 bis 6 zur Diagnose einer Krankheit.

8. Verfahren zur Herstellung eines diagnostisch oder therapeutisch nützlichen Trägers, umfassend den Schritt Beschichten des Trägers mit dem Polypeptid gemäß Anspruch 1.

9. Verfahren zum Aufreinigen eines Autoantikörpers, umfassend die Schritte
a) Kontaktieren einer Flüssigkeit umfassend den Autoantikörper mit dem Polypeptid nach Anspruch 1 unter Bedingungen, die die Ausbildung eines Komplexes umfassend den Autoantikörper und das Polypeptid gestatten,
b) Isolieren des Komplexes aus Schritt a), und
c) optional Nachweisen des Komplexes aus Schritt a) oder Dissoziieren des in Schritt b) isolierten Komplexes, gefolgt von Abtrennen des Autoantikörpers vom Polypeptid.

10. Verfahren umfassend den Schritt Nachweisen eines Autoantikörpers gegen Laminin beta-4 in einer Probe.

11. Pharmazeutische Zusammensetzung umfassend das Polypeptid nach Anspruch 1 oder eine Variante davon, bevorzugt zusammen mit einem pharmazeutisch akzeptablen Träger.

12. Kit umfassend das Polypeptid nach einem der Ansprüche 1 oder 2 oder den Träger nach einem der Ansprüche 3 bis 4, wobei das Kit ein oder mehr als ein Reagenz aus der Gruppe enthält, die eine Waschlösung, eine Kalibratorlösung, einen Antikörper gegen Laminin beta-4, und ein Mittel zum Nachweisen eines Autoantikörpers gegen Laminin beta-4, bevorzugt einen sekundären Antikörper, umfasst.

13. Verwendung des Polypeptids gemäß Anspruch 1, des Trägers gemäß einem der Ansprüche 3 bis 4 oder eines Antikörpers, bevorzugt Autoantikörpers gegen Laminin beta-4 zur Herstellung eines Kits oder diagnostisch oder therapeutisch nützlichen Trägers.

14. Verfahren, Verwendung oder Kit gemäß einem der Ansprüche 7 bis 10 und 12 bis 13, wobei ein Autoantikörper gegen Laminin beta-4 unter Verwendung einer Methode nachgewiesen wird, die aus der Gruppe ausgewählt ist, welche Immundiffusion, Immunelektrophorese, Lichtstreuung, Agglutination, Immuntest mit Markierung wie die aus der Gruppe umfassend Immuntest mit radioaktiver Markierung, mit enzymatischer Markierung, bevorzugter ELISA, mit Chemilumineszenz-Markierung, bevorzugter Elektrochemilumineszenz-Markierung, mit Immunfluoreszenz-Markierung, bevorzugt indirekte Immunfluoreszenz, umfasst.

15. Verwendung des Polypeptides gemäß Anspruch 1 oder des Antikörpers gemäß Anspruch 6 zur Bestimmung der Fähigkeit, bevorzugt Kapazität einer diagnostischen oder therapeutischen Vorrichtung oder eines diagnostischen oder therapeutischen Reagenz, einen Antikörper gegen Laminin beta-4 zu binden.

16. Verfahren gemäß Anspruch 10, wobei der Autoantikörper in einer Gewebeprobe eines Patienten oder mit einem Träger umfassend Laminin beta-4 oder eine Variante davon, bevorzugt gemäß Anspruch 3 oder 4 nachgewiesen wird.
